# EUROPEAN PATENT SPECIFICATION

(11) **EP 1 442 024 B1**
(45) Date of publication and mention of the grant of the patent: **19.03.2008**
(21) Application number: 02802307.5
(22) Date of filing: 29.10.2002
(51) Int. Cl.: C07D 239/30, C07D 239/46, C07D 239/48, C07D 403/10, A61K 31/506, A61P 3/10, A61P 25/28

(54) **AMINOBENZAMIDE DERIVATIVES AS GLYCOGEN SYNTHASE KINASE 3$g(b) INHIBITORS**
AMINOBENZAMIDDERIVATE ALS INHIBITOREN DER GLYCOGENSYNTHASEKINASE-3-
DERIVES D'AMINOBENZAMIDE UTILES COMME INHIBITEURS DE LA GLYCOGENE SYNTHASE KINASE 3$G(B)

(30) Priority: 01.11.2001 EP 01204192
(43) Date of publication of application: 04.08.2004
(73) Proprietor: Janssen Pharmaceutica NV, 2340 Beerse (BE)
(72) Inventor: FREYNE, Eddy J. E., Janssen Pharmaceutica N.V., B-2340 Beerse (BE); BUIJNSTERS, Peter J. J. A., Janssen Pharmaceutica N.V., B-2340 Beerse (BE); WILLEMS, Marc, Janssen Pharmaceutica N.V., B-2340 Beerse (BE); EMBRECHTS, Werner C. J., Janssen Pharmaceutica N.V., B-2340 Beerse (BE); JANSSEN, Paul A. J. (deceased), (BE); LEWI, Paulus Joannes, Janssen Pharmaceutica N.V., B-2340 Beerse (BE); HEERES, Jan, Janssen Pharmaceutica N.V., B-2340 Beerse (BE); DE JONGE, Marc R., Janssen Pharmaceutica N.V., B-2340 Beerse (BE); KOYMANS, Lucien M. H., Janssen Pharmaceutica N.V., B-2340 Beerse (BE); DAEYAERT, Frederik F. D., Janssen Pharmaceutica N.V., B-2340 Beerse (BE); KUKLA, Michael J., Maple Glen, PA 19002 (US); GEERTS, Hugo A. G., Berwyn, PA 19312 (US); NUYDENS, Rony M., Janssen Pharmaceutica N.V., B-2340 Beerse (BE); MERCKEN, Marc H., Janssen Pharmaceutica N.V., B-2340 Beerse (BE); LUDOVICI, Donald W., Janssen Pharmaceutica Inc., Titusville, NJ 08560 (US); JANSSEN, Jasmine, Josee, Werner, B-2360 Aartselaar (BE); JANSSEN, Maroussia, Godelieve, Frank, B-1410 Waterloo (BE); JANSSEN, Paul, Peter, Maria, B-9052 Zwijnaarde (BE); ARTS, Theodora, Joanna, Francisca, B-2350 Vosselaar (BE); JANSSEN, Herwig, Josephus, Margareta, B-2970 Schide (BE)
(86) International application number: PCT/EP2002/012079
(87) International publication number: WO 2003/037877

(56) References cited:
- EP-A- 0 162 204
- EP-A- 0 233 461
- EP-A- 0 337 943
- EP-A- 0 945 443
- WO-A-00/78731
- WO-A-01/64655
- WO-A-91/18887
- WO-A-99/65897
- US-A- 5 516 775
- US-A- 6 048 866
- ZIMMERMANN J ET AL: "Potent and selective inhibitors of the Abl-kinase: phenylamino-pyrimidine (PAP) derivatives" BIOORGANIC & MEDICINAL CHEMISTRY LETTERS, OXFORD, GB, vol. 7, no. 2, 21 January 1997 (1997-01-21), pages 187-192, XP004135990 ISSN: 0960-894X

## Description

The present invention concerns a novel group of compounds, their use as a medicine, their use for the manufacture of a medicament for the treatment of diseases mediated through glycogen synthase kinase 3, in particular glycogen synthase kinase 3β; processes for their preparation and pharmaceutical compositions comprising them.

WO 00/62778 describes cyclic protein tyrosine kinase inhibitors.
WO 91/18887 concerns diaminopyrimidine derivatives having gastric acid secretion inhibiting properties.
US 5,691,364 concerns benzamidine derivatives as anti-coagulants.
WO 98/41512 concerns substituted 2-anilinopyrimidines useful as inhibitors of src-family protein kinase.
WO 00/78731 discloses 5-cyano-2-aminopyrimidines as KDR and/or FGFr kinase inhibitors.
WO 99/50250 and WO 00/27825 concern HIV inhibiting aminopyrimidine derivatives. WO 95/09853 describes N-phenyl-2-pyrimidineamine derivatives for the treatment of tumor diseases.
WO 98/18782 concerns 2-pyrimidineamine derivatives as selective protein tyrosine kinase inhibitors.
EP 0,337,943 discloses N-phenyl-N-pyrimidin-2-yl derivatives having herbicidal plant growth regulating activity.
EP 0,164,204 concerns 2-aminopyrimidines which augment the immune respons.
EP 0,233,461 relates to 4,5,6-substituted 2-pyrimidineamines having anti-asthmatic activity.
US 5,516,775 concerns the use of 2-anilinopyrimidines as protein kinase C inhibitors. WO99/65897 concerns pyrimidine or pyridine based compounds as glycogen synthase kinase 3 inhibitors.

The present invention relates to compounds which are distinguishable from the prior art in structure, pharmacological activity, potency or selectivity.

The present disclosure concerns a compound of formula (I) 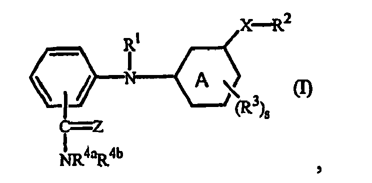 a N-oxide, a pharmaceutically acceptable addition salt, a quaternary amine and a stereochemically isomeric form thereof, wherein
Z represents O or S;
ring A is pyridyl, pyrimidinyl, pyrazinyl, pyridazinyl;
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is -NR¹-; NH NH-; -N=N-; -O-; -C(=O)-; -C(=S)-; -O-C(=O)-; -C(=O)-O-; -O-C(-O)-C₁₋₆ alkyl-; -C(=O)-O-C₁₋₆alkyl-; -O-C₁₋₆alkyl-C(=O)-; -C(=O)-C₁₋₆alkyl-O-; -O-C(=O)NR¹-; -NR¹-C(=O)-O-; -O-C(=O)-C(=O)-; -C(=O)-NR¹-, -NR¹-C(=O)-; -C(=S)-NR¹-, -NR¹-C(=S)-; -NR¹-C(=O)-NR¹-; -NR¹=C(=S)-NR¹-; NR¹-S(=O)NR¹-; -NR¹-S(=O)₂-NR¹-; -C₁₋₆alkyl-C(=O)-NR¹; -O-C₁₋₆alkyl-C(=O)-NR¹-; -C₁₋₆akyl-O-C(=O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -NR¹-C₂₋₆alkenyl-C₃₋₇cycloalkyl-; -O-C₂₋₆alkynyl-; -C₂₋₆alkynyl-O-; -NR¹-C₂₋₆alkynyl-; -C₂₋₆alkynyl-NR¹-; -NR¹-C₂₋₆yl-NR¹-; -NR¹-C₂₋₆alkyl-C₃₋₇cycloalkyl-; -O-C₁₋₆alkyl-O-; -O-C₂₋₆alkenyl-O-; -O-C₂₋₆alkynyl-O-; -CHOH-; -S-; -S(=O)-; -S(=O)₂-; -S(=O)-NR¹-; -S(=O)₂-NR¹-; -NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂- or a direct bond;
- R²: is hydrogen, C₁₋₁₀alkyl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R⁵R⁶N; R⁵R⁶N-C₁₋₆alkyl; R⁵R⁶N-C₃₋₇cycloalkyl; R⁵R⁶-C₁₋₆alkyloxy; R⁵R⁶N-C(=O)-; R⁵R⁶N-C(=S)-; R⁵R⁶N-C(=O)-NH-; R⁵R⁶-C(=S)-NH-; R⁵R⁶N-S(=O)ₙ-; R⁵R⁶N-S(=O) ₙ-NH-; R¹⁵ -C(=S)-; R¹⁵ -C(=O)-NH-; R¹⁵ -O-C(=O)-NH-; R¹⁵-S(=O)ₙ-NH-; R¹⁵-O-S(=O)ₙ-NH-; R¹⁵-C(=O)-NH-; R¹⁵ -O-C(=S)-NH-; R¹⁷R¹⁸N-Y₁ₐ-; R¹⁷R¹⁸N-Y₂-NR¹⁶-Y₁-; R¹⁵-Y₂-NR¹⁹-Y₁-; H-Y₂-NR¹⁹-Y₁-;
- R³: is hydrogen; hydroxy; halo; C₁₋₆alkyl; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷-S(=O)ₚ-; R⁷-S(=O)ₚ-NH-; R²¹-S(=O)ₚ-NH-; R⁷C(=O)-; -NHC(=O)H; -C(=O)NHNH₂; R⁷ -C(=O)-NH-; R²¹-C(=O)-NH-; -C(=NH)R⁷; -C(=NH)R²¹;
- R⁴⁸ or R^{4b}: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁵ and R⁶: each independently are hydrogen, R⁸, -Y₁-NR⁹-Y₂-NR¹⁰R¹¹, Y₁-NR⁹ - Y₁-R⁸, -Y₁-NR⁹R¹⁰, or
- R⁵ and R⁶: may together with the nitrogen to which they are attached form a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴, or each of said heterocycles may optionally be fused with a benzene ring, said benzene ring being optionally substituted with one or more substituents selected from R¹², R¹³ and R¹⁴.
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said groups representing R⁸ may optionally be substituted with one or more substituents selected from R¹², R¹³ and R¹⁴;
- R⁹, R¹⁰ and R¹¹: each independently are hydrogen or R⁸, or
- any: two of R⁹, R¹⁰ and R¹¹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R¹⁵R¹⁶-S(=O)-; R¹⁵R¹⁶-S(=O)₂; R¹⁷R¹⁸-Y₁-; R¹⁷R¹⁸N-Y₂-NR¹⁶-Y₁-; R¹⁸-Y₂-NR¹⁹-Y₁-; H-Y₂-NR¹⁹-Y₁-; oxo, or
- any: two of R¹², R¹³ and R¹⁴ may together be C₁₋₆alkanediyl or C₂₋₆alkenediyl thereby forming a saturated or partially saturated monocyclic 3 to 8 membered carbo - or heterocycle or an aromatic 4 to 8 membered monocyclic carbo - or heterocycle together with the atoms to which they are attached, or
- any: two of R¹² , R¹³ and R¹⁴ may together be -O-(CH₂)ᵣ-O- thereby forming a saturated, partially saturated or aromatic monocyclic 4 to 8 membered carbo - or heterocycle together with the atoms to which they are attached;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴; or each of said carbocycles or heterocycles may optionally be fused with a benzene ring, said benzene ring being optionally substituted with one or more substituents selected from R¹², R¹³ and R¹⁴;
- R¹⁶, R¹⁷, R¹⁸ and R¹⁹: each independently are hydrogen or R¹⁵, or R¹⁷ and R¹⁸, or R¹⁵ and R¹⁹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴; or
- R¹⁷ and R¹⁸: together with R¹⁶ may be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
- Y₁ₐ,: is -Y₃-S(=O)-Y₄-; -Y₃-S(=O)-Y₄-, -Y₃-C(=C)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₁ or Y₂: each independently are a direct bond, -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₃ or Y₄: each independently are a direct bond, C₁₋₆alkanediyl, C₂₋₆alkenediyl or C₂₋₆alkynediyl;
- n is: 1 or 2;
- m is: 1 or 2;
- p is: 1 or 2;
- r: is 1 to 5;
- s is: 1 to 3;
- aryl: is phenyl or phenyl substituted with one, two, three, four or five substituents each independently selected from halo. C₁₋₆alkyl, C₃₋₇cycloalkyl, C₁₋₆alkyloxy, cyano, nitro, polyhaloC₁₋₆alkyl and polyhaloC₁₋₆alkyloxy;
provided that -X-R² and/or R³ is other than hydrogen; and
provided that the following compounds
benzamide, 4-[(5-cyano-4-phenyl-2-pyrimidinyl)amino] N-[2-(diethylamino)ethyl]-_{;}
benzamide, 4-[[4-[6-(1-piperazinyl)-3-pyridinyl]-2-pyrimidinyl]amino]-;
benzamide, N-methyl-4-[[4-(3-pyridinyl)-2-pyrimidinyl]amino]-;
benzamide, 4-[[4-[(3-methoxyphenyl)thio]-2-pyrimidinyl]amino]-*N-*[2-(1-pyrrolidinyl)ethyl]-;
benzamide, *N*-[2-(diethylamino)ethyl]-4-[[4-(3-pyridinyl)-2-pyrimidinyl] amino]-;
benzamide, 4-[(5-amino-1,4-dihydro-4-oxo-2-pyrimidinyl)amino]-*N*,*N*-dimethyl-;
benzamide, 4-[(5-amino-1,4-dihydro-4-oxo-2-pyrimidinyl)amino]-*N*,*N* diethyl-;
benzamide, 4-[(5-amino-1,4-dihydro-4-oxo-2-pyrimidinyl)amino]-*N*-methyl-;
benzamide, 4-[[5-(4-methoxyphenyl)-2-pyrimidinyl]amino]-;
benzamide, 4-[[1-oxido-4-[(2,4,6-trimethylphenyl)amino]-2-pyrimidinyl]amino]-;
benzamide, 4-[[3-oxido-4-[(2,4,6-trimethylphenyl)amino]-2-pyrimidinyl]amino]-;
benzamide, 4-[[4-[(2,4,6-trimethylphenyl)amino]-2-pyrimidinyl]amino]-;
benzamide, 2-[[4-methyl-6-(trifluoromethyl)-2-pyrimidinyl]amino]-;
benzamide, N-(3-aminopropyl)- 3 -[[4-(3-pyridinyl)-2-pyrimidinyl]amino]-;
benzamide, *N*-(3-hydroxypropyl)-3-[[4-[2-[(3-hydroxypropyl)amino]-4-pyridinyl]-2-pyrimidinyl]amino]-;
benzamide, *N*-(3-arninopropyl)-3-[[4-[2-[(3-hydroxypropyl)amino]-4-pyridinyl]-2-pyrimidinyl] amino]-;
benzamide, 3-[[4,-[2-[(3-hydroxypropyl)amino]-4-pyridinyl]-2-pyrimidinyl]amino]-N-[2-(1H-imidazol-4-yl)ethyl]-;
benzamide, 4,4'-[(6-methyl-5-nitro-2,4-pyrimidinediyl)diimino]bis-;
benzamide, 4-[[5-amino-4-(methylamino)-2-pyrimidinyl]amino]-*N,N*-diethyl-;
benzamide, *N,N*-diethyl-4-[[4-(methylamino)-5-nitro-2-pyrimidinyl]amino]-are not included.

The present disclosure also relates to the use of a compound for the manufacture of a medicament for the prevention or the treatment of diseases mediated through GSK3, said compound being a compound of formula of formula (I') 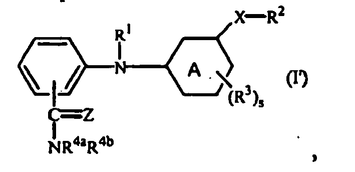 a *N*-oxide, a pharmaceutically acceptable addition salt, a quaternary amine and a stereochemically isomeric form thereof, wherein
Z represents O or S;
ring A is pyridyl, pyrimidinyl, pyrazinyl, pyridazinyl;
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is -NR¹-; -NH-NH-; -N=N-; -O-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O ; -O-C(=O)-NR¹-; -NR¹-C(=O)-O-; -O-C(=O)-C(=O)-; -C(=O)-NR¹-, -NR¹-C(=O)-; -C(=S)-NR¹-, -NR¹-C(=S)-; -NR¹-C(=O)-NR¹-; -NR¹-C(=S)-NR¹-; -NR¹-S(=O)-NR¹-; -NR¹-S(=O)₂- NR¹-; -C₁₋₆alkyl-C(=O)-NR¹-; -O-C₁₋₆alkyl-C(=O)-NR¹-; -C₁₋₆alkyl-O-C(=O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; - -NR¹-C₂₋₆alkenyl-; -C₂₋₆alkenyl-NR¹-; -NR¹-C₂₋₆alkenyl-NR¹-; NR¹-C₂₋₆alkenyl-C₃₋₇cycloalkyl-; -O-C₂₋₆alkynyl-; -C₂₋₆alkynyl-O-; -NR¹-C₂₋₆alkynyl-; -C₂₋₆alkynyl-NR¹-; -NR¹-C₂₋₆alkynyl-NR¹-; -NR¹-C₂₋₆alkynyl-C₃₋₇cycloalkyl-; -O-C₁₋₆alkyl-O-; -O-C₂₋₆alkenyl-O-; -O-C₂₋₆alkynyl-O-; -CHOH-; -S-; -S(=O)-; -S(=O)₂-; -S(=O)-NR¹-; -S(=O)₂-NR¹-, -NR¹-S(=O)-; NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂- or a direct bond;
- R²: is hydrogen, C₁₋₁₀akl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R⁵R⁶N; R⁵R⁶N-C₁₋₆alkyl; R⁵R⁶N-C₃₋₇cycloalkyl; R⁵R⁶-C₁₋₆alkyloxy; R⁵R⁶N-C(=O)-; R⁵R⁶N-C(=S)-; R⁵R⁶N-C(=O)-NH-; R⁵R⁶N-C(=S)-NH-; R⁵R⁶N-S(=O)ₙ-; R⁵R⁶N-S(=O)ₙ NH-; R¹⁵-C(=S)-; R¹⁵-C(=O)-NH-, R¹⁵-O-C(=O)-NH; R¹⁵-S(=O)ₙ-NH-; R¹⁵-O-S(=O)ₙ-NH-; R¹⁵-C(=S)-NH-; R¹⁵-O-C(-S)-NH-; R¹⁷R¹⁸N-Y₁ₐ-; R¹⁷R¹⁸N-Y₂-NR¹⁶-Y₁-; R¹⁵-Y₂-NR¹⁹-Y₁-; H-Y₂-NR¹⁹-Y₁-;
- R³: is hydrogen; hydroxy; halo; C₁₋₆alkyl; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷ -S(=O)ₚ-; R⁷-S(=O)ₚ-NH-; R²¹-S(=O)ₚ-NH-; R⁷-C(=O)-; -NHC(=O)H; -C(=O)NHNH₂; R⁷-C(=O)-NH-; R²¹-C(=O)-NH-; -C(=NH)R⁷; -C(=NH)R²¹;
- R^{4a} or R^{4b}: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁵ and R⁶: each independently are hydrogen, R⁸, -Y₁-NR⁹-Y₂-NR¹⁰R¹¹, -Y₁-NR⁹-Y₁-R⁸, -Y₁-NR⁹R¹⁰, or
- R⁵ and R⁶: may together with the nitrogen to which they are attached form a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴, or each of said heterocycles may optionally be fused with a benzene ring, said benzene ring being optionally substituted with one or more substituents selected from R¹², R¹³ and R¹⁴;
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said groups representing R⁸ may optionally be substituted with one or more substituents selected from R¹², R¹³ and R¹⁴;
- R⁹, R¹⁰ and R¹¹: ; each independently are hydrogen or R⁸, or
- any: two of R⁹, R¹⁰ and R¹¹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R¹⁵R¹⁶-S(=O)-; R¹⁵R¹⁶N-S(=O)₂-; R¹⁷R¹⁸-Y₁-; R¹⁷R¹⁸N-Y₂-NR¹⁶-Y₁-; R¹⁵-Y₂-NR¹⁹-Y₁-; H-Y₂-NR¹⁹-Y₁-; oxo, or
- any: two of R¹², R¹³ and R¹⁴ may together be C₁₋₆alkanediyl or C₂₋₆alkenediyl thereby forming a saturated or partially saturated monocyclic 3 to 8 membered carbo - or heterocycle or an aromatic 4 to 8 membered monocyclic carbo - or heterocycle together with the atoms to which they are attached, or
- any: two of R¹² , R¹³ and R¹⁴ may together be -O-(CH₂)ᵣ-O- thereby forming a saturated, partially saturated or aromatic monocyclic 4 to 8 membered carbo - or heterocycle together with the atoms to which they are attached;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴ ; or each of said carbocycles or heterocycles may optionally be fused with a benzene ring, said benzene ring being optionally substituted with one or more substituents selected from R¹², R¹³ and R¹⁴;
- R¹⁶, R¹⁷, R¹⁸ and R¹⁹: each independently are hydrogen or R¹⁵, or
- R¹⁷ and R¹⁸, or R¹⁵ and R¹⁹: may together be C₁₋₆alkanediyl or C₂₋₆alkenediyl thereby forming a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴; or
- R¹⁷ and R¹⁸: together with R¹⁶ may be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle,
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
- Y₁ₐ: is -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or Y₃-C(=O)-O-Y₄-;
- Y₁ or Y₂: each independently are a direct bond, -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₃ or Y₄: each independently are a direct bond, C₁₋₆alkanediyl, C₂₋₆alkenediyl or C₂₋₆alkynediyl;
- n is: 1 or 2;
- m: is 1 or 2;
- p is: 1 or 2;
- r is: 1 to 5;
- s: is 1 to 3;
- aryl: is phenyl or phenyl substituted with one, two, three, four or five substituents each independently selected from halo, C₁₋₆alkyl, C₃₋₇cycloalkyl, C₁₋₆alkyloxy cyano, nitro, polyhaloC₁₋₆alkyl and polyhaloC₁₋₆alkyloxy;
provided that -X-R² and/or R³ is other than hydrogen.

As used herein C₁₋₃alkyl as a group or part of a group defines straight or branched chain saturated hydrocarbon radicals having from 1 to 3 carbon atoms such as methyl, ethyl, propyl, 1-methylethyl; C₁₋₄alkyl as a group or part of a group defines straight or branched chain saturated hydrocarbon radicals having from 1 to 4 carbon atoms such as the groups defined for C₁₋₃alkyl and butyl; C₁₋₆alkyl as a group or part of a group defines straight or branched chain saturated hydrocarbon radicals having from 1 to 6 carbon atoms such as the groups defined for C₁₋₄alkyl and pentyl, hexyl, 2-methylbutyl and the like; C₁₋₁₀alkyl as a group or part of a group defines straight or branched chain saturated hydrocarbon radicals having from 1 to 10 carbon atoms such as the groups defined for C₁₋₆alkyl and heptyl, octyl, nonyl, decyl and the like; C₁₋₆alkanediyl as a group or part of a group defines bivalent straight or branched chain saturated hydrocarbon radicals having from 1 to 6 carbon atoms such as methylene, 1,2-ethanediyl or 1,2-ethylidene,1,3-propanediyl or 1,3-propylidene,1,4-butanediyl or 1,4-butylidene and the like; C₂₋₆alkenyl defines straight and branched chain hydrocarbon radicals having from 2 to 6 carbon atoms containing a double bond such as ethenyl, propenyl, butenyl, pentenyl, hexenyl and the like; C₂₋₁₀alkenyl defines straight and branched chain hydrocarbon radicals having from 2 to 10 carbon atoms containing a double bond such as the groups defined for C₂₋₆alkenyl and heptenyl, octenyl, nonenyl, decenyl and the like; C₂₋₆alkenediyl defines bivalent straight and branched chain hydrocarbon radicals having from 2 to 6 carbon atoms containing one or more double bonds such as ethenediyl, propenediyl, butenediyl, pentenediyl, hexenediyl and the like; C₂₋₆alkynyl defines straight and branched chain hydrocarbon radicals having from 2 to 6 carbon atoms containing a triple bond such as ethynyl, propynyl, butynyl, pentynyl, hexynyl and the like; C₂₋₁₀alkynyl defines straight and branched chain hydrocarbon radicals having from 2 to 10 carbon atoms containing a triple bond such as the groups defined for C₂₋₆alkynyl and heptynyl, octynyl, nonynyl, decynyl and the like; C₂₋₆alkynediyl defines bivalent straight and branched chain hydrocarbon radicals having from 2 to 6 carbon atoms containing a triple bond such as ethynediyl, propynediyl, butynediyl, pentynediyl, hexynediyl and the like; C₃₋₆cycloalkyl is generic to cyclopropyl, cyclobutyl, cyclopentyl, cyclohexyl; C₃₋₇cycloalkyl is generic to cyclopropyl, cyclobutyl, cyclopentyl, cyclohexyl and cycloheptyl; a monocyclic, bicyclic or tricyclic saturated carbocycle represents a ring system consisting of 1, 2 or 3 rings, said ring system being composed of only carbon atoms and said ring system containing only single bonds; a monocyclic, bicyclic or tricyclic partially saturated carbocycle represents a ring system consisting of 1,2 or 3 rings, said ring system being composed of only carbon atoms and comprising at least one double bond provided that the ring system is not an aromatic ring system; a monocyclic, bicyclic or tricyclic aromatic carbocycle represents an aromatic ring system consisting of 1, 2 or 3 rings, said ring system being composed of only carbon atoms; the term aromatic is well known to a person skilled in the art and designates cyclically conjugated systems of 4n' + 2 electrons, that is with 6, 10, 14 etc. n-electrons (rule of Hückel; n' being 1, 2,3 etc.); a monocyclic, bicyclic or tricyclic saturated heterocycle represents a ring system consisting of 1, 2 or 3 rings and comprising at least one heteroatom selected from O, N or S, said ring system containing only single bonds; a monocyclic, bicyclic or tricyclic partially saturated heterocycle represents a ring system consisting of 1, 2 or 3 rings and comprising at least one heteroatom selected from O, N or S, and at least one double bond provided that the ring system is not an aromatic ring system; a monocyclic, bicyclic or tricyclic aromatic heterocycle represents an aromatic ring system consisting of 1, 2 or 3 rings and comprising at least one heteroatom selected from O, N or S.

Particular examples of monocyclic, bicyclic or tricyclic saturated carbocycles are cyclopropyl, cyclobutyl, cyclopentyl, cyclohexyl, cycloheptyl, cyclooctyl, bicyclo[4,2,0]octanyl, cyclononanyl, cyclodecanyl, decahydronapthalenyl, tetradecahydroanthracenyl.

Particular examples of monocyclic, bicyclic or tricyclic partially saturated carbocycles are cyclopropenyl, cyclobutenyl, cyclopentenyl, cyclohexenyl, cycloheptenyl, cyclooctenyl, bicyclo[4,2,0]octenyl, cyclononenyl, cyclodecenyl, octahydronaphthalenyl, 1,2,3,4-tetrahydronaphthalenyl, 1,2,3,4,4a,9,9a,10-octahydro-anthracenyl.

Particular examples of monocyclic, bicyclic or tricyclic aromatic carbocycles are phenyl, naphthalenyl, anthracenyl.

Particular examples of monocyclic, bicyclic or tricyclic saturated heterocycles are tetrahydrofuranyl, pyrrolidinyl, dioxolanyl, imidazolidinyl, thiazolidinyl, tetrahydrothienyl, dihydrooxazolyl, isothiazolidinyl, isoxazolidinyl, oxadiazolidinyl, triazolidinyl, thiadiazolidinyl, pyrazolidinyl, piperidinyl, hexahydropyrimidinyl, hexahydropyrazinyl, dioxanyl, morpholinyl, dithianyl, thiomorpholinyl, piperazinyl, trithianyl, decahydroquinolinyl, octahydroindolyl.

Particular examples of monocyclic, bicyclic or tricyclic partially saturated heterocycles are pyrrolinyl, imidazolinyl, pyrazolinyl, 2,3-dihydrobenzofuranyl, 1,3-benzodioxolyl, 2,3-dihydro-1,4-benzodioxinyl, indolinyl and the like.

Particular examples of monocyclic, bicyclic or tricyclic aromatic heterocycles are azetyl, oxetylidenyl, pyrrolyl, furyl, thienyl, imidazolyl, oxazolyl, isoxazolyl, thiazolyl, isothiazolyl, pyrazolyl, triazolyl, thiadiazolyl, oxadiazolyl, tetrazolyl, pyridyl, pyrimidinyl, pyrazinyl, pyridazinyl, triazinyl, pyranyl, benzofuryl, isobenzofuryl, benzothienyl, isobenzothienyl, indolizinyl, indolyl, isoindolyl, benzoxazolyl, benzimidazolyl, indazolyl, benzisoxazolyl, benzisothiazolyl, benzopyrazolyl, benzoxadiazolyl, benzothiadiazolyl, benzotriazolyl, purinyl, quinolinyl, isoquinolinyl, cinnolinyl, quinolizinyl, phthalazinyl, quinoxalinyl, quinazolinyl, naphthiridinyl, pteridinyl, benzopyranyl, pyrrolopyridyl, thienopyridyl, furopyridyl, isothiazolopyridyl, thiazolopyridyl, isoxazolopyridyl, oxazolopyridyl, pyrazolopyridyl, imidazopyridyl, pyrrolopyrazinyl, thienopyrazinyl, furopyrazinyl, isothiazolopyrazinyl, thiazolopyrazinyl, isoxazolopyrazinyl, oxazolopyrazinyl, pyrazolopyrazinyl, imidazopyrazinyl, pyrrolopyrimidinyl, thienopyrimidinyl, furopyrimidinyl, isothiazolopyrimidinyl, thiazolopyrimidinyl, isoxazolopyrimidinyl, oxazolopyrimidinyl, pyrazolopyrimidinyl, imidazopyrimidinyl, pyrrolopyridazinyl, thienopyridazinyl, furopyridazinyl, isothiazolopyridazinyl, thiazolopyridazinyl, isoxazolopyridazinyl, oxazolopyridazinyl, pyrazolopyridazinyl, imidazopyridazinyl, oxadiazolopyridyl, thiadiazolopyridyl, triazolopyridyl, oxadiazolopyrazinyl, thiadiazolopyrazinyl, triazolopyrazinyl, oxadiazolopyrimidinyl, thiadiazolopyrimidinyl, triazolopyrimidinyl, oxadiazolopyridazinyl, thiadiazolopyridazinyl, triazolopyridazinyl, imidazooxazolyl, imidazothiazolyl, imidazoimidazolyl, isoxazolotriazinyl, isothiazolotriazinyl, pyrazolotriazinyl, oxazolotriazinyl, thiazolotriazinyl, imidazotriazinyl, oxadiazolotriazinyl, thiadiazolotriazinyl, triazolotriazinyl, carbazolyl, acridinyl, phenazinyl, phenothiazinyl, phenoxazinyl.

As used herein before, the term (=O) forms a carbonyl moiety when attached to a carbon atom, a sulfoxide moiety when attached to a sulfur atom and a sulfonyl moiety when two of said terms are attached to a sulfur atom.

The term halo is generic to fluoro, chloro, bromo and iodo. As used in the foregoing and hereinafter, polyhalomethyl as a group or part of a group is defined as mono- or polyhalosubstituted methyl, in particular methyl with one or more fluoro atoms, for example, difluoromethyl or trifluoromethyl; polyhaloC₁₋₆alkyl as a group or part of a group is defined as mono- or polyhalosubstituted C₁₋₆alkyl, for example, the groups defined in halomethyl, 1,1-difluoro-ethyl and the like. In case more than one halogen atoms are attached to an alkyl group within the definition of polyhalomethyl or polyhatoC₁₋₆alkyl, they may be the same or different.

The term heterocycle as in the definition of for instance R², R⁵, R⁶, R⁸ or R¹⁵ is meant to include all the possible isomeric forms of the heterocycles, for instance, pyrrolyl also includes 2H-pyrrolyl.

The hereinabove-mentioned carbocycles may be attached to the remainder of the molecule of formula (I) or (I') through any ring carbon as appropriate, if not otherwise specified. Thus, for example, when the partially saturated bicyclic carbocycle is 1,2,3,4-tetrahydronaphthalenyl, it may be 1,2,3,4-tetrahydronaphthalen-1-yl, 1,2,3,4-tetrahydronaphthalen-2-yl and the like.

The hereinabove-mentioned heterocycles may be attached to the remainder of the molecule of formula (I) or (I') through any ring carbon or heteroatom as appropriate, if not otherwise specified. Thus, for example, when the aromatic monocyclic heterocycle is imidazolyl, it may be 1-imidazolyl, 2-imidazolyl, 4-imidazolyl and the like.

When any variable (eg. R⁵, R⁶ etc.) occurs more than one time in any constituent, each definition is independent.

Lines drawn into ring systems from substituents indicate that the bond may be attached to any of the suitable ring atoms.

For therapeutic use, salts of the compounds of formula (I) or (I') are those wherein the counterion is pharmaceutically acceptable. However, salts of acids and bases which are non-phannaceutically acceptable may also find use, for example, in the preparation or purification of a pharmaceutically acceptable compound. All salts, whether pharmaceutically acceptable or not are included within the ambit of the present invention.

The pharmaceutically acceptable addition salts as mentioned hereinabove are meant to comprise the therapeutically active non-toxic acid addition salt forms which the compounds of formula (I) or (I') are able to form. The latter can conveniently be obtained by treating the base form with such appropriate acids as inorganic acids, for example, hydrohalic acids, e.g. hydrochloric, hydrobromic and the like; sulfuric acid; nitric acid; phosphoric acid and the like; or organic acids, for example, acetic, propanoic, hydroxyacetic, 2-hydroxypropanoic, 2-oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form.

The compounds of formula (I) or (I') containing acidic protons may be converted into their therapeutically active non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline, the benzathine, N-methyl-D-glucamine, 2-amino-2-(hydroxymethyl)-1,3-propanediol, hydrabamine salts, and salts with amino acids such as, for example, arginine, lysine and the like. Conversely the salt form can be converted by treatment with acid into the free acid form.
The term addition salt also comprises the hydrates and solvent addition forms which the compounds of formula (I) or (I') are able to form. Examples of such forms are e.g. hydrates, alcoholates and the like.

The term "quaternary amine" as used hereinbefore defines the quaternary ammonium salts which the compounds of formula (I) or (I') are able to form by reaction between a basic nitrogen of a compound of formula (I) or (I') and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

It will be appreciated that some of the compounds of formula (I) or (I') and their *N-*oxides, addition salts, quaternary amines and stereochemically isomeric forms may contain one or more centers of chirality and exist as stereochemically isomeric forms.

The term "stereochemically isomeric forms" as used hereinbefore defines all the possible stereoisomeric forms which the compounds of formula (I) or (I'), and their *N*-oxides, addition salts, quaternary amines or physiologically functional derivatives may possess. Unless otherwise mentioned or indicated, the chemical designation of compounds denotes the mixture of all possible stereochemically isomeric forms, said mixtures containing all diastereomers and enantiomers of the basic molecular structure as well as each of the individual isomeric forms of formula (I) or (I') and their *N*-oxides, salts, solvates or quaternary amines substantially free, *i.e.* associated with less than 10%, preferably less than 5%, in particular less than 2% and most preferably less than 1% of the other isomers. In particular, stereogenic centers may have the R- or S-configuration; substituents on bivalent cyclic (partially) saturated radicals may have either the *cis-* or *trans*-configuration. Compounds encompassing double bonds can have an E or Z-stereochemistry at said double bond. Stereochemically isomeric forms of the compounds of formula (I) or (I') are obviously intended to be embraced within the scope of this invention.

The N-oxide forms of the present compounds are meant to comprise the compounds of formula (I) wherein one or several tertiary nitrogen atoms are oxidized to the so-called N-oxide.

Some of the compounds of formula (I) or (I') may also exist in their tautomeric form (e.g. keto-enol tautomerie). Such forms although not explicitly indicated in the above formula are intended to be included within the scope of the present invention.

Whenever used hereinafter, the term "compounds of formula (I)" or "compounds of formula (I) or (I')" is meant to also include their N-oxide forms, their salts, their quaternary amines and their stereochemically isomeric forms. Of special interest are those compounds of formula (I) or (I') which are stereochemically pure.

Particular compounds are those compounds of formula (I) or (I') as defined hereinabove provided that the molecular mass of the compounds is at most 1000 u, in particular at most 800 u, more in particular at most 700 u (u stands for unified atomic mass unit and equals 1.66x10⁻²⁷ kg).

Also particular interesting compounds are those compounds of formula (I) or (I') as defined hereinabove, their N-oxides, pharmaceutically acceptable addition salts, quaternary amines and stereochemically isomeric forms thereof, wherein Z represents O or S;
ring A is pyridyl, pyrimidinyl, pyrazinyl, pyridazinyl;
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is -NR¹-; -NH-NH-; -N=N-; -O-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -O-C(=O)-C(=O)-; -C(=O)-NR¹-, -NR¹-C(=O)-; -C(=S)-NR¹-, -NR¹-C(=S)-; -NR¹-C(=O)-NR¹-; -NR¹-C(=S)-NR¹-; NR¹-S(=O)-NR¹-; -NR¹-S(=O)₂-NR¹-; -C₁₋₆alkyl-C(=O)-NR¹-; -O-C₁₋₆alkyl-C(=O)-NR¹-; -C₁₋₆alkyl-O-C(=O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -NR¹-C₂₋₆alkenyl-C₃₋₇cycloalkyl-; -O-C₂₋₆alkynyl-; -C₂₋₆alkynyl-O-; -NR¹_C₂-₆alkynyl-; -C₂₋₆alkynyl-NR¹-; -NR¹C₂₋₆alkynyl-NR¹-; -NR¹-C₂₋₆alkynyl-C₃₋₇cycloalkyl-; -O-C₁₋₆alkyl-O-; -O-C₂₋₆alkenyl-O-; -O-C₂₋₆alkynyl-O-; -CHOH-; -S-; -S(=O)-; -S(=O)₂-; -S(=O)-NR¹-; -S(=O)₂-NR¹-; NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂- or a direct bond;
- R²: is hydrogen, C₁₋₁₀alkyl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(-O)-; R¹⁵-C(-O)-O-; R¹⁵-O-C(=O)-O-; -SO₃H; R¹⁵-S(=O)-; R¹⁵-S(=O)₂-; R⁵R⁶N; R⁵R⁶N-C₁₋₆alkyl; R⁵R⁶N-C₃₋₇cycloalkyl; R⁵R⁶N-C₁₋₆alkyloxy; R⁵R⁶N-C(-O)-; R⁵R⁶N-C(=S)-; R⁵R⁶N-C(=O)-NH-; R⁵R⁶N-C(=S)-NH-; R⁵R⁶-S(=O)ₙ-; R⁵R⁶N-S(=O) ₙ-NH-; R¹⁵-C(=S)-; R¹⁵-C(=O)-NH-; R¹⁵-O-C(=O)-NH-; R¹⁵-S(=O)ₙ-NH-; R¹⁵-O-S(=O)ₙ-NH-; R¹⁵-C(=S)-NH-; R¹⁵-O-C(=S)-NH-; R¹⁷R¹⁸N-Y₁ₐ-; R¹⁷R¹⁸-Y₂-NR¹⁶-Y₁-; R¹⁵-Y₂-NR¹⁹-Y₁-; H-Y₂-NR¹⁹-Y₁-;
- R³: is hydrogen; hydroxy; halo; C₁₋₆alkyl; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷ -S(=O)ₚ-; R⁷ -S(=O)ₚ-NH-; R²¹-S(=O)ₚ-NH-; R⁷-C(=O)-; -NHC(=O)H; -C(=O)NHNH₂;R⁷-C(=O)-NH-; R²¹-C(=O)-NH-; -C(=NH)R⁷; -C(-NH)R²¹;
- R^{4a} or R^{4b}: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁵ and R⁶: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said groups representing R⁸ may optionally be substituted with one or more substituents selected from R¹², R¹³ and R¹⁴;
- R⁹, R¹⁰ and R¹¹: each independently are hydrogen or R⁸;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R¹⁵R¹⁶-S(=O)-; R¹⁵R¹⁶N-S(=O)₂-; R¹⁷R¹⁸-Y₁-; R¹⁷R¹⁸N-Y₂-NR¹⁶-Y₁-; R¹⁵-Y₂-NR¹⁹-Y₁-; H-Y₂-NR¹⁹-Y₁-; oxo;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹² R¹³ and R¹⁴.
- R¹⁶, R¹⁷, R¹⁸ and R¹⁹: each independently are hydrogen or R¹⁵;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹² , R¹³ and R¹⁴;
- Y₁ₐ: is -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or-Y₃-C(=O)-O-Y₄-;
- Y₁ or Y₂: each independently are a direct bond, -Y₃-S(=O)-Y₄-; -Y₃-S(=O)₂-Y₄-, -Y₃-C(=O)-Y₄a-, -Y₃-C(=S)-Y₄-, -Y₃-O-Y₄-, -Y₃-S-Y₄-, -Y₃-O-C(=-O)-Y₄- or -Y₃-C(=O)-O-Y₄-;
- Y₃ or Y₄: each independently are a direct bond, C₁₋₆alkanediyl, C₂₋₆alkenediyl or C₂₋₆alkynediyl;
- n is: 1 or 2;
- m: is 1 or 2;
- p is: 1 or 2;
- r is: 1 to 5;
- s: is 1 to 3;
- aryl: is phenyl or phenyl substituted with one, two, three, four or five substituents each independently selected from halo, C₁₋₆alkyl, C₃₋₇cycloalkyl, C₁₋₆alkyloxy, cyano, nitro, polyhaloC₁₋₆alkyl and polyhaloC₁₋₆alkyloxy;
provided that -X-R² and/or R³ is other than hydrogen; and
provided that the following compounds
benzamide, 4-[(5-cyano-4-phenyl-2-pyrimidinyl)amino]-N [2-(diethylamino)ethyl]-;
benzamide, 4-[[4-[6-(1-piperazinyl)-3-pyridinyl]-2-pyrimidinyl]amino]-;
benzamide, *N*-methyl-4-[[4-(3-pyridinyl)-2-pyrimidinyl]amino]-;
benzamide, 4-[[4-[(3-methoxyphenyl)thio]-2-pyrimidinyl]amino]-*N-*[2-(1-pyrrolidinyl)ethyl]-;
benzamide, *N*-[2-(diethylamino)ethyl]-4-[[4-(3-pyridinyl)-2-pyrimidinyl]amino]-;
benzamide, 4-[(5-amino-1,4-dihydro-4-oxo-2-pyrimidinyl)amino]-N,N-dimethyl-;
benzamide, 4-[(5-amino-1,4-dihydro-4-oxo-2-pyrimidinyl)amino]-NN-diethyl-;
benzamide, 4-[(5-amino-1,4-dihydro-4-oxo-2-pyrimidinyl)amino]-N methyl-;
benzamide, 4-[[5-(4-methoxyphenyl)-2-pyrimidinyl]amino]-;
benzamide, 4-[[1-oxido-4-[(2,4,6-trimethylphenyl)amino]-2-pyrimidinyl]amino]-;
benzamide, 4-[[3-oxido-4-[(2,4,6-trimethylphenyl)amino]-2-pyrimidinyl]amino]-;
benzamide, 4-[[4-[(2,4,6-trimethylphenyl)amino]-2-pyrimidinyl]amino]-;
benzamide, 2-[[4-nnethyl-6-(trifluoromethyl)-2-pyrimidinyl]amino]-;
benzamide, *N*-(3-aminopropyl)-3-[[4-(3-pyridinyl)-2-pyrimidinyl]amino]-;
benzamide, *N*- (3-hydroxypropyl)-3-[[4-[2-[(3-hydroxypropyl)amino]-4-pyridinyl]-2-pyrimidinyl]amino]-;
benzamide, *N*-(3-aminopropyl)-3-[[4-[2-[(3-hydroxypropyl)amino]-4-pyridinyl]-2-pyrimidinyl]amino]-;
benzamide, 3-[[4-[2-[(3-hydroxypropyl)amino]-4-pyridinyl]-2-pyrimidinyl]amino]-N [2-(1*H*-imidazol-4-yl)ethyl]-;
benzamide, 4,4'-[(6-methyl-S-nitro-2,4-pyrimidinediyl)diimino]bis-;
benzamide, 4-[[5-amino-4-(methylamino)-2-pyrimidinyl]amino]-*N,N*-diethyl-;
benzamide, *N,N*-diethyl-4-[[4-(methylamino)-5-nitro-2-pyrimidinyl]amino]-are not included.

Further interesting compounds are those compounds of formula (I) or (I') as defined hereinabove wherein Z represents O;
ring A is pyrimidinyl;
- R¹: is hydrogen;
- X: is -NR¹-; -O-; -O-C₁₋₆alkyl-; -NR¹-C₁₋₆alkyl- or a direct bond;
- R²: is hydrogen, C₁₋₁₀alkyl, R²⁰, each of said groups representing R² may optionally be substituted where possible with one or more substituents each independently being selected from R¹⁵; cyano; R¹⁵-O-; R⁵R⁶N-C(=O)-;
- R³: is hydrogen; halo;cyano; nitro; amino; R²¹-C₁₋₆alkyl;
- R⁴⁸ or R^{4b}: each independently are hydrogen or R⁸;
- R⁵ and R⁶: are hydrogen;
- R⁸: is C₁₋₆alkyl;
- R¹², R¹³ and R¹⁴: are R¹⁵;
- R¹⁵: is C₁₋₆alkyl; a monocyclic, bicyclic or tricyclic aromatic carbocycle; a monocyclic, bicyclic or tricyclic aromatic heterocycle; C₁₋₆alkyl substituted with a monocyclic, bicyclic or tricyclic aromatic carbocycle;
- R²⁰: is a monocyclic, bicyclic or tricyclic aromatic carbocycle;
- R²¹: is a monocyclic, bicyclic or tricyclic aromatic carbocycle;
- s: is 1 or 2;
provided that X-R² and/or R³ is other than hydrogen; and
provided that the following compounds
benzamide, 4-[(5-amino-1,4-dihydro-4-oxo-2-pyrimidinyl)amino]-*N*,*N*-dimethyl-;
benzamide, 4-[(5-amino-1,4-dihydro-4-oxo-2-pyrimidinyl)amino]-*N*,*N*-diethyl-;
benzamide, 4-[(5-amino-1,4-dihydro-4-oxo-2-pyrimidinyl)amino]-*N*-methyl-;
benzamide, 4-[[1-oxido-4-[(2,4,6-trimethylphenyl)amino]-2-pyrimidinyl]amino]-;
benzamide, 4-[[3-oxido-4-[(2,4,6-ttimethylphenyl)amino]-2-pyrimidinyl]amino]-;
benzamide, 4-[[4-[(2,4,6-trimethylphenyl)amino]-2-pyrimidinyl]amino]-;
benzamide, 4-[[5-amino-4-(methylamino)-2-pyrimidinyl]amino]-*N,N* diethyl-;
benzamide, *N,N*-diethyl4-[[4-(methylamino)-5-nitro-2-pyrimidinyl]amino]-are not included.

Yet further particular interesting compounds are those compounds of formula (I) or (I') as defined hereinabove provided that the compound is other than
a) 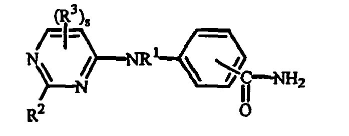 wherein
   R² is hydrogen, trifluoromethyl, C₁₋₄alkyl; R³ is hydrogen, C₁₋₄alkyl, hydroxyC₁₋₄alkyl, amino, C₁₋₄alkylcarbonyl, or phenylC₁₋₄alkyl wherein phenyl may optionally be substituted or R² is NR^{2'}R^{2"} with R^{2'} and R^{2"} each independently representing hydrogen or C₁₋₄alkyl or optionally substituted phenyl; a monocyclic, bicyclic or tricyclic saturated heterocycle; a monocyclic, bicyclic or tricyclic partially saturated heterocycle; a monocyclic, bicyclic or tricyclic aromatic heterocycle, provided that when R² is a monocyclic, bicyclic or tricyclic saturated heterocycle or a monocyclic, bicyclic or tricyclic partially saturated heterocycle or a monocyclic, bicyclic or tricyclic aromatic heterocycle then at least one N atom is present and R² is bound to the pyrimidinyl ring via a nitrogen atom; R¹ is hydrogen or C₁₋₄alkyl and s is defined as hereinabove;
b) 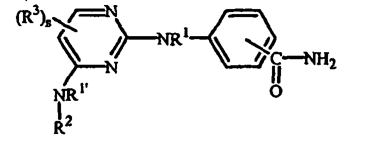 wherein R¹ is hydrogen, C₁₋₄alkyl or optionally substituted phenyl; R^{1'} is hydrogen or C₁₋₄alkyl; R² is optionally substituted phenyl; R³ is hydrogen, C₁₋₄alkyl, hydroxyC₁₋₄alkyl, amino, C₁₋₄alkylcarbonyl, or phenylC₁₋₄alkyl wherein phenyl may optionally be substituted and s is as defined hereinabove;
C) 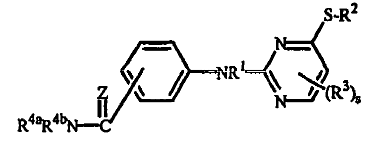 wherein
   R^{4a} and R^{4b} each independently are as defined hereinabove; R² is C₁₋₁₀alkyl;
   C₂₋₁₀alkenyl; C₂₋₁₀alkynyl; monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said groups representing R² may optionally be substituted; R¹ is hydrogen or C₁₋₆alkyl; R³ and s are as defined hereinabove;
d) 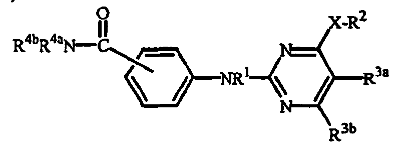 wherein
   R¹ is as defined hereinabove; R^{4a} and R^{4b} each independently are hydrogen or methyl; X is a direct bond, -C₁₋₆alkyl-, NR¹-, NH-NH-, -N=N-, -O-, -C(=O)-, -CHOH-, -S-, -S(=O)-, -S(=O)₂-, -O-C₁₋₄alkyl-, -NR¹-C₁₋₄alkyl-, -S-C₁₋₄alkyl-; R² is C₁₋₁₀alkyl, C₂₋₁₀alkenyl, C₂₋₁₀alkynyl, C₃₋₇cycloalkyl, phenyl, pyridyl, pyrimidinyl, pyrazinyl, pyridazinyl, said groups representing R² may optionally be substituted; R^{3a} represents hydrogen, hydroxy, halo, C₁₋₆alkyl, C₃₋₇cycloalkyl, C₂₋₆alkenyl optionally substituted with one or more halogen atoms, C₂₋₆alkynyl optionally substituted with one or more halogen atoms, C₁₋₆alkyl substituted with cyano or -C(=O)R⁷, C₁₋₆alkyloxy, C₁₋₆alkyloxycarbonyl, carboxyl, cyano, nitro, amino, mono- or di(C₁₋₆alkyl)amino, polyhalomethyl, polyhalomethyloxy, polyhalomethylthio, -S(=O)ₚR⁷, -NH-S(=O)ₚR⁷, -C(=O)R⁷, -NHC(=O)H, -C(=O)NHNH₂, -NHC(=O)R⁷,-C(=NH)R⁷ or aryl; R^{3b} is hydroxy, cyano, carboxyl, halo, cyanoC₁₋₆alkyl, hydroxyC₁₋₆alkyl, aminocarbonyl, mono -or di(C₁₋₄alkyl)aminocarbonyl, C₁₋₆alkyloxy, C₁₋₆alkylthio, C₁₋₆alkyl-S(=O)ₚ, C₁₋₆alkylcarbonyl, C₁₋₆alkyloxycarbonyl, C₁₋₆alkylearbonyloxy, C₂₋₆alkenyl, C₂₋₆alkynyl, polyhaloC₁₋₆alkyl, imidazolyl, thiazolyl, oxazolyl, pyrazolyl, isothiazolyl, isoxazolyl, triazolyl, tetrazolyl optionally substituted with imino, a 5-membered heteroaromatic ring, imidazolidinyl, pyrazolidinyl, thiazolidinyl, isothiazolidinyl, oxazolidinyl, isoxazolidinyl optionally substituted with hydroxy, isoxazolidinone, or a radical of formula 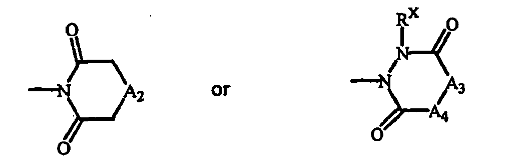 with A₂ being O, CH₂ or a direct bond;
   A₃ being CH₂ or NH;
   A₄ being CH₂ or a direct bond; or A₃-A₄ representing CH=CH;
   R^{x} being hydrogen or C₁₋₄alkylcarbonyl.
e) 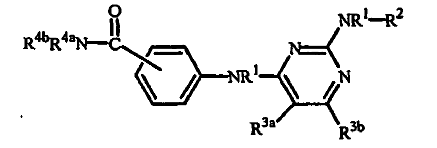 wherein
   R¹ is as defined hereinabove; R⁴⁸ and R^{4b} each independently are hydrogen or methyl;
   R² is phenyl, pyridyl, pyrimidinyl, pyrazinyl, pyridazinyl, said groups representing R² may optionally be substituted; R^{3a} represents hydrogen, hydroxy, halo, C₁₋₆alkyl, C₃₋₇cycloalkyl, C₂₋₆alkenyl optionally substituted with one or more halogen atoms, C₂₋₆alkynyl optionally substituted with one or more halogen atoms, C₁₋₆alkyl substituted with cyano or -C(=O)R⁷, C₁₋₆alkyloxy, C₁₋₆alkyloxycarbonyl, carboxyl, cyano, nitro, amino, mono- or di(C₁₋₆alkyl)amino, polyhalomethyl, polyhalomethyloxy, polyhalomethylthio, -S(=O)ₚR⁷, -NH-S(=O)ₚR⁷, -C(=O)R⁷, -NHC(=O)H, -C(=O)NHNH₂, -NHC(=O)R⁷,-C(=NH)R⁷ or aryl; R^{3b} is hydroxy, cyano, carboxyl, halo, cyanoC₁₋₆alkyl, hydroxyC₁₋₆alkyl, aminocarbonyl, mono -or di(C₁₋₄alkyl)aminocarbonyl, C₁₋₆alkyloxy, C₁₋₆alkylthio, C₁₋₆alkyl-S(=O)ₚ, C₁₋₆alkylcarbonyl, C₁₋₆alkyloxycarbonyl, C₁₋₆alkylcarbonyloxy, C₂₋₆alkenyl, C₂₋₆alkynyl, polyhaloC₁₋₆alkyl, imidazolyl, thiazolyl, oxazolyl, pyrazolyl, isothiazolyl, isoxazolyl, triazolyl, tetrazolyl optionally substituted with imino, a 5-membered heteroaromatic ring, imidazolidinyl, pyrazolidinyl, thiazolidinyl, isothiazolidinyl, oxazolidinyl, isoxazolidinyl optionally substituted with hydroxy, isoxazolidinone, or a radical of formula 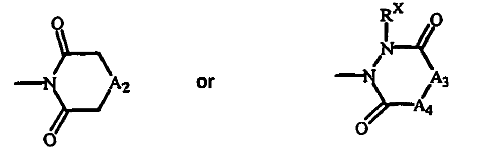 with A₂ being O, CH₂ or a direct bond;
   A₃ being CH₂ or NH;
   A₄ being CH₂ or a direct bond; or A₃-A₄ representing CH=CH;
   R^{x} being hydrogen or C₁₋₄alkylcarbonyl.
f) 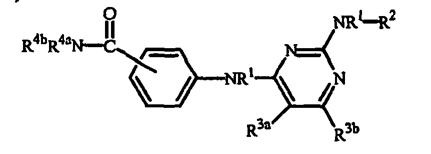 wherein
   R¹ is as defined hereinabove; R^{4a} and R^{4b} each independently are hydrogen or methyl; R² is phenyl, pyridyl, pyrimidinyl, pyrazinyl, pyridazinyl, said groups representing R² are substituted in para position (compared to the NR¹ linker) and optionally substituted in ortho or meta position (compared to the NR¹ linker); R^{3a} is hydroxy, halo, C₃₋₇cycloalkyl, C₂₋₆alkenyl optionally substituted with one or more halogen atoms, C₂₋₆alkynyl optionally substituted with one or more halogen atoms, C₁₋₆alkyl substituted with cyano or -C(=O)R⁷, C₁₋₆alkyloxy, C₁₋₆alkyloxycarbonyl, carboxyl, cyano, nitro, amino, mono- or di(C₁₋₆alkyl)amino, polyhalomethyl, polyhalomethyloxy, polyhalomethylthio, -S(=O)ₚR⁷, -NH-S(=O)ₚR⁷, -C(=O)R⁷, -NHC(=O)H, -C(=O)NHNH₂, -NHC(=O)R⁷,-C(=NH)R⁷ or aryl; R^{3b} is hydrogen, halo, C₁₋₆alkyl, polyhaloC₁₋₆alkyl, amino, mono -or di(C₁₋₆alkyl)amino, optionally substituted pyrrolidinyl, piperidinyl, morpholinyl, R⁷-C(=O)-NH-;
g) 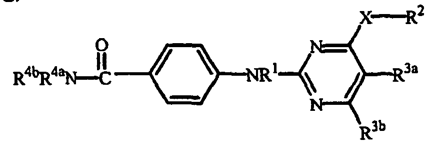 wherein
   R¹ is as defined hereinabove; R^{4a} and R^{4b} each independently are hydrogen or methyl; R² is C₁₋₁₀alkyl, C₂₋₁₀alkenyl, C₂₋₁₀alkynyl, C₃₋₇cycloalkyl, phenyl, pyridyl, pyrimidinyl, pyrazinyl, pyridazinyl, said groups may optionally be substituted; X is a direct bond, -C₁₋₆alkyl-, -NR¹-, NH NH-, N N-, -O-, -C(=O)-, -CHOH-, -S-, -S(=O)ₚ-, -O-C₁₋₄alkyl-, -NR¹-C₁₋₄alkyl-, -S-C₁₋₄alkyl-; R^{3a} represents hydroxy, halo, C₃₋₇cycloalkyl, C₂₋₆alkenyl optionally substituted with one or more halogen atoms, C₂₋₆alkynyl optionally substituted with one or more halogen atoms, C₁₋₆alkyl substituted with cyano or -C(=O)R⁷, C₁₋₆alkyloxy, C₁₋₆alkyloxycarbonyl, carboxyl, cyano, nitro, amino, mono- or di(C₁₋₆alkyl)amino, polyhalomethyl, polyhalomethyloxy, polyhalomethylthio,
   -S(=O)ₚR⁷, -NH-S(=O)ₚR⁷, -C(=O)R⁷, -NHC(=O)H, -C(=O)NHNH₂,
   -NHC(=O)R⁷,-C(=NH)R⁷ or aryl; R^{3b} is hydrogen, halo, C₁₋₆alkyl, polyhaloC₁₋₆alkyl, amino, mono -or di(C₁₋₆alkyl)amino, optionally substituted pyrrolidinyl, piperidinyl, morpholinyl, R⁷-C(=O)-NH-;
h) 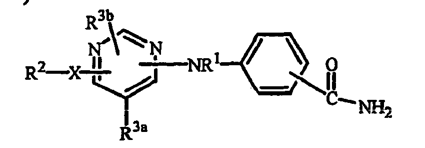 wherein
   R¹ is as defined hereinabove; R^{3a} is hydrogen or C₁₋₄alkyl; R^{3b} is hydrogen, amino, mono -or di(C₁₋₆alkyl)amino, optionally substituted pyrrolidinyl, piperidinyl, morpholinyl, R⁷-C(=O)-NH-; X is a direct bond, -C₁₋₁₀alkyl-, -NR¹-, -NH-NH-, -N=N-, -O-, -C(=O)-, -CHOH-, -S-, -S(=O)ₚ,-, -O-C₁₋₄alkyl-, -NR¹-C₁₋₄alkyl-, -S-C₁₋₄alkyl-; R² is C₁₋₁₀alkyl, C₂₋₁₀alkenyl, C₂₋₁₀alkynyl, C₃₋₇cycloalkyl, indanyl, indolyl, phenyl, pyridyl, pyrimidinyl, pyrazinyl, pyridazinyl, said groups representing R² may optionally be substituted;
i) 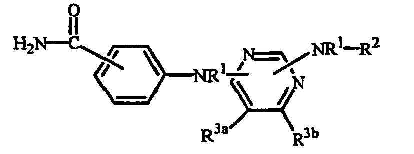 wherein
   R¹ is as defined hereinabove; R² is phenyl or pyridyl, each of said groups representing R² may optionally be substituted with hydroxy, halo, C₁₋₆alkyl, C₁₋₆alkyloxy, cyano, aminocarbonyl, nitro, amino, trihalomethyl, trihalomethyloxy or C₁₋₆alkyl substituted with cyano or aminocarbonyl; R^{3a} is hydrogen or C₁₋₄alkyl; R^{3b} is hydrogen, amino, mono -or di(C₁₋₆alkyl)amino, optionally substituted pyrrolidinyl, piperidinyl, morpholinyl, R⁷ -C(=O)-NH-;
j) 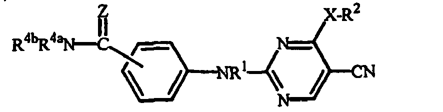 wherein
   R¹ is hydrogen or C₁₋₆alkyl; R^{4a} and R^{4b} each independently are hydrogen; optionally substituted C₁₋₆alkyl; optionally substituted C₂₋₆alkenyl; optionally substituted C₂₋₆alkynyl; an optionally substituted monocyclic, bicyclic or tricyclic aromatic carbocycle or or an optionally substituted monocyclic, bicyclic or tricyclic aromatic heterocycle, X-R² is as defined hereinabove;
k) 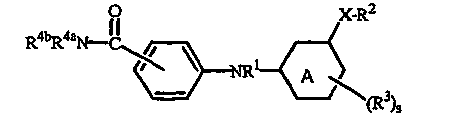 wherein
   R^{4a} and R^{4b} each independently represent hydrogen, C₁₋₆alkyl, phenyl, naphthyl, C₁₋₆alkyl substituted with phenyl or naphthyl, wherein phenyl or naphthyl may optionally be substituted with halo, C₁₋₆alkyl, C₁₋₆Alkyloxy, nitro or carboxyl; R¹ is hydrogen; phenyl optionally substituted with halo; C₁₋₆alkyl; C₁₋₆alkyloxy; nitro or carboxyl; X is -0-, -NH-, -N-C₁₋₆alkyl-, -S-, -OCH₂-; R² is phenyl optionally substituted with halo, C₁₋₄alkyl, C₁₋₆alkyloxy, nitro or carboxyl; ring A is pyrimidinyl, pyridyl, pyrazinyl; s and R³ are as defined hereinabove;
1) 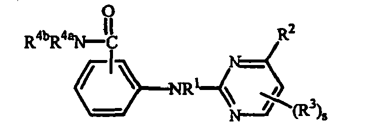 wherein
   R^{4a} and R^{4b} each independently are hydrogen or C₁₋₃alkyl; R² is 2-pyridyl, 3-pyridyl, 4-pyridyl, 2-methyl-3-pyridyl, 4-methyl-3-pyridyl, 2-furyl, 5-methyl-2-furyl, 2,5-dimethyl-3-furyl, 2-thienyl, 3-thienyl, 5-methyl-2-thienyl, 2-phenothiazinyl, 4-pyrazinyl, 2-benzofuryl, N-oxido-2-pyridyl, N-oxido-3-pyridyl, N-oxido-4-pyridyl, 1H-indol-2-yl, 1H-indol-3-yl, 1-methyl-1H-pyrrol-2-yl, 4-quinolinyl, 1-methyl-pyridinium-4-yliodide; R¹ is hydrogen or C₁₋₃alkyl; R³ is hydrogen or C₁₋₃alkyl; s is as defined hereinabove;
m) 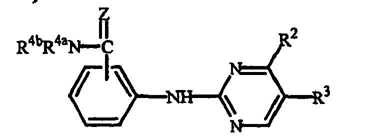 wherein Z, R^{4a}, R^{4b} are as defined hereinabove; R² is pyridyl substituted with optionally substituted saturated heterocycle containing from 3 to 7 atoms; R³ is hydrogen; halo; C₁₋₆alkyl; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; polyhaloC₁₋₆alkyl; polyhaloC₁₋₆alkyloxy; polyhaloC₁₋₆alkylthio; R²¹-C₁₋₆alkyl; R⁷-S(=O)ₚ-; R⁷-S(=O)ₚNH-; R⁷-C(=O)-; R⁷-C(=O) NH-; -C(=NH)R⁷ with R⁷ representing C₁₋₆alkyl, C₁₋₆alkyloxy or polyhaloC₁₋₆alkyl;
n) 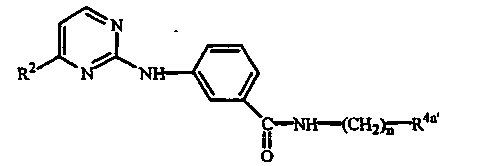 wherein R² is 4-pyridyl substituted in position 3; n is 2 or 3; R^{4a}' is hydroxy, amino, imidazolyl or di(C₁₋₃alkyl)amino;
o) 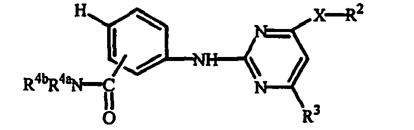 wherein R^{4a} and R^{4b} each independently are hydrogen or C₁₋₄alkyl; R³ is hydrogen, C₁₋₃alkyl, polyhaloC₁₋₃alkyl, halo, or polyhaloC₁₋₃alkyloxy; -X-R² is C₁₋₆alkyl, C₁₋₄alkyloxy, C₁₋₄alkylthio, C₁₋₄alkyl-S(=O)ₚ-, polyhaloC₁₋₄alkyl, polyhaloC₁₋₄alkyloxy, phenyl optionally substituted with up to three substituents selected from halo or C₁₋₄alkyl or polyhaloC₁₋₄alkyl or C₁₋₄alkyloxy, 2-furanyl, 2-thienyl, 3-thienyl, C₃₋₆cycloalkyl optionally substituted with up to three substituents selected from C₁₋₄alkyl, polyhaloC₂₋₄alkenyl, C₁₋₄alkyloxyC₁₋₄alkyl, C₁₋₄alkyloxyC₁₋₄alkyloxy, polyhaloC₁₋₄alkylthio; or X-R² is hydrogen when R³ is cyano;
P) 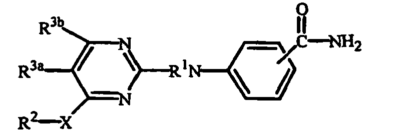 wherein R¹ is as defined hereinabove; -X-R² is pyridyl, pyrimidinyl, thiazolyl, pyrazinyl, pyridazinyl, imidazolyl, phenyl, each of said rings optionally substituted with one or more substituents selected from halo, cyano, aminocarbonyl, -C(=O)-O-R^{2'}, -C(=O)-R^{2'}, -S(=O)₂-NR^{2'}R^{2"}, NR^{2'}R^{2"}, -O-R^{2'} or C₁₋₆alkyl optionally substituted with fluoro with R^{2'} and R^{2"} each independently representing hydrogen or C₁₋₆alkyl optionally substituted with mono- or di(C₁₋₆alkyl)amino; or with X-R² is hydrogen, hydroxy, C₁₋₆alkyl, C₁₋₆alkyloxy; R^{3a} and R^{3b} each independently are pyridyl, pyrimidinyl, thiazolyl, pyrazinyl, pyridazinyl, imidazolyl, phenyl, each of said rings representing R^{3a} and R^{3b} may optionally be substituted with one or more substituents selected from halo, cyano, aminocarbonyl, -C(=O)-O-R^{3'}, -C(=O)-R^{3'}, -S(=O)₂-NR^{3'}R^{3"}, NR^{3'}R^{3"}, -O-R^{3'} or C₁₋₆alkyl optionally substituted with fluoro with R^{3'} and R^{3"} each independently representing hydrogen or C₁₋₆alkyl optionally substituted with mono- or di(C₁₋₆alkyl)amino; or R^{3a} and R^{3b} are hydrogen, hydroxy, C₁₋₆alkyl, C₁₋₆alkyloxy;
g) 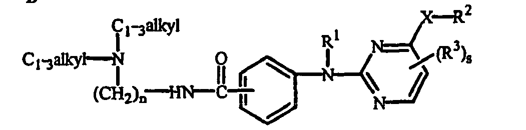 wherein n is 2 or 3; R¹ is hydrogen or C₁₋₃alkyl; s is 1 or 2; -X-R² is hydrogen, C₁₋₃alkyl, 2-pyridyl, 3-pyridyl, 4-pyridyl, 2-methyl-3-pyridyl, 6-methyl-3-pyridyl, 2-furanyl, 5-methyl-2-furanyl, 2,5-dimethyl-3-furanyl, 2-thienyl, 3-thienyl, 5-methyl-2-thienyl, 2-pheno-thiazinyl, 2-pyrazinyl, 2-benzofuranyl, 2-pyridyl-N-oxide, 3-pyridyl-N-oxide, 4-pyridyl-N-oxide, 1H-indol-2-yl, 1H-indol-3-yl, 1-methyl-1H-pyrrol-2-yl, 4-quinolinyl, 4-pyridyl methyl iodide, dimethylaminophenyl; R³ is hydrogen, C₁₋₃alkyl, 2-pyridyl, 3-pyridyl, 4-pyridyl, 2-methyl-3-pyridyl, 6-methyl-3-pyridyl, 2-furanyl, 5-methyl-2-furanyl, 2,5-dimethyl-3-furanyl, 2-thienyl, 3-thienyl, 5-methyl-2-thienyl, 2-pheno-thiazinyl, 2-pyrazinyl, 2-benzofuranyl, 2-pyridyl-N-oxide, 3-pyridyl-N-oxide, 4-pyridyl-N-oxide,1H-indol-2-yl, 1H-indol-3-yl, 1-methyl-1H-pyrrol-2-yl, 4-quinolinyl, 4-pyridyl methyl iodide, dimethylaminophenyl.

Further preferred compounds are those compounds of formula (I) or (I') wherein one or where possible more of the following restrictions apply:
a) X is a direct bond and R² is hydrogen;
b) R² and R³ are other than hydrogen;
c) R³ is hydrogen;
d) X is other than a direct bond;
e) the moiety -C(=Z)NR^{4a}R^{4b} is placed at the meta position compared to the NR¹ linker;
f) R³ when placed at the para position compared to the NR¹ linker is other than cyano;
g) X is other than NR¹ or S;
h) X is other than a direct bond and R² is other than hydrogen;
i) when X is NR¹ than R^{4a} and/or R^{4b} are/is other than hydrogen;
j) ring A is pyridyl, pyrimidinyl or pyridazinyl.

Also preferred are those compounds of formula (I) or (I') wherein the compounds are compounds of the following formula:

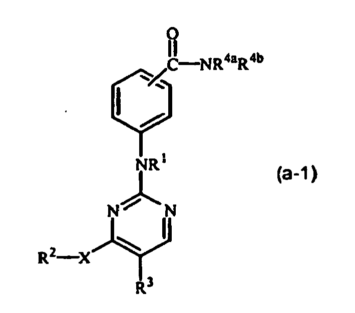

The present invention relates in particular to those compounds of formula (I) or (I') wherein the compounds are compounds of the following formula

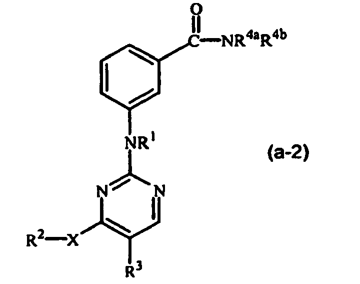

Also preferred are those compounds of formula (a-1) wherein one or where possible more of the following restrictions apply :
(a) X is other than a direct bond, S or C₁₋₆alkyl;
(b) R² is other than optionally substituted phenyl when X is NR¹;
(c) R³ is hydrogen;
(d) R³ is other than hydrogen, cyano or C₁₋₄alkyl;
(e) R² is other than optionally substituted phenyl;
(f) -X-R² is other than hydrogen, hydroxy, C₁₋₆alkyl, C₁₋₆alkyloxy, C₁₋₄alkylthio, C₁₋₄alkyl-S(=O)ₚ-, polyhaloC₁₋₄alkyl, polyhaloC₁₋₄alkyloxy;
(g) R^{4b} is other than -(CH₂)ₙ-N(C₁₋₃alkyl)₂ with n being 2 or 3 when R^{4a} is hydrogen;
(h) X-R² and R³ are other than hydrogen;
(i) R² is R²⁰.

Also preferred are those compounds of formula (a-2) wherein the following restriction applies :
(a) X-R² and R³ are other than hydrogen.

Particular preferred compounds are those compounds of formula (a-1) wherein
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is -NR¹-; -NH-NH-; -N=N-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -O-C(=O)-C(=O)-; -C(=O)-NR¹-, -NR¹-C(=O)-; -C(=S)-NR¹-, NR¹-C(=S)-; NR¹-C(=O)-NR¹-; -NR¹-C(=S)-NR¹-; -NR¹-S(=O)-NR¹-; -NR¹-S(=O)₂-NR¹-; -C₁₋₆alkyl-C(=O)-NR¹-; -O-C₁₋₆alkyl-C(=O)-NR¹; -C₁₋₆alkyl-O-C(-O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -NR¹-C₂₋₆alkenyl-C₃₋₇cycloalkyl-; -O-C₂₋₆alkynyl-; -C₂₋₆alkynyl-O-; NR¹-C₂₋₆alkynyl-; -C₂₋₆alkynyl-NR¹-; -NR¹-C₂₋₆alkynyl-NR¹-; -NR¹-C₂₋₆alkynyl-C₃₋₇cycloalkyl-; -O-C₁₋₆alkyl-O-; -O-C₂₋₆alkenyl-O-; -O-C₂₋₆alkynyl-O-; -CHOH-; -S(=O)-; -S(=O)₂-; -S(=O)-NR¹-; -S(=O)₂-NR¹-; -NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂-;
- R²: is hydrogen, C₁₋₁₀alkyl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(=O)-_{;} R¹⁵-C(=O)-O-; R¹⁵-O-C(=O)-O-; -SO₃H; R¹⁵-S(=O)-; R¹⁵-S(=O)₂-; R⁵R⁶N; R⁵R⁶-C₁₋₆alkyl; R⁵R⁶N-C₃₋₇cycloalkyl; R⁵R⁶N-C₁₋₆alkyloxy; R⁵R⁶N-C(=O)-; R⁵R⁶N-C(=S)-; R⁵R⁶N-C(=O)-NH-; R⁵R⁶-C(=S)-NH-; R⁵R⁶N-S(=O)ₙ-; R⁵R⁶N-S(=O)ₙ-NH-; R¹⁵-C(=S)-; R¹⁵-C(=O)-NH-; R¹⁵-O-C(=O)-NH-; R¹⁵-S(=O)ₙ-NH-; R¹⁵-O-S(=O)ₙ-NH-; R¹⁵-C(=S)-NH-; R¹⁵-O-C(=S)-NH-; R¹⁷R¹⁸N-Y₁ₐ-; R¹⁷R¹⁸N-Y₂-NR¹⁶-Y₁-; R¹⁵-Y₂-Y¹⁹-Y₁-; H-Y₂-NR¹⁹-Y₁-;
- R³: is hydroxy; halo; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷-S(=O)ₚ-; R⁷-S(=O)ₚ-NH-; R²¹-S(=O)ₚ-NH-; R⁷-C(=O)-; -NHC(=O)H; -C(=O)NHNH₂;R⁷-C(=O)-NH-; R²¹-C(=O)-NH-; -C(=NH)R⁷; -C(=NH)R²¹;
- R^{4a} or R^{4b}: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁵ and R⁶: each independently are hydrogen, R⁸, -Y₁-NR⁹-Y₂-NR¹⁰R¹¹, -Y₁-NR⁹-Y₁-R⁸, -Y₁-NR⁹R¹⁰, or
- R⁵ and R⁶: may together with the nitrogen to which they are attached form a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴, or each of said heterocycles may optionally be fused with a benzene ring, said benzene ring being optionally substituted with one or more substituents selected from R¹², R¹³ and R¹⁴;
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle;
- R⁹, R¹⁰ and R¹¹: each independently are hydrogen or R⁸, or
- any: two of R⁹, R¹⁰ and R¹¹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(-O)-; R¹⁵-C(=O)-O-; R¹⁵-O-C(=O)-O-; -SO₃H; R¹⁵-S(=O)-; R¹⁵-S(=O)₂-; R¹⁵R¹⁶N-S(=O)-; R¹⁵R¹⁶N-S(=O)₂-; R¹⁷R¹⁸N-Y₁-; R¹⁷R¹⁸N-Y₂-NR¹⁶-Y₁-; R¹⁵-Y₂-Y¹⁹-Y₁-; H-Y₂-NR¹⁹-Y₁-; oxo, or
- any: two of R¹², R¹³ and R¹⁴ may together be C₁₋₆alkanediyl or C₂₋₆alkenediyl thereby forming a saturated or partially saturated monocyclic 3 to 8 membered carbo - or heterocycle or an aromatic 4 to 8 membered monocyclic carbo - or heterocycle together with the atoms to which they are attached, or
- any: two of R¹² , R¹³ and R¹⁴ may together be -O-(CH₂)ᵣO- thereby forming a saturated, partially saturated or aromatic monocyclic 4 to 8 membered carbo - or heterocycle together with the atoms to which they are attached;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴; or each of said carbocycles or heterocycles may optionally be fused with a benzene ring, said benzene ring being optionally substituted with one or more substituents selected from R¹², R¹³ and R¹⁴;
- R¹⁶, R¹⁷, R¹⁸ and R¹⁹: each independently are hydrogen or R¹⁵, or
- R¹⁷ and R¹⁸, or R¹⁵ and R¹⁹: may together be C₁₋₆alkanediyl or C₂₋₆alkenediyl thereby forming a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴; or
- R¹⁷ and R¹⁸: together with R¹⁶ may be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
- Y₁ₐ: is -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₁ or Y₂: each independently are a direct bond, -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₃ or Y₄: each independently are a direct bond, C₁₋₆alkanediyl, C₂₋₆alkenediyl or C₂₋₆alkynediyl;
- n: is 1 or 2;
- m: is 1 or 2;
- p is: 1 or 2;
- r: is 1 to 5;
- s: is 1 to 3;
- aryl: is phenyl or phenyl substituted with one, two, three, four or five substituents each independently selected from halo, C₁₋₆alkyl, C₃₋₇cycloalkyl, C₁₋₆alkyloxy, cyano, nitro, polyhaloC₁₋₆alkyl and polyhaloC₁₋₆alkyloxy;
provided
benzamide, 4-[[5-amino-4-(methylamino)-2-pyrimidinyl]amino]-*N,N*-diethyl-; and benzamide, *N,N*-diethyl-4-[[4-(methylamino)-5-nitro-2-pyrimidinyl]amino]-are not included.

Further particular preferred compounds are those compounds of formula (a-2) wherein
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is NR¹-; -NH-NH-; -N=N-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -C-C(=O)-C(=C)-; -C(=O)-NR¹-, -NR¹-C(=O); -C(=S)-NR¹-, -NR¹-C(=S)-; -NR¹-C(=O)-NR¹-; NR¹-C(=S)-NR¹-; -NR¹-S(=O)-NR¹-; -NR¹-S(=O)₂-NR¹-; -C₁₋₆alkyl-C(=C)-NR¹-; O-C₁₋₆alkyl-C(=C)NR¹-; -C₁₋₆alkyl-O-C(=O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -NR¹-C₂₋₆alkenyl-C₃₋₇cycloalkyl-; -O-C₂₋₆alkynyl-; -C₂₋₆alkynyl-O-; -NR¹-C₂₋₆alkynyl-; -C₂₋₆alkynyl-NR¹-; NR¹-C₂₋₆alkynyl-NR¹-; -NR¹-C₂₋₆alkynyl-C₃₋₇cycloalkyl-; -O-C₁₋₆alky-O-; -O-C₂₋₆alkenyl-O-; -O-C₂₋₆alkynyl-O-; -CHOH-; -S(=O)-; -S(=O)₂-; -S(=O)-NR¹-; -S(=O)₂-NR¹-; -NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂-;
- R²: is hydrogen, C₁₋₁₀alkyl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R⁵R⁶N; R⁵R⁶-C₁₋₆alkyl; R⁵R⁶N-C₃₋₇cycloalkyl; R⁵R⁶N-C₁₋₆alkloxy; R⁵R⁶N-C(=O)-; R⁵R⁶N-C(=S)-; R⁵R⁶N-C(=O)-NH-; R⁵R⁶N-C(=S)-NH-; R⁵R⁶N-S(=O)ₙ-; R⁵R⁶N-S(=O)ₙ- NH-; R¹⁵-C(=S)-; R¹⁵-C(=O)-NH-; R¹⁵-O-C(=O)-NH-; R¹⁵-S(=O)ₙ-NH-; R¹⁵-O-S(=O)ₙ-NH-; R¹⁵-C(=S)-NH-; R¹⁵-O-C(=S)-NH-; R¹⁷R¹⁸N-Y₁ₐ-; R¹⁷R¹⁸N-Y₂-NR¹⁶⁻Y₁-; R¹⁵-Y₂-NR¹⁹-Y₁-; H-Y₂-NR¹⁹-Y₁-;
- R³: is hydroxy; halo; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆akylthio; R²¹; R²¹-C₁₋₆alkyl; R²¹-O-; R²¹-S-; R²¹-C(=O)-; R²¹-S(=O)ₚ-; R⁷-S(=O)ₚ-; R⁷-S(=O)ₚ-NH-; R²¹-S(=O)ₚ-NH-; R⁷ -C(=O)-; -NHC(=O)H; -C(=O)NHNH₂; R⁷ -C(=O)-NH-; R²¹-C(=O)-NH-; -C(=NH)R⁷; -C(=NH)R²¹;
- R^{4a} or R^{4b}: each independently are hydrogen, R⁸, -Y₁-NR⁹-Y₂-NR¹⁰R¹¹, -Y₁-NR⁹-Y₁-R⁸, -Y₁-NR⁹R¹⁰;
- R⁵ and R⁶: each independently are hydrogen, R⁸, -Y₁-NR⁹-Y₂ NR₁₀R¹¹, -Y₁-NR⁹-Y₁-R⁸, -Y₁- NR⁹R¹⁰, or
- R⁵ and R⁶: may together with the nitrogen to which they are attached form a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴, or each of said heterocycles may optionally be fused with a benzene ring, said benzene ring being optionally substituted with one or more substituents selected from R¹², R¹³ and R¹⁴;
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle;
- R⁹, R¹⁰ and R¹¹: each independently are hydrogen or R⁸, or
- any: two of R⁹, R¹⁰ and R¹¹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S-(=O)₂-; R¹⁵R¹⁶-S(=O)-; R¹⁵R¹⁶-S(=O)₂-; R¹⁷R¹⁸-Y₁-; R¹⁷R¹⁸-Y₂ -NR¹⁶-Y₁-; R¹⁵-Y₂ NR¹⁹-Y₁-; H-Y₂ NR¹⁹-Y₁-; oxo, or
- any: two of R¹², R¹³ and R¹⁴ may together be C₁₋₆alkanediyl or C₂₋₆alkenediyl thereby forming a saturated or partially saturated monocyclic 3 to 8 membered carbo - or heterocycle or an aromatic 4 to 8 membered monocyclic carbo - or heterocycle together with the atoms to which they are attached, or
- any: two of R¹² , R¹³ and R¹⁴ may together be -O-(CH₂)ᵣ-O- thereby forming a saturated, partially saturated or aromatic monocyclic 4 to 8 membered carbo - or heterocycle together with the atoms to which they are attached;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴; or each of said carbocycles or heterocycles may optionally be fused with a benzene ring, said benzene ring being optionally substituted with one or more substituents selected from R¹², R¹³ and R¹⁴;
- R¹⁶, R¹⁷, R¹⁸ and R¹⁹: each independently are hydrogen or R¹⁵, or
- R¹⁷ and R¹⁸, or R¹⁵ and R¹⁹: may together be C₁₋₆alkanediyl or C₂₋₆alkenediyl thereby forming a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴; or
- R¹⁷ and R¹⁸: together with R¹⁶ may be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and _{R}¹⁴;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
- Y₁ₐ: is -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or-Y₃-C(=O)-O-Y₄-;
- Y₁ or Y₂: each independently are a direct bond, -Y₃-S(=O)-Y₄-; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
- Y₃ or Y₄: each independently are a direct bond, C₁₋₆alkanediyl, C₂₋₆alkenediyl or C₂₋₆alkynediyl;
- n: is 1 or 2;
- m: is 1 or 2;
- p: is 1 or 2;
- r: is 1 to 5;
- s: is 1 to 3;
- aryl: is phenyl or phenyl substituted with one, two, three, four or five substituents each independently selected from halo, C₁₋₆alkyl, C₃₋₇cycloalkyl, C₁₋₆alkyloxy, cyano, nitro, polyhaloC₁₋₆alkyl and polyhaloC₁₋₆alkyloxy.

Also preferred are those compounds of formula (a-1) or (a-2) wherein
- R¹: is hydrogen; aryl; formyl; C₁₋₆alkylcarbonyl; C₁₋₆alkyl; C₁₋₆alkyloxycarbonyl; C₁₋₆alkyl substituted with formyl, C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
- X: is -NR¹-; -C(=O)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -C(=O)-NR¹-, -NR¹-C(=O)-; -C₁₋₆alkyl-; -O-C₁₋₆alkyl-; -C₁₋₆alkyl-O-; -NR¹-C₁₋₆alkyl-; -C₁₋₆alkyl-NR¹-; NR¹-C₁₋₆alkyl-NR¹-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -O-C₂₋₆alkynyl-; -C₂₋₆alkynyl-O-; -NR¹-C₂₋₆alkynyl-; -C₂₋₆alkynyl-NR¹-; -NR¹-C₂₋₆alkynyl-NR¹-; -O-C₁₋₆alkyl-O-; -O-C₂₋₆alkenyl-O-; -O-C₂₋₆alkynyl-O-; -CHOH-; -S(=O)-; -S(=O)₂-; -S(=O)-NR¹-; -S(=O)₂-NR¹-; -NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-;
- R²: is hydrogen, C₁₋₁₀alkyl, C₂₋₁₀alkenyl, C₂₋₁₀alkynyl, R²⁰, each of said groups representing R² may optionally be substituted where possible with one or more substituents each independently being selected from =O; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵ -O-C(=O)-O-; -SO₃H; R¹⁵-S(=O)-; R¹⁵ -S(=O)₂-; R⁵R⁶N; R⁵R⁶N-C₁₋₆alkyl; R⁵R₆N-C₁₋₆alkyloxy; R⁵R⁶N-C(=O)-; R⁵R⁶N-S(=O)ₙ-; R⁵R⁶N-S(=O)ₙ-NH-; R¹⁵-C(=O)-NH-;
- R³: is hydroxy; halo; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷-S(=O)ₚ-; R⁷-C(=O)-; -NHC(=O)H; -C(=O)NHNH₂;R⁷-C(=O)-NH-; R²¹-C(=O)-NH-; -C(=NH)R⁷; -C(=NH)R²¹;
- R^{4a} or R^{4b}: each independently are hydrogen or R⁸;
- R⁵ and R⁶: each independently are hydrogen or R⁸;
- R⁷: is C₁₋₆alkyl, C₁₋₆alkyloxy, amino, mono- or di(C₁₋₆alkyl)amino or polyhaloC₁₋₆alkyl;
- R⁸: is C₁₋₆alkyl; C₂₋₆alkenyl; C₂₋₆alkynyl; a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle;
- R¹², R¹³ and R¹⁴: each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R¹⁵R¹⁶-S(=O)-; R¹⁵R¹⁶N-S(=O)₂-;
- R¹⁵: is C₁₋₆alkyl, C₂₋₆alkenyl, C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₆alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴;
- R¹⁶: is hydrogen or R¹⁵;
- R²⁰: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
- R²¹: is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
- n: is 1 or 2;
- m: is 1 or 2;
- p: is 1 or 2;
- s: is 1 to 3;
- aryl: is phenyl or phenyl substituted with one, two, three, four or five substituents each independently selected from halo, C₁₋₆alkyl, C₃₋₇cycloalkyl, C₁₋₆alkyloxy, cyano, nitro, polyhaloC₁₋₆alkyl and polyhaloC₁₋₆alkyloxy;
and suitably provided that
benzamide, 4-[[5-amino-4-(methylamino)-2-pyrimidinyl]amino]-N,N diethyl-; and benzamide, *N,N* diethyl-4-[[4-(methylami.no)-5-nitro-2-pyrimidinyl]amino]-are not included.

Also preferred are those compounds of formula (a-1) or (a-2) as defined hereinabove wherein R² is other than hydrogen or C₁₋₆alkyl.

Another preferred group of compounds of formula (I) or (I') are those compounds having the following formula 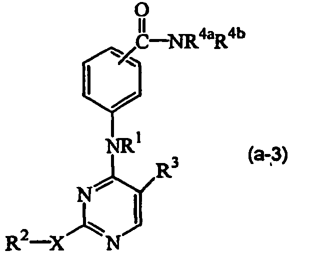 with R¹, R², R³, R^{4a}, R^{4b} and X as defined for the compounds of formula (I) and wherein both X-R² and R³ are other than hydrogen.

Particular preferred are those compounds of formula (a-3) wherein one or where possible more of the following restrictions apply :
(a) X is other than a direct bond, S or C₁₋₆alkyl;
(b) R² is other than optionally substituted phenyl when X is NR¹;
(c) R³ is hydrogen;
(d) R³ is other than hydrogen, cyano or C₁₋₄alkyl;
(e) R² is other than optionally substituted phenyl;
(f) -X-R² is other than hydrogen, hydroxy, C₁₋₆alkyl, C₁₋₆alkyloxy, C₁₋₄alkylthio, C₁₋₄alkyl-S(=O)ₚ-, polyhaloC₁₋₄alkyl, polyhaloC₁₋₄alkyloxy;
(g) R^{4b} is other than -(CH₂)ₙ-N(C₁₋₃alkyl)₂ with n being 2 or 3 when R^{4a} is hydrogen.

Particular preferred compounds of formula (I) or (I') are those compounds selected from
4-[[5-bromo-4-(phenylmethoxy)-2-pyrimidinyl]amino]-benzamide (compound 22);
3-[[5-bromo-4-(phenylmethoxy)-2-pyrimidinyl]amino]-benzanaide (compound 4);
4-[[5-cyano-4-(phenyhnethoxy)-2-pyrimidinyl]amino]-benzamide (compound 23);
3-[[5-cyano-4-(phenylmethoxy)-2-pyrimidinyl]amino]-benzamide (compound 9);
a *N*-oxide, a pharmaceutically acceptable addition salt, a quaternary amine and a stereochemically isomeric form thereof.

Further preferred compounds of formula (I) or (I') are those compounds selected from *N,N*-dimethyl-4-[4-(2,4,6-trimethyl-phenylamino)-pytimidin-2-ylamino]-benzamide; *N*-methyl-4-[4-(2,4,6-trimethyl-phenylamino)-pyrimidin-2-ylamino]-benzamide *N*-isopropyl-4-[4-(2,4,6-trimethyl-phenylamino)-pyrimidin-2-ylamino]-benzamide 3-(4-benzyloxy-pyrimidin-2-ylamino)-benzamide;
3-(4-hydrox-y-pyrimidin-2-ylamino)-benzamide;
3-(5-bromo-4-hydroxy-pyrimidin-2-ylamino)-benzamide;
a N-oxide, a pharmaceutically acceptable addition salt, a quaternary amine and a stereochemically isomeric form thereof.

Compounds of formula (I) can be prepared by reacting an intermediate of formula (II) with an intermediate of formula (III) wherein W₁ represents a suitable leaving group, such as for example a halo atom, e.g. chloro, bromo, or C₁₋₆alkyl-S-, in the presence of a suitable solvent, such as for example *N,N*-dimethylacetamide, *N,N*-dimethylformamide, methylene chloride, diglyme, tetrahydrofuran, water, an alcohol, e. g. ethanol, isopropanol and the like, and optionally in the presence of a suitable acid, such as for example hydrochloric acid, or a suitable base, such as for example sodium carbonate, *N,N-*diethylethanamine or *N,N*-diisopropylethanamine.

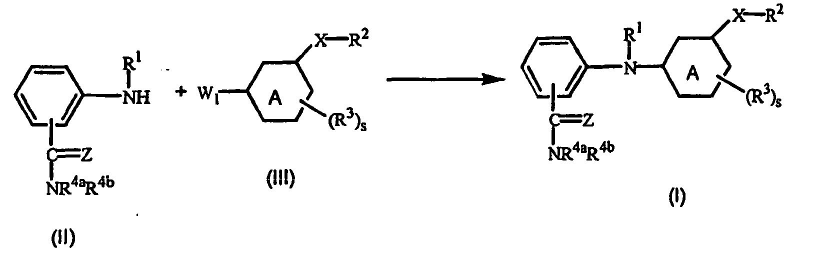

Compounds of formula (I) can also be prepared by reacting an intermediate of formula (IV) wherein W₂ represents a suitable leaving group, such as for example a halo atom, e.g. chloro, bromo and the like, with an intermediate of formula (V) optionally in the presence of a suitable solvent, such as for example CH₃OCH₂CH₂OH.

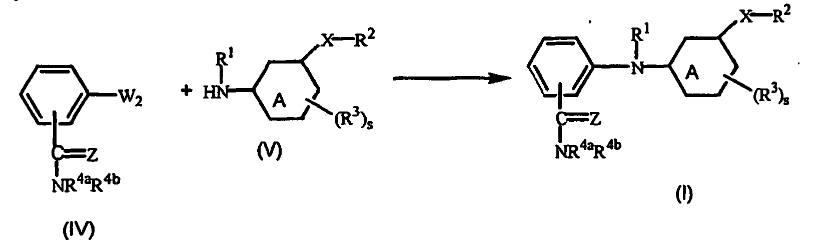

Compounds of formula (I) wherein Z is O, said compounds being represented by formula (I-a) can be prepared by reacting an intermediate of formula (VI) wherein W₃ represents a suitable leaving group, such as for example a halo atom, e.g. chloro, bromo and the like, or C₁₋₆alkyloxy, with an intermediate of formula (VII) in the presence of a suitable solvent, such as for example tetrahydrofuran or an alcohol, e.g. methanol, ethanol and the like.

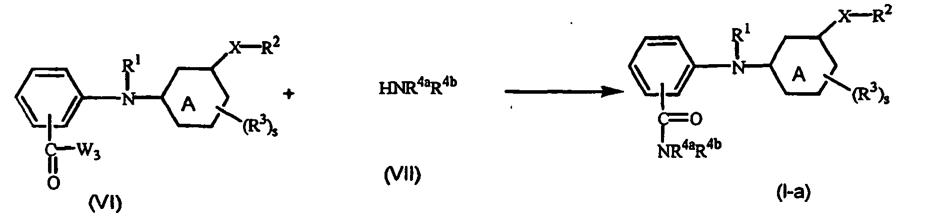

Compounds of formula (I) wherein Z is O and R^{4a} and R^{4b} are hydrogen, said compounds being represented by formula (I-a-1), can be prepared by reacting an intermediate of formula (VIII) with a suitable oxidizing agent, such as for example H₂O₂ or NaBO₃, in the presence of a suitable solvent, such as for example water, dimethylsulfoxide or an alcohol, e.g. methanol, ethanol and the like, and optionally in the presence of a suitable base, such as for example dipotassium carbonate.

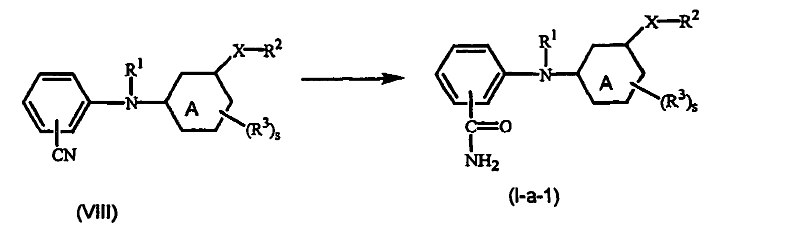

In this and the following preparations, the reaction products may be isolated from the reaction medium and, if necessary, further purified according to methodologies generally known in the art such as, for example, extraction, crystallization, distillation, trituration and chromatography.

The compounds of formula (I) may further be prepared by converting compounds of formula (I) into each other according to art-known group transformation reactions.

The compounds of formula (I) may be converted to the corresponding *N*-oxide forms following art-known procedures for converting a trivalent nitrogen into its N-oxide form. Said *N*-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (I) wherein R³ is halo, or wherein R² is substituted with halo, can be converted into a compound of formula (I) wherein R³ is cyano, or wherein R² is substituted with cyano, by reaction with a suitable cyano-introducing agent, such as sodium cyanide or CuCN, optionally in the presence of a suitable catalyst, such as for example tetrakis(triphenylphosphine)palladium and a suitable solvent, such as *N,N*-dimethylacetamide or N,N-dimethylformamide. A compound of formula (I) wherein R³ is cyano, or wherein R² is substituted with cyano, can further be converted into a compound of formula (I) wherein R³ is aminocarbonyl, or wherein R² is substituted with aminocarbonyl, by reaction with HCOOH, in the presence of a suitable acid, such as hydrochloric acid. A compound of formula (I) wherein R³ is cyano, or wherein R² is substituted with cyano, can also further be converted into a compound of formula (I) wherein R³ is tetrazolyl, or wherein R² is substituted with tetrazolyl, by reaction with sodium azide in the presence of ammonium chloride and *N*,*N-*dimethylacetoacetamide.

Compounds of formula (I) wherein R² is substituted with halo, can also be converted into a compound of formula (I) wherein R² is substituted with mercapto, by reaction with disodium sulfide in the presence of a suitable solvent, such as, for example, 1,4-dioxane.

Compounds of formula (I) wherein R² is substituted with halo, can also be converted into a compound of formula (I) wherein R² is substituted with C₁₋₆alkylthio, by reaction with a reagent of formula alkaline metal⁺ -S-C₁₋₆alkyl, e.g. Na⁺ -S-C₁₋₆alkyl, in the presence of a suitable solvent, such as dimethylsulfoxide. The latter compounds can further be converted into a compound of formula (I) wherein R² is substituted with C₁₋₆alkyl-S(=O)-, by reaction with a suitable oxidizing agent, such as a peroxide, e.g. 3-chlorobenzenecarboperoxoic acid, in the presence of a suitable solvent, such as an alcohol, e.g. ethanol.

Compounds of formula (I) wherein R³ is halo, or wherein R² is substituted with halo, can also be converted into a compound of formula (I) wherein R³ is C₁₋₆alkyloxy, or wherein R² is substituted with C₁₋₆alkyloxy, by reaction with alcoholate salt, such as, for example, LiOC₁₋₆alkyl, in the presence of a suitable solvent, such as an alcohol, e.g. methanol.

Compounds of formula (I) wherein R³ is halo, or wherein R² is substituted with halo, can also be converted into a compound of formula (I) wherein R³ is hydroxy, or wherein R² is substituted with hydroxy, by reaction with a suitable carboxylate, e.g. sodium acetate, in a suitable reaction-inert solvent, such as, for example, dimethylsulfoxide, followed by treating the obtained reaction product with a suitable base, such as pyridine, and acetyl chloride.

Compounds of formula (I) wherein R³ is halo, or wherein R² is substituted with halo, can also be converted into a compound of formula (I) wherein R³ is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, or wherein R² is substituted with a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, said substituents being represented by -L, by reaction with H-L in the presence of a suitable base, such as for example sodium hydroxide, dipotassium carbonate, sodium hydride, in the presence of a suitable solvent, such as, for example, 1,4-dioxane, *N,N*-dimethylacetamide, *N,N*-dimethylformamide.

Compounds of formula (I) wherein R³ is chloro, or wherein R² is substituted with chloro, can be converted into a compound of formula (I) wherein R³ is fluoro, or wherein R² is substituted with fluoro, by reaction with a suitable fluoride salt, such as for example potassium fluoride, in the presence of a suitable solvent, e.g. sulfolane.

Compounds of formula (I) wherein X-R² is hydrogen and wherein the R³ substituent positioned at the meta position compared to the NR¹ linker, is halo, can be converted into a compound of formula (I) wherein said R³ substituent is replaced by X-R² wherein X is other than a direct bond when R² is hydrogen, by reaction with H-X-R² in the presence of a suitable solvent, such as *N,N*-dimethylacetamide or *N,N*-dimethylformamide optionally in the presence of a suitable base, such as for example *N,N*-diisopropylethanamine.

Compounds of formula (I) wherein R² is substituted with C₁₋₄alkyloxyC₁₋₆alkyl, can be converted into a compound of formula (I) wherein R² is substituted with hydroxyC₁₋₆alkyl, by dealkylating the ether in the presence of a suitable dealkylating agent, such as, for example, tribromoborane, and a suitable solvent, such as methylene chloride.

Compounds of formula (I) wherein R³ or X-R² are C₁₋₆alkyloxycarbonyl, or wherein R² is substituted with C₁₋₆alkyloxycarbonyl, can be converted into a compound of formula (I) wherein R³ or X-R² are aminocarbonyl, or wherein R² is substituted with aminocarbonyl or mono- or di(C₁₋₆alkyl)aminocarbonyl by reaction with a suitable agent such as ammonia, NH₂(C₁₋₆alkyl), AlCH₃[N(C₁₋₆alkyl)₂]Cl optionally in the presence of a suitable acid, such as for example hydrochloric acid, and in the presence of a suitable solvent such as an alcohol, e.g. methanol; tetrahydrofuran; *N,N-*diisopropylethane.

Compounds of formula (I) wherein R³ is hydrogen or wherein R² is unsubstituted, can be converted into a compound wherein R³ is halo or wherein R² is substituted with halo, by reaction with a suitable halogenating agent, such as, for example Br₂ or 1-(chloromethyl)-4-fluoro-1,4-diazoniabicyclo[2,2,2]octane bis[tetrafluoroborate], in the presence of a suitable solvent, such as tetrahydrofuran, water, acetonitrile, chloroform and optionally in the presence of a suitable base such as *N,N-*diethylethanamine.

Compounds of formula (I) wherein R³ or -X-R² are C₁₋₆alkyloxycarbonyl or wherein R² is substituted with C₁₋₆alkyloxycarbonyl, can be converted into a compound of formula (I) wherein R³ or X-R² are hydroxymethyl or wherein R² is substituted with hydroxymethyl by reaction with a suitable reducing agent, such as for example LiAlH₄.

Compounds of formula (I) wherein -X-R² is -O-CH₂-(optionally substituted)phenyl may be converted into a compound of formula (I) wherein X-R² represents OH by reaction with a suitable reducing agent, such as H₂, in the presence of a suitable catalyst, such as for example palladium on charcoal, and a suitable solvent, such as for example an alcohol, e.g. methanol, ethanol and the like, or N, N-dimethylacetamide. Compounds of formula (I) wherein -X-R² represents OH may be converted into a compound of formula (I) wherein X-R² represents -O-X₁-R² by reaction with W₁-X₁-R² wherein W₁ represents a suitable leaving group, such as for example a halo atom, e.g. chloro, and wherein -O-X₁ represents those linkers falling under the definition of X which are attached to ring A via a O atom (in said definition X₁ represents that part of the linker wherein the O atom is not included), in the presence of a suitable base, such as for example dipotassium carbonate, and a suitable solvent, such as for example *N,N-*dimethylacetamide.

Compounds of formula (I) wherein R³ is nitro, or wherein R² is substituted with nitro, may be converted into a compound of formula (I) wherein R³ is amino or wherein R² is substituted with amino, by reaction with a suitable reducing agent, such as for example H₂, in the presence of a suitable catalyst, such as for example palladium on charcoal, a suitable catalyst poison, such as for example a thiophene solution, and a suitable solvent, such as for example an alcohol, e.g. methanol, ethanol and the like.

Compounds of formula (I) wherein R² is substituted with NH₂, can be converted into a compound of formula (I) wherein R² is substituted with NH-S(=O)₂-NR⁵R⁶, by reaction with W₁-S(=O)₂-NR⁵R⁶ wherein W₁ represents a suitable leaving group such as for example a halo atom, e.g. chloro, in the presence of a suitable solvent, such as for example *N,N*-dimethylacetamide and a suitable base, such as for example *N,N*-diethylethanamine.

Some of the compounds of formula (I) and some of the intermediates in the present in vention may contain an asymmetric carbon atom. Pure stereochemically isomeric forms of said compounds and said intermediates can be obtained by the application of art-known procedures. For example, diastereoisomers can be separated by physical methods such as selective crystallization or chromatographic techniques, e.g. counter current distribution, liquid chromatography and the like methods. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, selective crystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

An alternative manner of separating the enantiomeric forms of the compounds of formula (I) and intermediates involves liquid chromatography, in particular liquid chromatography using a chiral stationary phase.

Some of the intermediates and starting materials are known compounds and may be commercially available or may be prepared according to art-known procedures, such as those described in WO 99/50250, WO 00/27825 or EP 0,834,507.

Intermediates of formula (III) can be prepared by reacting an intermediate of formula (IX) wherein W₁ is as defined hereinabove, with an intermediate of formula (X) in the presence of a suitable solvent, such as for example acetonitrile or dioxane, and in the presence of a suitable base, such as for example *N,N-*diisopropylethanamine.

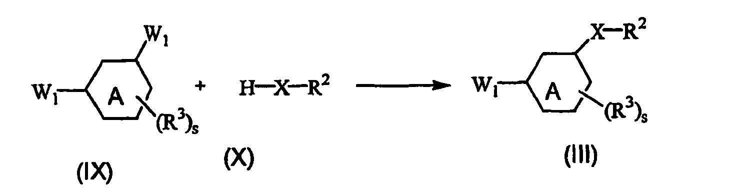

Intermediates of formula (VI) can be prepared by reacting an intermediate of formula (V) with an intermediate of formula (XI) wherein W₄ represents a suitable leaving group, such as for example a halo atom, e.g. chloro and the like, in the presence of a suitable solvent, such as for example CH₃OCH₂CH₂OH.

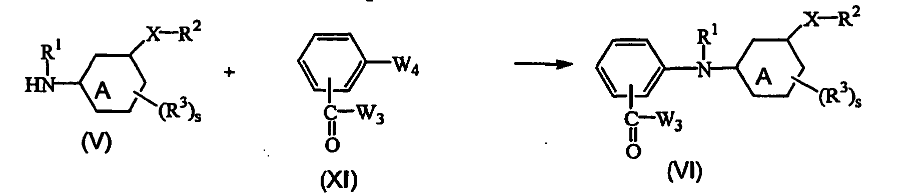

Intermediates of formula (VI) wherein R¹ is hydrogen, said intermediates being represented by formula (VI-a), can be prepared by reacting an intermediate of formula (XI) with an intermediate of formula (XII) in the presence of a suitable salt such as for example dipotassium carbonate and CuI.

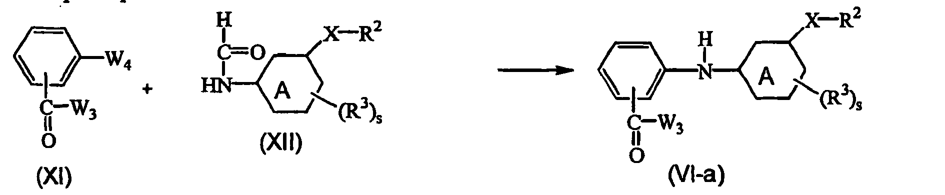

Intermediates of formula (XII) can be prepared by reacting an intermediate of formula (V) wherein R¹ is hydrogen, said intermediate being represented by formula (V-a), with formic acid.

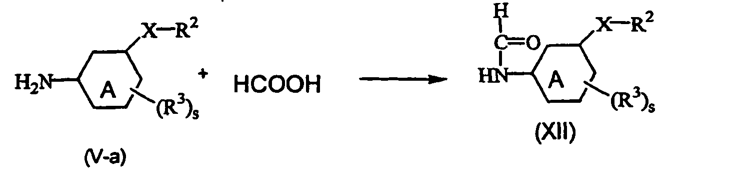

Intermediates of formula (VI) wherein X-R² is OH, said intermediates being represented by formula (VI-b), can be prepared by reducing an intermediate of formula (XIII) in the presence of a suitable reducing agent, such as for example H₂, a suitable catalyst, such as palladium on charcoal, and a suitable solvent, such as an alcohol, e.g. ethanol and the like.

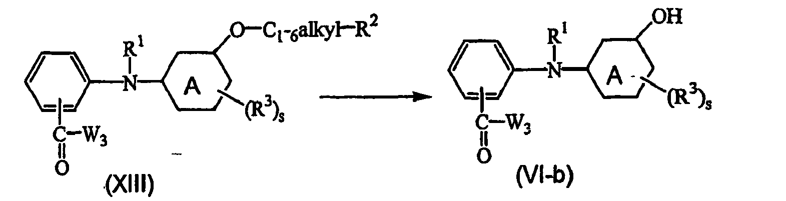

Intermediates of formula (VIII) can be prepared by reacting an intermediate of formula (III) with an intermediate of formula (XIV) in the presence of a suitable solvent, such as for example dioxane and diethylether, and a suitable acid, such as for example hydrochloric acid.

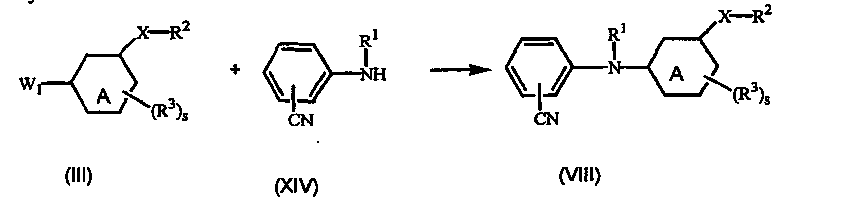

Intermediates of formula (VIII) wherein X is -O-C₁₋₆alkyl, said intermediates being represented by formula (VIII-a), can be prepared by reacting an intermediate of formula (XV) with an intermediate of formula (XVI) in the presence of sodium hydride, and a suitable solvent, such as for example tetrahydrofuran.

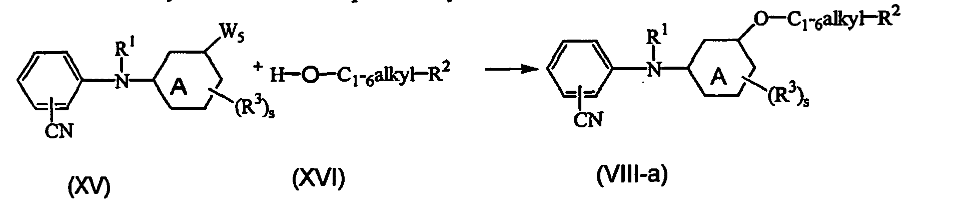

The compounds of formula (I) or (I') inhibit Glycogen synthase kinase 3 (GSK3), in particular glycogen synthase kinase 3 beta (GSK3β). They are selective Glycogen synthase kinase 3 inhibitors. Specific inhibitory compounds are superior therapeutic agents since they are characterized by a greater efficacy and lower toxicity by virtue of their specificity.
Synonyms for GSK3 are tau protein kinase I (TPK I), FA (Factor A) kinase, kinase FA and ATP-citrate lysase kinase (ACLK).

Glycogen synthase kinase 3 (GSK3), which exists in two isoforms, i.e. GSK3α and GSK3β, is a proline-directed serine/threonine kinase originally identified as an enzyme that phosphorylates glycogen synthase. However, it has been demonstrated that GSK3 phosphorylates numerous proteins in vitro such as glycogen synthase, phosphatase inhibitor 1-2, the type-II subunit of cAMP-dependent protein kinase, the G-subunit of phosphatase-1, ATP-citrate lyase, acetyl coenzyme A carboxylase, myelin basic protein, a microtubule-associated protein, a neurofilament protein, an N-CAM cell adhesion molecule, nerve growth factor receptor, c-Jun transcription factor, JunD transcription factor, c-Myb transcription factor, c-Myc transcription factor, L-Myc transcription factor, adenomatous polyposis coli tumor supressor protein, tau protein and β-catenin.

The above-indicated diversity of proteins which may be phosphorylated by GSK3 implies that GSK3 is implicated in numerous metabolic and regulatory processes in cells.
GSK3 inhibitors may therefore be useful in the prevention or treatment of diseases mediated through GSK3 activity such as bipolar disorder (in particular manic depression), diabetes, Alzheimer's disease, leukopenia, F TDP-17 (Fronto-temporal dementia associated with Parkinson's disease), cortico-basal degeneration, progressive supranuclear palsy, multiple system atrophy, Pick's disease, Niemann Pick's disease type C, Dementia Pugilistica, dementia with tangles only, dementia with tangles and calcification, Down syndrome, myotonic dystrophy, Parkinsonism-dementia complex of Guam, aids related dementia, Postencephalic Parkinsonism, prion diseases with tangles, subacute sclerosing panencephalitis, frontal lobe degeneration (FLD), argyrophilic grains disease, subacute sclerotizing panencephalitis (SSPE) (late complication of viral infections in the central nervous system), inflammatory diseases, cancer, dermatological disorders such as baldness, neuronal damage, schizophrenia, pain, in particular neuropathic pain. GSK3 inhibitors can also be used to inhibit sperm motility and can therefore be used as male contraceptives.
In particular, the compounds of the present invention are useful in the prevention or treatment of Alzheimer's disease, diabetes, especially type 2 diabetes (non insulin dependent diabetes).

The major neuropathological landmarks in Alzheimer's disease are neuronal loss, the deposition of amyloid fibers and paired helical filaments (PHF) or neurofibrillary tangles (NF1). Tangle formation appears to be the consequence of accumulation of aberrantly phosphorylated tau protein. This aberrant phosphorylation destabilizes neuronal cytoskeleton, which leads to reduced axonal transport, deficient functioning and ultimately neuronal death. The density of neurofibrillary tangles has been shown to parallel duration and severity of Alzheimer's disease. Reduction of the degree of tau phosphorylation can provide for neuroprotection and can prevent or treat Alzheimer's disease or can slow the progression of the disease. As mentioned hereinabove, GSK3 phosphorylates tau protein. Thus compounds having an inhibitory activity for GSK3 may be useful for the prevention or the treatment of Alzheimer's disease.

Insulin regulates the synthesis of the storage polysaccharide glycogen. The rate-limiting step in the glycogen synthesis is catalyzed by the enzym glycogen synthase. It is believed that glycogen synthase is inhibited by phosphorylation and that insulin stimulates glycogen synthase by causing a net decrease in the phosphorylation of this enzym. Thus, in order to activate glycogen synthase, insulin must either activate phosphatases or inhibit kinases, or both.
It is believed that glycogen synthase is a substrate for glycogen synthase kinase 3 and that insulin inactivates GSK3 thereby promoting the dephosphorylation of glycogen synthase.
In addition to the role of GSK3 in insulin-induced glycogen synthesis, GSK3 may also play a role in insulin resistance. It is believed that GSK3 dependent Insulin Receptor Substrate-1 phosphorylation contributes to insulin resistance.
Therefore, GSK3 inhibition may result in the increased deposition of glycogen and a concomitant reduction of blood glucose, thus mimicing the hypoglycemic effect of insulin. GSK3 inhibition provides an alternative therapy to manage insulin resistance commonly observed in non insulin dependent diabetes mellitus and obesity. GSK3 inhibitors may thus provide a novel modality for the treatment of type 1 and type 2 diabetes.

GSK3 inhibitors, in particular GSK3β inhibitors, may also be indicated for use in the prevention or the treatment of pain, in particular neuropathic pain.
After axotomy or chronic constriction injury, neuronal cells die through an apoptotic pathway and the morphological changes correlate with the onset of hyperalgesia and/or allodynia.
The induction of apoptosis is probably triggered by a reduced supply of neurotrophic factors as the time course of neuronal loss is positively altered by administration of neurotrophins. GSK, in particular GSK3β, has been shown to be involved in the initiation of the apoptotic cascade and trophic factor withdrawal stimulates the GSK3β apoptosis pathway.
In view of the above, GSK3β inhibitors might reduce signals of and even prevent levels of neuropathic pain.

Due to their GSK3 inhibitory properties, particularly their GSK3β inhibitory properties, the compounds of formula (I) or (I'), their N-oxides, pharmaceutically acceptable addition salts, quaternary amines and stereochemically isomeric forms thereof, are useful to prevent or treat GSK3 mediated diseases, in particular GSK3β mediated diseases, such as bipolar disorder (in particular manic depression), diabetes, Alzheimer's disease, leukopenia, FTDP-17 (Fronto-temporal dementia associated with Parkinson's disease), cortico-basal degeneration, progressive supranuclear palsy, multiple system atrophy, Pick's disease, Niemann Pick's disease type C, Dementia Pugilistica, dementia with tangles only, dementia with tangles and calcification, Down syndrome, myotonic dystrophy, Parkinsonism-dementia complex of Guam, aids related dementia, Postencephalic Parkinsonism, prion diseases with tangles, subacute sclerosing panencephalitis, frontal lobe degeneration (FLD), argyrophilic grains disease, subacute sclerotizing panencephalitis (SSPE) (late complication of viral infections in the central nervous system), inflammatory diseases, cancer, dermatological disorders such as baldness, neuronal damage, schizophrenia, pain, in particular neuropathic pain. The present compounds are also useful as male contraceptives. In general, the compounds of the present invention may be useful in the treatment of warm-blooded animals suffering from disease mediated through GSK3, in particular GSK3β, or they may be useful to prevent warm-blooded animals to suffer from disease mediated through GSK3, in particular GSK3β. More in particular, the compounds of the present invention may be useful in the treatment of warm-blooded animals suffering from Alzheimer's disease, diabetes, especially type 2 diabetes, cancer, inflammatory diseases or bipolar disorder.

In view of the above described pharmacological properties, the compounds of formula (I) or any subgroup thereof, their N-oxides, pharmaceutically acceptable addition salts, quaternary amines and stereochemically isomeric forms, may be used as a medicine. In particular, the present compounds can be used for the manufacture of a medicament for treating or preventing diseases mediated through GSK3, in particular GSK3β. More in particular, the present compounds can be used for the manufacture of a medicament for treating or preventing Alzheimer's disease, diabetes, especially type 2 diabetes, cancer, inflammatory diseases or bipolar disorder.

In view of the utility of the compounds of formula (I) or (I'), there is provided a method of treating warm-blooded animals, including humans, suffering from or a method of preventing warm-blooded animals, including humans, to suffer from diseases mediated through GSK3, in particular GSK3β, more in particular a method of treating or preventing Alzheimer's disease, diabetes, especially type 2 diabetes, cancer, inflammatory diseases or bipolar disorder. Said method comprises the administration, preferably oral administration, of an effective amount of a compound of formula (I) or (I'), a *N*-oxide form, a pharmaceutically acceptable addition salt, a quaternary amine or a possible stereoisomeric form thereof, to warm-blooded animals, including humans.

The present invention also provides compositions for preventing or treating diseases mediated through GSK3, in particular GSK3β, comprising a therapeutically effective amount of a compound of formula (I) or (I') and a pharmaceutically acceptable carrier or diluent.

The compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment. The compounds of the present invention may also be administered via inhalation or insufflation by means of methods and formulations employed in the art for administration via this way. Thus, in general the compounds of the present invention may be administered to the lungs in the form of a solution, a suspension or a dry powder. Any system developed for the delivery of solutions, suspensions or dry powders via oral or nasal inhalation or insufflation are suitable for the administration of the present compounds.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The present compounds are orally active compounds, and are preferably orally administered.
The exact dosage, the therapeutically effective amount and frequency of administration depends on the particular compound of formula (I) or (I') used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

When used as a medicament to prevent or treat Alzheimer's disease, the compounds of formula (I) or (I') may be used in combination with other conventional drugs used to combat Alzheimer's disease, such as galantamine, donepezil, rivastigmine or tacrine. Thus, the present invention also relates to the combination of a compound of formula (I) or (I') and another agent capable of preventing or treating Alzheimer's disease. Said combination may be used as a medicine. The present invention also relates to a product containing (a) a compound of formula (I) or (I'), and (b) another agent capable of preventing or treating Alzheimer's disease, as a combined preparation for simultaneous, separate or sequential use in the prevention or treatment of Alzheimer's disease. The different drugs may be combined in a single preparation together with pharmaceutically acceptable carriers.

When used as a medicament to prevent or treat type 2 diabetes, the compounds of formula (I) or (I') may be used in combination with other conventional drugs used to combat type 2 diabetes, such as glibenclamide, chlorpropamide, gliclazide, glipizide, gliquidon, tolbutamide, metformin, acarbose, miglitol, nateglinide, repaglinide, acetohexamide, glimepiride, glyburide, tolazamide, troglitazone, rosiglitazone, pioglitazone, isaglitazone.
Thus, the present invention also relates to the combination of a compound of formula (I) or (I') and another agent capable of preventing or treating type 2 diabetes. Said combination may be used as a medicine. The present invention also relates to a product containing (a) a compound of formula (I) or (I'), and (b) another agent capable of preventing or treating type 2 diabetes, as a combined preparation for simultaneous, separate or sequential use in the prevention or treatment of type 2 diabetes. The different drugs may be combined in a single preparation together with pharmaceutically acceptable carriers.

When used as a medicament to prevent or treat cancer, the compounds of formula (I) or (I') may be used in combination with other conventional drugs used to combat cancer such as platinum coordination compounds for example cisplatin or carboplatin; taxane compounds for example paclitaxel or docetaxel; camptothecin compounds for example irinotecan or topotecan; anti-tumour vinca alkaloids for example vinblastine, vincristine or vinorelbine; anti-tumour nucleoside derivatives for example 5-fluorouracil, gemcitabine or capecitabine; nitrogen mustard or nitrosourea alkylating agents for example cyclophosphamide, chlorambucil, carmustine or lomustine; anti-tumour anthracycline derivatives for example daunorubicin, doxorubicin or idarubicin; HER2 antibodies for example trastzumab; and anti-tumour podophyllotoxin derivatives for example etoposide or teniposide; and antiestrogen agents including estrogen receptor antagonists or selective estrogen receptor modulators preferably tamoxifen, or alternatively toremifene, droloxifene, faslodex and raloxifene; aromatase inhibitors such as exemestane, anastrozole, letrazole and vorozole; differentiating agents for example retinoids, vitamin D and DNA methyl transferase inhibitors for example azacytidine; kinase inhibitors for example flavoperidol and imatinib mesylate or farnesyltransferase inhibitors for example R115777.
Thus, the present invention also relates to the combination of a compound of formula . (I) or (I') and another agent capable of preventing or treating cancer. Said combination may be used as a medicine. The present invention also relates to a product containing (a) a compound of formula (I) or (I'), and (b) another agent capable of preventing or treating cancer, as a combined preparation for simultaneous, separate or sequential use in the prevention or treatment of cancer. The different drugs may be combined in a single preparation together with pharmaceutically acceptable carriers.

When used as a medicament to prevent or treat bipolar disorder, the compounds of formula (I) or (I') may be used in combination with other conventional drugs used to combat bipolar disorder such as atypical antipsychotics, anti-epileptica, benzodiazepines, lithium salts, for example olanzapine, risperidone, carbamazepine, valproate, topiramate.
Thus, the present invention also relates to the combination of a compound of formula (1) or (I') and another agent capable of preventing or treating bipolar disorder. Said combination may be used as a medicine. The present invention also relates to a product containing (a) a compound of formula (I) or (I'), and (b) another agent capable of preventing or treating bipolar disorder, as a combined preparation for simultaneous, separate or sequential use in the prevention or treatment of bipolar disorder. The different drugs may be combined in a single preparation together with pharmaceutically acceptable carriers.

When used as a medicament to prevent or treat inflammatory diseases, the compounds of formula (I) or (I') may be used in combination with other conventional drugs used to combat inflammatory diseases such as steroids, cyclooxygenase-2 inhibitors, non-steroidal-anti-inflammatory drugs, TNF- α antibodies, such as for example acetyl salicylic acid, bufexamac, diclofenac potassium, sulindac, diclofenac sodium, ketorolac trometamol, tolmetine, ibuprofen, naproxen, naproxen sodium, tiaprofen acid, flurbiprofen, mefenamic acid, nifluminic acid, meclofenamate, indomethacin, proglumetacine, ketoprofen, nabumetone, paracetamol, piroxicam, tenoxicam, nimesulide, fenylbutazon, tramadol, beclomethasone dipropionate, betamethasone, beclamethasone, budesonide, fluticasone, mometasone, dexamethasone, hydrocortisone, methylprednisolone, prednisolone, prednisone, triamcinolone, celecoxib, rofecoxib, infliximab, leflunomide, etanercept, CPH 82, methotrexate, sulfasalazine.

Thus, the present invention also relates to the combination of a compound of formula (I) or (I') and another agent capable of preventing or treating inflammatory diseases. Said combination may be used as a medicine. The present invention also relates to a product containing (a) a compound of formula (I) or (I'), and (b) another agent capable of preventing or treating inflammatory diseases, as a combined preparation for simultaneous, separate or sequential use in the prevention or treatment of inflammatory disorders. The different drugs may be combined in a single preparation together with pharmaceutically acceptable carriers.

### Experimental part

Hereinafter, "DMF" is defined as *N,N-*dimethylformamide, "THF" is defined as tetrahydrofuran, "DMSO" is defined as dimethylsulfoxide, "TFA" is defined as trifluoroacetic acid.

### A. Preparation of the intermediate compounds

### Example A1

a) Reaction under Argon atmosphere. 2,4,6-Trimethylaniline (0.0678 mol) was added to 2,4-dichloropyrimidine (0.0664 mol) in 1,4-dioxane (100 ml). N,N-di(1-methylethyl)-ethaneamine (N,N-diisopropylethanamine) (0.0830mol) was added. The reaction mixture was stirred and refluxed for 4 days and the solvent was evaporated. The residue was dissolved in CH₂Cl₂, washed with a saturated NaHCO₃ solution, then dried (Na₂SO₄), filtered and the solvent was evaporated to give 17.1 g solid residue. This solid was dissolved in CH₂Cl₂:hexane (1:1; 150 ml), and the resulting solution was concentrated to 100 ml, then filtered. The residue was purified by column chromatography on KP-Sil (eluent: CH₂Cl₂). The desired fractions were collected and the solvent was evaporated. The less polar fraction was stirred in CH₂Cl₂ for 3 hours and filtered, yielding 0.44 g 4-chloro-N-(2,4,6-trimethylphenyl)-2-pyrimidinamine. A second fraction was recrystallized from acetonitrile, filtered off and dried, yielding 2-chloro-N-(2,4,6-trimethyl-phenyl)-4-pyrimidinamine (intermediate 1) (mp. 182-183°C).
b) A mixture of intermediate 1 (1.06 mol) and 5.4 N HCl/2-propanol (1.15 mol) in water (4000 ml) was stirred and warmed to 40-45 °C over 30 minutes. 4-Aminobenzonitrile (1.29 mol) was added at 40-45 °C. The reaction mixture was stirred and refluxed for 3.5 hours, then cooled to room temperature. EtOAc (1000 ml) was added and the mixture was alkalized by portionwise addition of NaHCO₃. EtOAc (1000 ml) was added and the mixture was stirred vigorously for 10 minutes. The whole was filtered off to give precipitate (I) and filtrate (I). The filtrate's (I) layers were separated. The organic layer was washed with brine, dried (MgSO₄), filtered and the solvent was evaporated. The residue was stirred in ethanol (300 ml), filtered off, then dried (vacuum, 40 °C), yielding: 50 g of fraction 1. Precipitate (I) was stirred in EtOAc (1000 ml), filtered off and dried (vacuum, 40 °C). This fraction was stirred in ethanol (400 ml), filtered off and dried (vacuum, 40 °C). Yielding: 248 g of fraction 2. Fractions 1 and 2 were combined, stirred for 45 minutes in boiling ethanol (2000 ml, p.a.), then allowed to cool while stirring overnight. The precipitate was filtered off, washed with ethanol, and dried (vacuum, 40 °C, 24 hours), yielding 271 g of 4-[[4-[(2,4,6-trimethylphenyl]amino]-2-pyrimidinyl]amino]benzonitrile (intermediate 2) (mp. 217.1-218.2°C).

### Example A2

### a) Preparation of intermediate 3

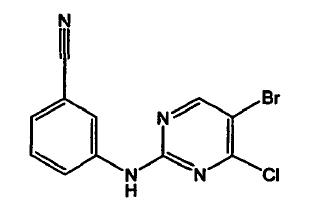

Compound 8 (prepared according to B4) (0.0162 mol) and POCl₃ (25 ml) were heated in an oil bath at 125-135°C, and stirred for 12 minutes. The sample was poured over ice, stirred, and filtered. The resulting solid was dried at room temperature for 3 days at 200 mm Hg, yielding 4.86g of intermediate 3 (yellow solid, 97%)

### b) Preparation of intermediate 4

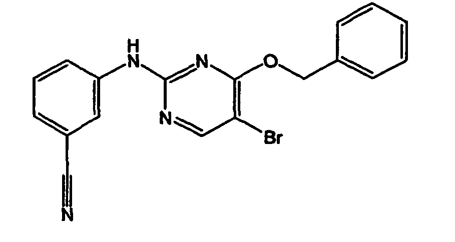

NaH (0.00808 mol), THF (0.00808 mol) and benzenemethanol (0.00808 mol) were stirred for 5 minutes under Ar. Intermediate 3 (0.00646 mol) was added, and the sample was refluxed overnight. The sample was evaporated. More THF, water, and CH₃CN were added. The mixture was stirred for 60 minutes, then filtered to produce 2.49 g solid. The solid was dried at 65°C for 3 days in 200 mm Hg, then 1 day at 80°C and 0.2 mm Hg. A 0.26 g sample was purified by flash column chromatography in CH₂Cl₂, then dried at 80°C for 16 hours at 0.2 mm Hg, yielding 0.23 g of intermediate 4 (88%) (mp. 156-157°C).

### B. Preparation of the final compounds

### Example B 1

### a) Preparation of compound 1

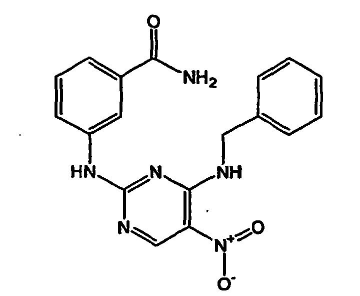

A solution of 2-chloro-5-nitro-N-(phenylmethyl)-4-pyrimidinamine (0.012 mol), 3-aminobenzamide (0.012 mol) and Et₃N (0.012 mol) in DMF (50 ml) was stirred for 2 hours at 60°C. The mixture was allowed to cool to room temperature and methanol (10 ml) was added. The mixture was stirred for 10 minutes and the resulting precipitate was filtered off, washed and dried, yielding 3.3 g of compound 1 (77%).

### b) Preparation of compound 2

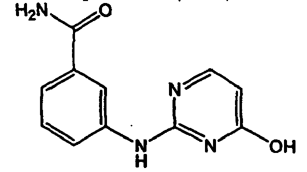

4,5-Diamino-6-methyl-2(IH)-pyrimidinethione (0.0704 mol), 3-aminobenzamide (0.106 mol), and (CH₃OCH₂CH₂)₂O (20 ml) were refluxed under Ar for 3 hours, then stirred overnight at room temperature. The sample was heated to reflux, filtered, and washed with hot (CH₃0CH₂CH₂)₂O (2x) then filtered, yielding 15.25 g of compound 2.

### c) Preparation of compound 3

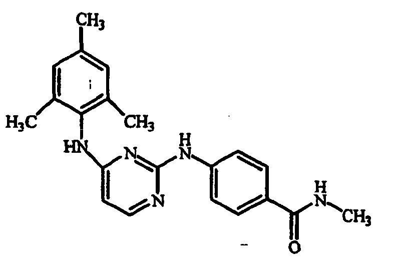

A mixture of MeOH (4 ml), H₂O (4 ml) and HCl/2-propanol (0.2 ml) was added to a mixture of intermediate 1 (0.000242 mol) and 4-amino-N-methyl-benzamide (0.000242 mol). The reaction mixture was stirred overnight at 60 °C. The desired compound was isolated and purified by high performance liquid chromatography over RP C-18 (eluent: (0.5% NH₄OAc in H₂O/CH₃CN 90/10)/CH₃OH/CH₃CN 70/15/15; 0/50/50; 0/0/100). The desired fractions were collected and the solvent was evaporated, yielding 0.017 g of compound 3.

### Example B2

### Preparation of compound 4

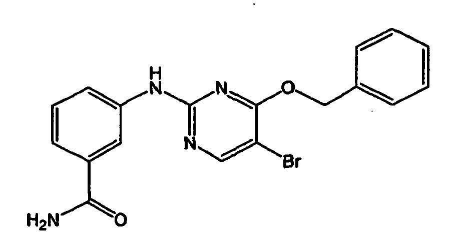

30% H₂O₂ (7.3 ml) was added dropwise to intermediate 4 (0.00378 mol) and K₂CO₃ (1.22 g) in DMSO (17.5 ml) in a water bath at 16-17°C. More DMSO (20 ml) was added, and the reaction was stirred and warmed to room temperature for 4 hours. More DMSO (20 ml) was added to reduce foam, and the mixture was stirred for 1 hour. Water was added, and the sample was filtered to 2.56 g solid. The solid was partitioned between CHCl₃ and water, stirred overnight, then filtered to 1.11 g white solid, and the filtrate was evaporated to 0.17g. The filtrate solid was recrystallized with methanol. The sample was dried at 80°C for 16 hours at 0.2 mm Hg, yielding 0.08g of compound 4 (mp.:216-217°C).

### Example B3

### a) Preparation of compound 5

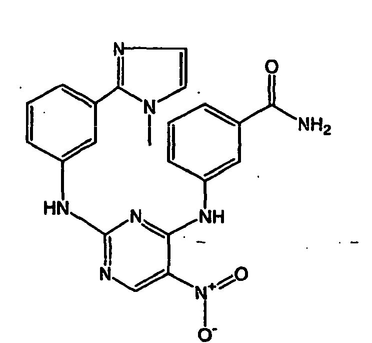

A mixture of 0.0127 mol of compound 6 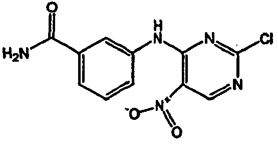 (prepared according to B1a) in DMF (80 ml)) was treated with 3-(1-methyl-1H-imidazol-2-yl)benzeneamine (0.0127 mol) and *N*-ethyl-*N*-(1-methylethyl)-2-propanamine (0.0127 mol). The reaction mixture was stirred for 4 hours at 60 °C, then cooled to room temperature, yielding compound 5 (100%).

### b) Preparation of compound 7

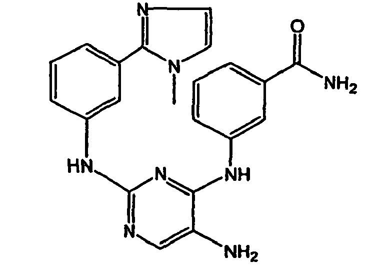

The mixture of 0.0127 mol of compound 5 in DMF (80 ml) was hydrogenated (H166-080) at 50 °C with Pd/C, 10% (2 g) as a catalyst in the presence of thiophene solution (2 ml) and extra DMF (20 ml). After uptake of H₂ (3 equiv), the catalyst was filtered off, washed and the filtrate was evaporated under reduced pressure, yielding compound 7 (100%).

### Example B4

### Preparation of compound 8

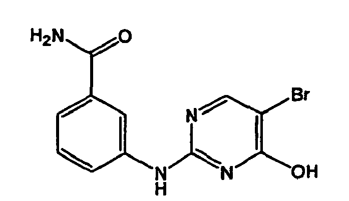

N₂ was bubbled through a solution of compound 2 (prepared according to B1b) (0.0663 mol), DMSO (800 ml) and Et₃N (0.0729 mol) under Ar for 0.5 hours. Br₂ (0.0729 mol) was added. The reaction was stirred at room temperature overnight. Water (11) was added dropwise. Filtration produced 17.5 g solid. The sample was refluxed in MeOH (11) for 60 minutes, then cooled, filtered, and dried at 80°C for 3 days at 200 mm Hg, yielding 11.51g of compound 8 (55%)..

### Example B5

### Preparation of compound 9

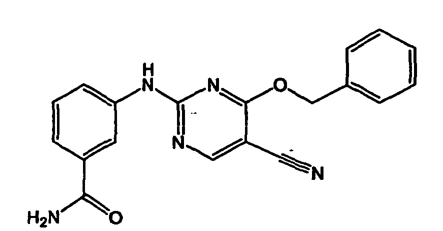

Compound 4 (prepared according to B2) (0.00226 mol), CuCN (0.03383 mol), and DMF (27 ml) were added to a pressure vessel under Argon. The mixture was stirred at 110-120°C for 2 days. The mixture was filtered, and the solvent was evaporated for 2 days. The mixture was sonicated and stirred in 10% MeOH: CH₂Cl₂ (250 ml). The sample was purified by column chromatography, washing with 10% MeOH: CH₂Cl₂ to produce 0.05 g solid. The sample was purified by Gilson prep HPLC (gradient of 0.1% TFA in H₂O and 0.1% TFA in CH₃CN), lyophilized, then dried at 80°C for 16 hours at 0.2 mm Hg, yielding 0.02g of compound 9 (mp.: 260-261°C).

Tables 1 to 3 list the compounds of formula (I) which were prepared according to one of the above examples.

**Table 1**

| | | | | |
|---|---|---|---|---|
| 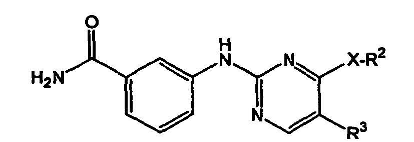 | | | | |

| Comp. no | Exp. no. | -X-R² | R³ | Physical data |
|---|---|---|---|---|
| 9 | B5 | 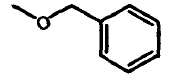 | -CN | mp.260-261°C |
| 4 | B2 | 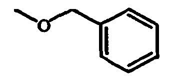 | -Br | mp.216-217°C |
| 8 | B4 | -OH | -Br | |
| 2 | B1b | -OH | -H | |
| 1 | B1a | 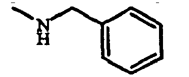 | -NO₂ | |
| 10 | B3b | 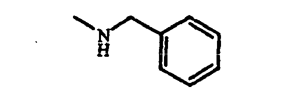 | -NH₂ | |
| 11 | B1a | 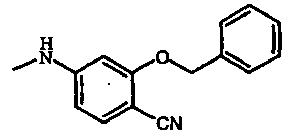 | -NO₂ | |
| 12 | B3b | 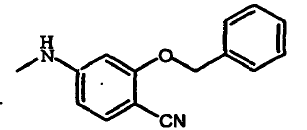 | -NH₂ | |
| 13 | B1a | 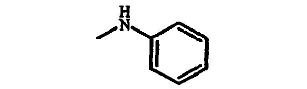 | NO₂ | |
| 14 | B3b | 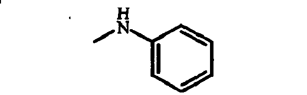 | -NH₂ | |
| 15 | B1a | 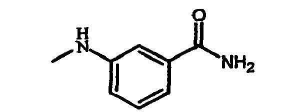 | NO₂ | |
| 16 | B3b | 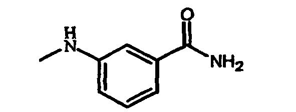 | -NH₂ | |
| 17 | B1a | 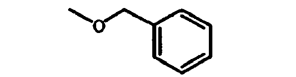 | -H | |

**Table 2**

| | | | | |
|---|---|---|---|---|
| 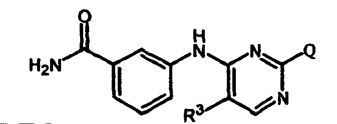 | | | | |

| Comp. no | Exp. no. | -Q | R³ | Physical data |
|---|---|---|---|---|
| 6 | B1a | -Cl | -NO₂ | |
| 18 | B3a | 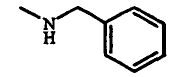 | NO₂ | |
| 19 | B3b | 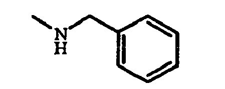 | -NH₂ | |
| 5 | B3a | 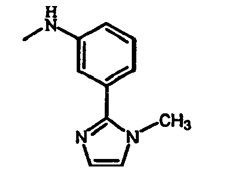 | NO₂ | |
| 7 | B3b | 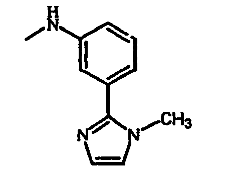 | -NH₂ | |

**Table 3**

| | | | | | | |
|---|---|---|---|---|---|---|
| 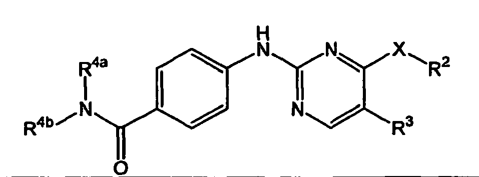 | | | | | | |

| Comp. no | Exp. no. | -X-R² | R³ | R^{4a} | R^{4b} | Physical data |
|---|---|---|---|---|---|---|
| 20 | B1c | 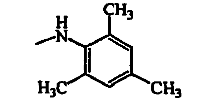 | -H | -CH₃ | -CH₃ | |
| 3 | B1c | 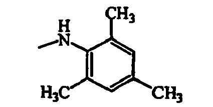 | -H | -H | -CH₃ | |
| 21 | B1c | 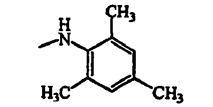 | -H | -H | -C₃H₇ | |
| 22 | B2 | 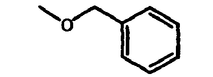 | -Br | -H | -H | mp.223-224°C |
| 23 | B8 | 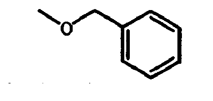 | -CN | -H | -H | mp.240-241 °C |

### C. Pharmacological Example

The pharmacological activity of the present compounds was examined using the following test.

GSK3beta assays were performed at 25°C in a 100 µl reaction volume of 25mM Tris (pH 7.4) containing 10 mM MgCl₂, 1 mM DTT, 0.1 mg/ml BSA, 5% glycerol and containing 19 nM GSK3β, 5 µM biotinylated phosphorylated CREB peptide , 1 µM ATP, 2nM ATP-P³³ and a suitable amount of a test compound of formula (I) or (I'). After one hour, the reaction was terminated by adding 70 µl of Stop mix (1 mM ATP, 18 mg/ml streptavidin coated PVT SPA bead pH 11.0). The beads to which the phosphorylated CREB peptide is attached were allowed to settle for 30 minutes and the radioactivity of the beads was counted in a microtiterplate scintillation counter and compared with the results obtained in a control experiment (without the presence of a test compound) in order to determine the percentage of GSK3β inhibition. The IC₅₀ value, i.e. the concentration (M) of the test compound at which 50 % of GSK3β is inhibited, was calculated from the dose response curve obtained by performing the above-described GSK3β assay in the presence of different amounts of the test compound.
Table 4 lists pIC₅₀ values (-log IC₅₀ (M)) obtained in the above-described test for the present compounds.

**Table 4**

| **Comp. No.** | **pIC₅₀** |
|---|---|
| 17 | 5.85 |
| 1 | 6.74 |
| 22 | 7.19 |
| 4 | 7.28 |
| 23 | 7.66 |
| 9 | 7.74 |

## Claims

1. A compound of formula 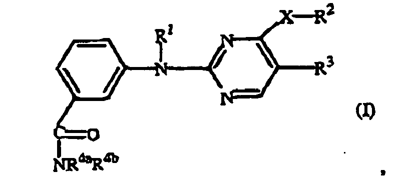 a *N*-oxide, a pharmaceutically acceptable addition salt, a quaternary amine and a stereochemically isomeric form thereof, wherein
R¹ is hydrogen; aryl; formyl; C₁₋₆alkylcarbonyl; C₁₋₆alkyl; C₁₋₆alkyloxycarbonyl; C₁₋₆alkyl substituted with formyl C₁₋₆alkylcarbonyl, C₁₋₆alkyloxycarbonyl, C₁₋₆alkylcarbonyloxy; C₁₋₆alkyloxyC₁₋₆alkylcarbonyl optionally substituted with C₁₋₆alkyloxycarbonyl;
X is -NR¹-; -NH-NH-; -N=N-; -O-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -O-C(=O)-C(=O)-; -C(=O)-NR¹-, -NR¹-C(=O)-; -C(=S)-NR¹-, -NR¹-C(=S)-; -NR¹-C(=O)-NR¹-; -NR¹-C(=S)-NR¹-; -NR¹-S(=O)-NR¹-; -NR¹-S(=O)₂-NR¹-, -C₁₋₆alkyl-C(=O)-NR¹-; -O-C₁₋₆alkyl-C(=O)-NR¹-; -C₁₋₆alkyl-O-C(=O)-NR¹-; -C₁₋₆alkyl-; -O-C₁₋₆alkyl-; -C₁₋₆alkyl-O-; -NR¹ -C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-: -C₂₋₆alkenyl-O-; -NR¹-C₂₋₆alkynyl-; -C₂₋₆alkenyl-NR¹-; -NR¹-C₂₋₆alkenyl-NR¹-; -NR¹-C₂₋₆alkenyl-C₃₋₇cycloalkyl-; -O-C₂₋₆alkynyl-; -C₂₋₆alkynyl-O-; -NR¹-C₂₋₆alkynyl-; -C₂₋₆alkynyl-NR¹-; -NR¹-C₂₋₆alkynyl-NR¹-; -NR¹-C₂₋₆alkynyl-C₃₋₇cycloalkyl-; -O-C₁₋₆alkyl-O-; -O-C₂₋₆alkenyl-O-; -O-C₂₋₆alkynyl-O-; -CHOH-; -S-; -S(=O)-; -S(=O)₂-; -S(=O)-NR¹-; -S(=O)₂-NR¹-; -NR¹-S(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂- or a direct bond;
R² is hydrogen. C₁₋₁₀alkyl, C₂₋₁₀alkenyl, C₂₋₁₀alkynyl, R²⁰, each of said groups representing R² may optionally be substituted where possible with one or more substituents each independently being selected from =S; =O; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S-S(=O)₂-; R⁵R⁶N; R⁵R⁶N-C₁₋₆alkyl; R⁵R⁶N-C₃₋₇cycloalkyl; R⁵R⁶N-C₁₋₆alkyloxy; R⁵R⁶N-C(=O)-; R⁵R⁶N-C(=S)-; R⁵R⁶-C(=O)-NH-; R⁵R⁶N-C(=S)-NH-; R⁵R⁶N-S(=O)ₙ-; R⁵R⁶N-S(=O)ₙ NH-; R¹⁵-C(=S)-; R¹⁵-C(=O)-NH-; R¹⁵-O-C(=O)-NH-; R¹⁵-S(=O)ₙ-NH-; R¹⁵ -O-S(=O)ₙ-NH-; R¹⁵-C(=S)-NH-; R¹⁵-O-C(=S)-NH-; R¹⁷R¹⁸N-Y₁ₐ-; R¹⁷R¹⁸N-Y₂ NR¹⁶-Y₁-; R¹⁵Y₂-NR¹⁹-Y₁-; H-Y₂-NR¹⁹-Y₁-;
R³ is hydrogen; hydroxy; halo; C₁₋₆alkyl; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O- R²¹-S-; R²¹-C(=O); R²¹-S(=O)ₚ-; R⁷-S(=O)ₚ-; R⁷-S(=O)ₚ-NH-; R²¹-S(=O)ₚ-NH-; R⁷-C(=O)-; -NHC(-O)H; -C(=O)NHNH₂;R⁷-C(=O)-NH-; R²¹-C(=O)-NH-; -C(=NH)R⁷; -C(=NH)R²¹;
R^{4a} or R^{4b} each independently are hydrogen or R⁸;
R⁵ and R⁶ each independently are hydrogen, R⁸, -Y₁-NR⁹-Y₂-NR¹⁰,R¹¹ -Y₁-NR⁹-Y₁-R⁸, -Y₁-NR⁹R¹⁰, or
R⁵ and R⁶ may together with the nitrogen to which they are attached form a saturated or partially saturated mono cyclic 3 to 8 membered heterocycle or an aromatic 4 to 8 membered monocyclic heterocycle, each of said heterocycles may optionally be substituted with one or more substituents selected from R¹², R¹³ and R¹⁴, or each of said heterocycles may optionally be fused with a benzene ling, said benzene ring being optionally substituted with one or more substituents selected from R¹², R¹³ and R¹⁴;
R⁷ is C₁₋₆alkyl, C₁₋₆alkyloxy, amino, mono- or di(C₁₋₆alkyl)amino or polyhaloC₁₋₆alkyl;
R⁸ is C₁₋₆alkyl:
R⁹, R¹⁰ and R¹¹ each independently are hydrogen or R⁸, or
any two of R⁹, R¹⁰ and R¹¹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
R¹², R¹³ and R¹⁴ each independently are hydrogen; R¹⁵; hydroxy; halo; nitro; cyano; R¹⁵-O-; SH; R¹⁵-S-; formyl; carboxyl; R¹⁵-C(=O)-; R¹⁵-O-C(=O)-; R¹⁵-C(=O)-O-; R¹⁵-O-C(=O)-O-; -SO₃H; R¹⁵-S(=O)-; R¹⁵-S(=O)₂-; R¹⁵R¹⁶N-S(=O)-; R¹⁵R¹⁶-S(=O)-; R¹⁷R¹⁸-Y₁-; R¹⁷R¹⁸Y₂-NR¹⁶-Y₁-; R¹⁵-Y₂-NR¹⁹-Y₁-; H-Y₂-NR¹⁹-Y₁-; oxo, or
any two of R¹², R¹³ and R¹⁴ may together be C₁₋₆alkanediyl or C₂₋₆alkenediyl thereby forming a saturated or partially saturated monocyclic 3 to 8 membered carbo - or heterocycle or an aromatic 4 to 8 membered monocyclic carbo - or heterocycle together with the atoms to which they are attached, or
any two of R¹², R¹³ and R¹⁴ may together be -O-(CH₂)ᵣ-O- thereby forming a saturated, partially saturated or aromatic monocyclic 4 to 8 membered carbo - or heterocycle together with the atoms to which they are attached;
R¹⁵ is C₁₋₆alkyl, C₂₋₆alkenyl. C₂₋₆alkynyl, a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle; C₁₋₄alkyl substituted with a monocyclic, bicyclic or tricyclic saturated carbocycle or with a monocyclic, bicyclic or tricyclic partially saturated carbocycle or with a monocyclic, bicyclic or tricyclic aromatic carbocycle or with a monocyclic, bicyclic or tricyclic saturated heterocycle or with a monocyclic, bicyclic or tricyclic partially saturated heterocycle or with a monocyclic, bicyclic or tricyclic aromatic heterocycle; each of said substituents representing R¹⁵ may optionally be substituted with one or more substituents selected from R¹², R¹³ and R¹⁴; or each of said carbocycles or heterocycles may optionally be fused with a benzene ring, said benzene ring being optionally substituted with one or more substituents selected from R¹², R¹³ and R¹⁴;
R¹⁶, R¹⁷, R¹⁸ and R¹⁹ each independently are hydrogen or R¹⁵, or
R¹⁷ and R¹⁸, or R¹⁵ and R¹⁹ may together be C₁₋₆alkanediyl or C₂₋₆alkenediyl thereby forming a saturated or partially saturated monocyclic 3 to 8 membered heterocycle or an aromatic 4 to 8 membered monocyclic heterocycle, each of said heterocycles may optionally be substituted with one or more substituents selected from R¹², R¹³ and R¹⁴; or
R¹⁷ and R¹⁸ together with R¹⁶ may be C₁₋₆alkanediyl or C₂₋₆alkenediyl thereby forming a saturated or partially saturated monocyclic 3 to 8 membered heterocycle or an aromatic 4 to 8 membered monocyclic heterocycle together with the nitrogen atoms to which they are attached, each of said heterocycles may optionally be substituted with one or more substituents selected from R¹², R¹³ and R¹⁴;
R²⁰ is a monocyclic, bicyclic or tricyclic saturated carbocycle; a monocyclic, bicyclic or tricyclic partially saturated carbocycle, a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle;
R²¹ is a monocyclic, bicyclic or tricyclic saturated carbocycle; a monocyclic, bicyclic or tricyclic partially saturated carbocycle; a monocyclic, bicyclic or tricyclic aromatic carbocycle; a monocyclic, bicyclic or tricyclic saturated heterocycle; a monocyclic, bicyclic or tricyclic partially saturated heterocycle; a monocyclic, bicyclic or tricyclic aromatic heterocycle, each of said carbocycles or heterocycles representing R²¹ may optionally be substituted with one or more substituents selected from R¹², R¹³ and R¹⁴;
Y₁ₐ is -Y₃-S(=O)-Y₄; -Y₃-S(=O)₂-Y₄-, -Y₃-C(=O)-Y₄-, -Y₃-C(=S)-Y₄-, -Y₃-O-Y₄-, -Y₃-S-Y₄-, -Y₃-O-C(=O)-Y₄- or -Y₃-C(=O)-O-Y₄-;
Y₁ or Y₂ each independently are a direct bond, -Y₃-S(=O)-Y₄-;-Y₃-S(=O)₂-Y₄-, Y₃-C(=O)-Y₄ , Y₃-C(=S)-Y₄-, -Y₃-O-Y₄-, -Y₃-S-Y₄-, -Y₃-O-C(=O)-Y₄- or -Y₃-C(=O)-O-Y₄-;
Y₃ or Y₄ each independently are a direct bond, C₁₋₆alkanediyl, C₂₋₆alkenediyl or C₂₋₆alkynediyl;
n is 1 or 2;
m is 1 or 2;
p is 1 or 2;
r is 1 to 5;
aryl is phenyl or phenyl substituted with one, two, three, four or five substituents each independently selected from halo, C₁₋₆alkyl, C₃₋₇cycloalkyl, C₁₋₆alkyloxy, cyano, nitro, polyhaloC₁₋₆alkyl and polyhaloC₁₋₆alkyloxy;
provided that -X-R² and/or R³ is other than hydrogen.

2. A compound according to claim 1 wherein
X is -NR¹-; -NH-NH-; -N=N-; -C(=O)-; -C(=S)-; -O-C(=O)-; -C(=O)-O-; -O-C(=O)-C₁₋₆alkyl-; -C(=O)-O-C₁₋₆alkyl-; -O-C₁₋₆alkyl-C(=O)-; -C(=O)-C₁₋₆alkyl-O-; -O-C(=O)-NR¹-; -NR¹-C(=O)-O-; -O-C(=O)-C(=O); -C(=O)-NR¹-, -NR¹-C(=O)-; -C(=S)-NR¹-, -NR¹-C(=S)-; -NR¹-C(=O)-NR¹-; -NR¹-C(=S)-NR¹-; -NR¹-S(=O)-NR¹-; -NR¹-S(=O)₂-NR¹-; -C₁₋₆alkyl-C(=O)-NR¹-; -O-C₁₋₆alkyl-C(=O)-NR¹-; -C₁₋₆alkyl-O-C(=O)-NR¹-; -C₁₋₆alkyl-; -O-C₁₋₆alkyl-; -C₁₋₆alkyl-O-; -NR¹-C₁₋₆alkyl-; -C₁₋₆alkyl-NR¹-; -NR¹-C₁₋₆alkyl-NR¹-; -NR¹-C₁₋₆alkyl-C₃₋₇cycloalkyl-; -C₂₋₆alkenyl-; -C₂₋₆alkynyl-; -O-C₂₋₆alkenyl-; -C₂₋₆alkenyl-O-; -NR¹-C₂₋₆alkenyl-; -C₂₋₆alkenyl-NR¹-; -NR¹-C₂₋₆alkenyl-NR¹-; -NR¹-C₂₋₆alkenyl-C₃₋₇cycloalkyl-: -O-C₂₋₆alkynyl-; -C₂₋₆alkynyl-O-. -NR¹-C₂₋₆alkynyl-; -C₂₋₆alkynyl-NR¹; -NR¹-C₂₋₆alkynyl-NR¹; -NR¹-C₂₋₆alkynyl -C₃₋₇cycloalkyl ; -O-C₁₋₆alkyl-O-; -O-C₂₋₆alkanyl-O-; -O-C₂₋₆alkynyl-O-: -CHOH-; -S(=O).; -S(=O)₂-; -S(=O)-NR¹-; S(=O)₂-NR¹-; -NR¹-5(=O)-; -NR¹-S(=O)₂-; -S-C₁₋₆alkyl-; -C₁₋₆alkyl-S-; -S-C₂₋₆alkenyl-; -C₂₋₆alkenyl-S-; -S-C₂₋₆alkynyl-; -C₂₋₆alkynyl-S-; -O-C₁₋₆alkyl-S(=O)₂-;
R³ is hydroxy; halo; C₁₋₆alkyl substituted with cyano, hydroxy or -C(=O)R⁷; C₂₋₆alkenyl; C₂₋₆alkenyl substituted with one or more halogen atoms or cyano; C₂₋₆alkynyl; C₂₋₆alkynyl substituted with one or more halogen atoms or cyano; C₁₋₆alkyloxy; C₁₋₆alkylthio; C₁₋₆alkyloxycarbonyl; C₁₋₆alkylcarbonyloxy; carboxyl; cyano; nitro; amino; mono- or di(C₁₋₆alkyl)amino; polyhaloC₁₋₆alkyl; polyhaloC₁₋₆alkyloxy; polyhaloC₁₋₆alkylthio; R²¹; R²¹-C₁₋₆alkyl; R²¹-O-; R²¹-S-; R²¹-C(=O)-; R²¹-S(=O)ₚ-; R⁷-S(=O)ₚ-; R⁷ -S(=O)ₚ-NH-; R²¹-S(=O)ₚ-NH-; R⁷-C(=O)-; -NHC(=O)H; -C(=O)NHNH₂; R⁷-C(=O)-NH-; R²¹-C(=O)-NH-; -C(=NH)R⁷; -C(=NH)R²¹;

3. A compound as claimed in claim 1 or 2 wherein X is other than NR¹ or S.

4. A compound according to claim 1 wherein
R¹ is hydrogen;
X is -NR¹-; -O-; -O-C₁₋₆alkyl-; -NR¹-C₁₋₆alkyl- or a direct bond;
R² is hydrogen, C₁₋₁₀alkyl, R²⁰, each of said groups representing R² may optionally be substituted where possible with one or more substituents each independently being selected from R¹⁵ ; cyano; R¹⁵-O-; R⁵R⁶-C(=O)-;
R³ is hydrogen; halo; cyano; nitro; amino; R²¹-C₁₋₆alkyl;
R⁵ and R⁶ are hydrogen;
R¹², R¹³ and R¹⁴ are R¹⁵;
R¹⁵ is C₁₋₆alkyl; a monocyclic, bicyclic or bicyclic aromatic carbocycle; a monocyclic, bicyclic or tricyclic aromatic heterocycle; C₁₋₆alkyl substituted with a monocyclic, bicyclic or tricyclic aromatic carbocycle;
R²⁰ is a monocyclic, bicyclic or tricyclic aromatic carbocycle;
R²¹ is a monocyclic, bicyclic or tricyclic aromatic carbocycle.

5. A compound according to any one of claims 1 to 4 wherein X-R² and R³ are other than hydrogen.

6. A compound according to anyone of the preceding claims wherein R² is other than hydrogen or C₁₋₆alkyl.

7. A compound as claimed in claim 1 wherein the compound is
3-[[5-bromo-4-(phenylmcthoxy)-2-pyrimidinyl]amino]-benzandde;
3-[[5-cyano-4-(phenylmethoxy)-2-pyrimidinyl]amino]-benzamide;
a N-oxide, a pharmaceutically acceptable addition salt, a quaternary amine and a stereochemically isomeric form thereof

8. A compound as claimed in claim 1 wherein the compound is
3-(4-benzyloxy-pyrimidin-2-ylamino)-benzamide;
3-(4-hydroxy-pyrimidin-2-ylamino)-benzamide;
3-(5-bromo-4-hydroxy-pyrimidin-2-ylamino)-benzamide.
a *N*-oxide, a pharmaceutically acceptable addition salt, a quaternary amine and a stereochemically isomeric form thereof.

9. A compound as claimed in any one of claims 1 to 8 for use as a medicine.

10. The use of a compound as claimed in any one of claims 1 to 8 for the manufacture of a medicament for the prevention or the treatment of diseases mediated through GSK3.

11. The use of a compound as claimed in any one of claims 1 to 8 for the manufacture of a medicament for the prevention or the treatment of bipolar disorder (in particular manic depression), diabetes, Alzheimer's disease, leukopenia, FTDP-17 (Fronto-temporal dementia associated with Parkinson's disease), cortico-basal degeneration, progressive supranuclear palsy, multiple system atrophy, Pick's disease, Niemann Pick's disease type C, Dementia Pugilistica, dementia with tangles only, dementia with tangles and calcification, Down syndrome, myotonic dystrophy, Parkinsonism-dementia complex of Guam, aids related dementia, Postencephalic Parkinsonism, prion diseases with tangles, subacute sclerosing panencephalitis, frontal lobe degeneration (FLD), argyrophilic grains disease, subacute sclerotizing panencephalitis (SSPE) (late complication of viral infections in the central nervous system), inflammatory diseases, cancer, dermatological disorders, neuronal damage, schizophrenia, pain.

12. The use as claimed in claim 11 for the prevention or the treatment of Alzheimer's disease, diabetes, cancer, inflammatory diseases or bipolar disorder.

13. A pharmaceutical composition comprising a pharmaceutically acceptable carrier and as active ingredient a therapeutically effective amount of a compound as claimed in any one of claims 1 to 8.

14. A process for preparing a pharmaceutical composition as claimed in claim 13 **characterized in that** a therapeutically effective amount of a compound as claimed in any one of claims 1 to 8 is intimately mixed with a pharmaceutically acceptable carrier.

15. A process for preparing a compound as claimed in claim 1, **characterized by**
a) reacting an intermediate of formula (II) with an intermediate of formula (III) in the presence of a suitable solvent and optionally in the presence of a suitable acid or a suitable base 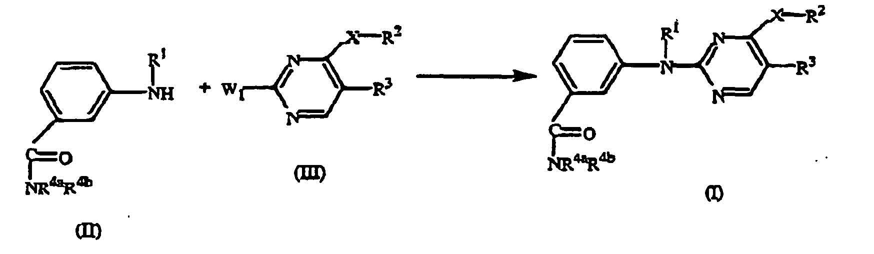 with W₁ representing a suitable leaving group and with R¹, R², R³, R^{4a}, R^{4b} and X as defined in claim 1;
b) reacting an intermediate of formula (IV) with an intermediate of formula (V) optionally in the presence of a suitable solvent 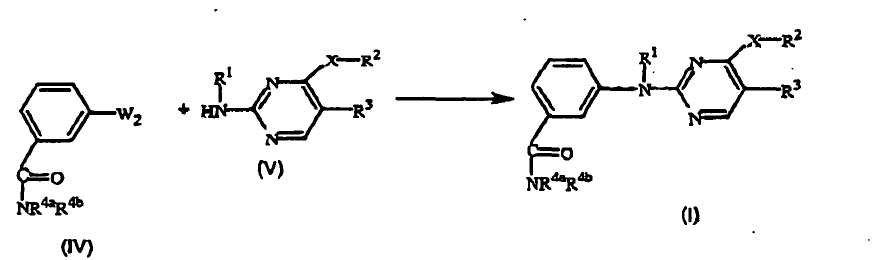 with W₂ representing a suitable leaving group and with R¹, R². R³, R^{4a}, R^{4b} and X as defined in claim 1;
c) reacting an intermediate of formula (VI) with an intermediate of formula (VII) in the presence of a suitable solvent 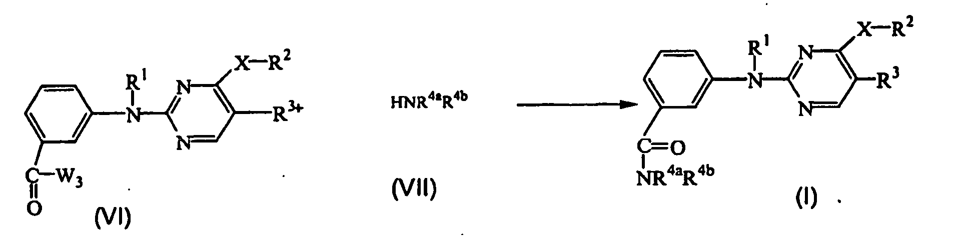 with W₃ representing a suitable leaving group and with R¹, R², R³, R^{4a}, R^{4b} and X as defined in claim 1;
d) reacting an intermediate of formula (VIII) with a suitable oxidizing agent in the presence of a suitable solvent and optionally in the presence of a suitable base 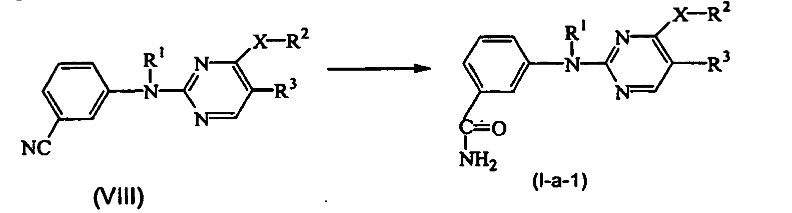 with R¹, R², R³ and X as defined in claim 1;
and, if desired, converting the compounds of formula (I), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms, quaternary amines or N-oxide forms thereof.

## Patentansprüche

1. Verbindung der Formel 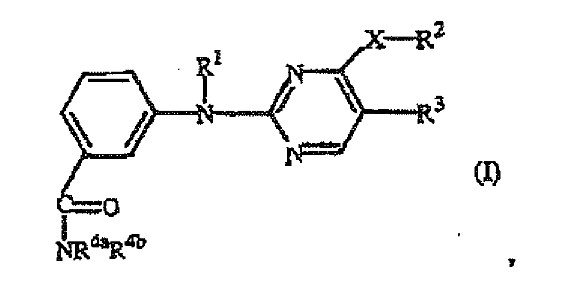 ein N-Oxid, ein pharmazeutisch unbedenkliches Additionssalz, ein quaternäres Amin und eine stereochemisch isomere Form davon, wobei
R¹ für Wasserstoff; Aryl; Formyl; C₁₋₆-Alkylcarbonyl; C₁₋₆-Alkyl; C₁₋₆-Alkyloxycarbonyl; mit Formyl, C₁₋₆-Alkylcarbonyl, C₁₋₆-Alkyloxycarbonyl
oder C₁₋₆-Alkylcarbonyloxy substituiertes C₁₋₆-Alkyl oder gegebenenfalls mit C₁₋₆-Alkyloxycarbonyl substituiertes C₁₋₆-Alkyloxy-C₁₋₆-alkylcarbonyl steht;
X für -NR¹-; -NH-NH-; -N=N-; -O-; -C(=O)-; -C(=S)-; -O-C(=0)-; -C(=0)-0-; -O-C (=O) -C₁₋₆-Alkyl-; -C (=O) - O-C₁₋₆-Alkyl-; -O-C₁₋₆-Alkyl-C (=O) -; -C (=O)-C₁₋₆-Alkyl-O-; -O-C(=O)-NR¹-; -NR¹-C(=O)-O-; -O-C(=O)-C (=O) -; -C(=O)-NR¹-; -NR¹-C (=O) -; -C(=S)-NR¹-; -NR¹-C(=S)-; -NR¹-C (=O)-NR¹- ; -NR¹C(=S)-NR¹-; -NR¹-S(=O)-NR¹-; -NR¹-S(=O)₂-NR¹-; -C₁₋₆-Alkyl-C(=O)-NR¹-; -O-C₁₋₆-Alkyl-C (=O) -NR¹-; -C₁₋₆-Alkyl-O-C (=O) -NR¹-; -C₁₋₆-Alkyl-; -O-C₁₋₆-Alkyl-; -C₁₋₆-Alkyl-O-; NR¹-C₁₋₆-Alkyl-; -C₁₋₆-Alkyl-NR¹-; -NR¹-C₁₋₆-Alkyl-NR¹- -NR¹-C₁₋₆-Alkyl-C₃₋₇-cycloalkyl-; -C₂₋₆-Alkenyl-; -C₂₋₆-Alkinyl-; -O-C₂₋₆-Alkenyl-; -C₂₋₆-Alkenyl-O-; -NR¹-C₂₋₆-Alkenyl-; -C₂₋₆-Alkenyl-NR¹-; -NR¹-C₂₋₆-Alkenyl-NR¹-; -NR¹-C₂₋₆-Alkenyl-C₃₋₇-cycloalkyl-; -O-C₂₋₆-Alkinyl-; -C₂₋₆-Alkinyl-O-; -NR¹-C₂₋₆-Alkinyl-; -C₂₋₆-Alkinyl-NR¹- -NR¹-C₂₋₆-Alkinyl-NR¹- -NR¹-C₂₋₆-Alkinyl-C₃₋₇-cycloalakyl-; -O-C₁₋₆-Alkyl-O-; -O-C₂₋₆-Alkenyl-O-; -O-C₂₋₆-Alkinyl-O-; -CHOH-; -S-; -S (=O) -; -S (=O)₂-; -S(=O)-NR¹-; -S(=O)₂-NR¹-; -NR¹-S(=O)-; -NR¹-S (=O)₂-; -S-C₁₋₆-Alkyl-; -C₁₋₆-Alkyl-S- *;* -S-C₂₋₆-Alkenyl-; -C₂₋₆-Alkenyl-S-; -S-C₂₋₆-Alkinyl-; -C₂₋₆-Alkinyl-S-; -O-C₁₋₆-Alkyl-S (=O)₂- oder eine direkte Bindung steht;
R² für Wasserstoff, C₁₋₁₀-Alkyl, C₂₋₁₀-Alkenyl, C₂₋₁₀-Alkinyl oder R²⁰ steht, wobei jede dieser R² darstellenden Gruppen, sofern möglich, gegebenenfalls mit einem oder mehreren unabhängig voneinander unter =S; =O; R¹⁵; Hydroxy; Halogen; Nitro; Cyano; R¹⁵-O-; SH; R¹⁵ -S-; Formyl; Carboxyl; R¹⁵-C (=O)-; R¹⁵-O-C (=O)-; R¹⁵-C (=O) -O-; R¹⁵-O-C (=O) -O-; -SO₃H; R¹⁵-S(=O) -; R¹⁵-S(=O)₂-; R⁵R⁶N; R⁵R⁶N-C₁₋₆-Alkyl; R⁵R⁶N-C₃₋₇-Cycloalkyl; R⁵R⁶N-C₁₋₆-Alkyloxy; R⁵R⁶N-C(=O)-; R⁵R⁶N-C(=S)-; R⁵R⁶N-C (=O) -NH-; R⁵R⁶N-C(=S)-NH- ; R⁵R⁶N-S(=O)ₙ-; R⁵R⁶N- S(=O)ₙ-NH-; R¹⁵-C (=S)- ; R¹⁵-C(=O)-NH-; R¹⁵-C-C(=O)-NH- ; R¹⁵-S(=O)ₙ-NH-; R¹⁵-O-S (=O)ₙ-NH-; R¹⁵-C(=S) -NH-; R¹⁵ -O-C (=S) NH-; R¹⁷R¹⁸N-Y₁ₐ-; R¹⁷R¹⁸N-Y₂-NR¹⁶-Y₁-; R¹⁵-Y₂-NR¹⁹-Y₁- oder H-Y₂-NR¹⁹-Y₁- ausgewählten Substituenten substituiert sein kann;
R³ für Wasserstoff; Hydroxy; Halogen; C₁₋₆-Alkyl; mit Cyano, Hydroxy oder -C(=O) R⁷ substituiertes C₁₋₆-Alkyl; C₂₋₆-Alkenyl; mit einem oder mehreren Halogenatomen oder Cyano substituiertes C₂₋₆-Alkenyl; C₂₋₆-Alkinyl; mit einem oder mehreren Halogenatomen oder Cyano substituiertes C₂₋₆-Alkinyl; C₁₋₆-Alkyloxy; C₁₋₆-Alkylthio; C₁₋₆-Alkyloxycarbonyl; C₁₋₆-Alkylcarbonyloxy, Carboxyl; Cyano; Nitro; Amino; Mono- oder Di (C₁₋₆-alkyl)amino; Polyhalogen-C₁₋₆-alkyl; Polyhalogen-C₁₋₆-alkyloxy; Polyhalogen-C₁₋₆-alkylthio; R²¹; R²¹_ C₁₋₆-Alkyl; R²¹-O-; R²¹-S-; R²¹-C (=O) -; R²¹-S (=O)ₚ-; R⁷-S (=O)ₚ-; R⁷-S (=O)ₚ-NH; R²¹-S (=O)ₚ-NH-; R⁷ -C(=O)-; -NHC (=O)H ; -C(=O) NHNH₂; R⁷-C (=O) -NH- ; R²¹-C (=O) -NH- ; -C (=NH) R⁷ oder -C (=NH) R²¹ steht;
R^{4a} oder R^{4b} jeweils unabhängig voneinander für Wasserstoff oder R⁸ stehen;
R⁵ und R⁶ jeweils unabhängig voneinander für Wasserstoff, R⁸, -Y₁-NR⁹ -Y₂-NR¹⁰R¹¹, -Y₁-NR⁹ -Y₁-R⁸ oder -Y₁-NR⁹R¹⁰ stehen, oder
R⁵ und R⁶ gemeinsam mit dem Stickstoffatom, an das sie gebunden sind, einen gesättigten oder teilweise gesättigten monocyclischen 3- bis 8-gliedrigen Heterocyclus oder einen aromatischen 4- bis 8-gliedrigen monocyclischen Heterocyclus bilden können, wobei jeder dieser Heterocyclen gegebenenfalls mit einem oder mehreren unter R¹², R¹³ und R¹⁴ ausgewählten Substituenten substituiert sein kann oder jeder dieser Heterocyclen gegebenenfalls mit einem Benzolring anelliert sein kann, wobei der Benzolring gegebenenfalls mit einem oder mehreren unter R¹² ,R¹³und R¹⁴ ausgewählten Substituenten substituiert ist; R⁷ für C₁₋₆-Alkyl, C₁₋₆-Alkyloxy, Amino, Mono- oder Di(C₁₋₆-alkyl)amino oder Polyhalogen-C₁₋₆-alkyl steht;
R⁸ für C₁₋₆-Alkyl steht;
R⁹, R¹⁰ und R¹¹ jeweils unabhängig voneinander für Wasserstoff oder R⁸ stehen, oder
zwei beliebige der Gruppen R⁹ , R¹⁰ und R¹¹ gemeinsam für C₁₋₆-Alkandiyl oder C₂₋₆-Alkendiyl stehen und **dadurch** gemeinsam mit den Stickstoffatomen, an die sie gebunden sind, einen gesättigten oder teilweise gesättigten monocyclischen 3- bis 8-gliedrigen Heterocyclus oder einen aromatischen 4- bis 8-gliedrigen monocyclischen Heterocyclus bilden können, wobei jeder dieser Heterocyclen gegebenenfalls mit einem oder mehreren unter R¹², R¹³ und R¹⁴ ausgewählten Substituenten substituiert sein kann;
R¹² R¹³ und R¹⁴ jeweils unabhängig voneinander für Wasserstoff; R¹⁵; Hydroxy; Halogen; Nitro; Cyano; R¹⁵-O-; SH; R¹⁵- S-; Formyl; Carboxyl; R¹⁵-C(=O)-; R¹⁵-O-C (=O)- ; R¹⁵-C (=O )-O-; R¹⁵-O-C(=O)-O-; -SO₃H; R¹⁵-S (=O)-; R¹⁵-S (=O)₂-; R¹⁵R¹⁶N-S(=O)-; R¹⁵ R¹⁶N-S(O)₂-; R¹⁷R¹⁸ N-Y₁-_{;} R¹⁷R¹⁸N-Y₂-NR¹⁶-Y₁- ; R¹⁵-Y₂-NR¹⁹-Y₁; H-Y₂-NR¹⁹-Y₁- oder Oxo stehen, oder
zwei beliebige der Gruppen R¹², R¹³ und R¹⁴ gemeinsam für C₁₋₆-Alkandiyl oder C₂₋₆-Alkendiyl stehen und **dadurch** gemeinsam mit den Atomen, an die sie gebunden sind, einen gesättigten oder teilweise gesättigten monocyclischen 3- bis 8- gliedrigen Carbo- oder Heterocyclus oder einen aromatischen 4- bis 8-gliedrigen monocyclischen Carbo- oder Heterocyclus bilden können; oder
zwei beliebige der Gruppen R¹², R¹³ und R¹⁴ gemeinsam für -O-(CH₂)ᵣ-O- stehen und **dadurch** gemeinsam mit den Atomen, an die sie gebunden sind, einen gesättigten, teilweise gesättigten oder aromatischen monocyclischen 4- bis 8-gliedrigen Carbo- oder Heterocyclus bilden können;
R¹⁵ für C₁₋₆-Alkyl; C₂₋₆-Alkenyl; C₂₋₆-Alkinyl; einen monocyclischen, bicyclischen oder tricyclischen gesättigten Carbocyclus; einen monocyclischen, bicyclischen oder tricyclischen teilweise gesättigten Carbocyclus; einen monocyclischen, bicyclischen oder tricyclischen aromatischen Carbocyclus; einen monocyclischen, bicyclischen oder tricyclischen gesättigten Heterocyclus; einen monocyclischen, bicyclischen oder tricyclischen teilweise gesättigten Heterocyclus; einen monocyclischen, bicyclischen oder tricyclischen aromatischen Heterocyclus oder mit einem monocyclischen, bicyclischen oder tricyclischen gesättigten Carbocyclus oder mit einem monocyclischen, bicyclischen oder tricyclischen teilweise gesättigten Carbocyclus oder mit einem monocyclischen, bicyclischen oder tricyclischen aromatischen Carbocyclus oder mit einem monocyclischen, bicyclischen oder tricyclischen gesättigten Heterocyclus oder mit einem monocyclischen, bicyclischen oder tricyclischen teilweise gesättigten Heterocyclus oder mit einem monocyclischen, bicyclischen oder tricyclischen aromatischen Heterocyclus substituiertes C₁₋₆-Alkyl steht; wobei jeder dieser R¹⁵ darstellenden Substituenten gegebenenfalls mit einem oder mehreren unter R¹², R¹³ und R¹⁴ ausgewählten Substituenten substituiert sein kann; oder jeder dieser Carbocyclen oder Heterocyclen gegebenenfalls mit einem Benzolring anelliert sein kann, wobei der Benzolring gegebenenfalls mit einem oder mehreren unter R¹², R¹³ und R¹⁴ ausgewählten Substituenten substituiert ist;
R¹⁶ , R¹⁷ , R¹⁸ und R¹⁹ jeweils unabhängig voneinander für Wasserstoff oder R¹⁵ stehen, oder
R¹⁷ und R¹⁸ oder R¹⁵ und R¹⁹ gemeinsam für C₁₋₆-Alkandiyl oder C₂₋₆-Alkendiyl stehen und **dadurch** gemeinsam einen gesättigten oder teilweise gesättigten monocyclischen 3- bis 8-gliedrigen Heterocyclus oder einen aromatischen 4- bis 8-gliedrigen monocyclischen Heterocyclus bilden können, wobei jeder dieser Heterocyclen gegebenenfalls mit einem oder mehreren unter R¹², R¹³ und R¹⁴ ausgewählten Substituenten substituiert sein kann; oder
R¹⁷ und R¹⁸ gemeinsam mit R¹⁶ für C₁₋₆-Alkandiyl oder C₂₋₆-Alkendiyl stehen und **dadurch** gemeinsam mit den Stickstoffatomen, an die sie gebunden sind, einen gesättigten oder teilweise gesättigten monocyclischen 3- bis 8-gliedrigen Heterocyclus oder einen aromatischen 4- bis 8-gliedrigen monocyclischen Heterocyclus bilden können, wobei jeder dieser Heterocyclen gegebenenfalls mit einem oder mehreren unter R¹², R¹³ und R¹⁴ ausgewählten Substituenten substituiert sein kann;
R²⁰ für einen monocyclischen, bicyclischen oder tricyclischen gesättigten Carbocyclus; einen monocyclischen, bicyclischen oder tricyclischen teilweise gesättigten Carbocyclus; einen monocyclischen, bicyclischen oder tricyclischen aromatischen Carbocyclus; einen monocyclischen,
bicyclischen oder tricyclischen gesättigten Heterocyclus; einen monocyclischen, bicyclischen oder tricyclischen teilweise gesättigten Heterocyclus; einen monocyclischen, bicyclischen oder tricyclischen aromatischen Heterocyclus steht;
R²¹ für einen monocyclischen, bicyclischen oder tricyclischen gesättigten Carbocyclus; einen monocyclischen, bicyclischen oder tricyclischen teilweise gesättigten Carbocyclus; einen monocyclischen, bicyclischen oder tricyclischen aromatischen Carbocyclus; einen monocyclischen,
bicyclischen oder tricyclischen gesättigten Heterocyclus; einen monocyclischen, bicyclischen oder tricyclischen teilweise gesättigten Heterocyclus; einen monocyclischen, bicyclischen oder tricyclischen aromatischen Heterocyclus steht, wobei jeder dieser R²¹ darstellenden Carbocyclen oder Heterocyclen gegebenenfalls mit einem oder mehreren unter R¹², R¹³ und R¹⁴ ausgewählten Substituenten substituiert sein kann;
Y₁ₐ für -Y₃-S (=O) -Y₄-; -Y₃-S(=O)₂-Y₄-; -Y₃-C(=O)-Y₄-; -Y₃-C(=S)-Y₄-; -Y₃-O-Y₄-; -Y₃-S-Y₄-; -Y₃-O-C(=O)-Y₄- oder -Y₃-C(=O)-O-Y₄- steht;
Y₁ oder Y₂ jeweils unabhängig voneinander für eine direkte Bindung, -Y₃-S (=O) -Y₄-; -Y₃-S (=O)₂-Y₄-; -Y₃-C(=O)-Y₄-; -Y₃-C (=S)-Y₄-; -Y₃-O-Y₄-; -Y₃-S-Y₄-; -Y₃-O-C(=O)-Y₄- oder -Y₃-C (=O) -O-Y₄- stehen;
Y₃ oder Y₄ jeweils unabhängig voneinander für eine direkte Bindung, C₁₋₆-Alkandiyl, C₂₋₆-Alkendiyl oder C₂₋₆-Alkindiyl stehen;
n für 1 oder 2 steht;
m für 1 oder 2 steht;
p für 1 oder 2 steht;
r für 1 bis 5 steht;
Aryl für Phenyl oder mit einem, zwei, drei, vier oder fünf jeweils unabhängig voneinander unter Halogen, C₁₋₆-Alkyl, C₃₋₇-Cycloalkyl, C₁₋₆-Alkyloxy, Cyano, Nitro, Polyhalogen-C₁₋₆-alkyl und Polyhalogen-C₁₋₆-alkyloxy ausgewählten Substituenten substituiertes Phenyl steht;
mit der Maßgabe, daß -X-R² und/oder R³ nicht für Wasserstoff stehen.

2. Verbindung nach Anspruch 1, in der
X für -NR¹-; -NH-NH-; -N=N-; -C(=O)-; -C (=S) -; -0-C(=O)-; -C(=O)-O-; -O-C(=O)-C₁₋₆-Alkyl-; -C(=O)-O-C₁₋₆-Alkyl-; -O-C₁₋₆-Alkyl-C (=O) - ; -C (=0) -C₁₋₆-Alkyl-0-; -O-C(=O)-NR¹-; -NR¹-CS(=O)-O-; -O-C(=O)-C(=O)-; -C (=O) -NR¹-; -NR¹-C(=O)-; -C (=S)-NR¹-; -NR¹-C (=S) -; -NR¹ -C (=O) -NR¹ -; -NR¹ -C (=S) -NR¹ -; -NR¹-S(=O)-NR¹-;-NR¹-S (=O)₂-NR¹-; -C₁₋₆-Alkyl-C (=O) -NR¹- ; -O-C₁₋₆-Alkyl-C (=O) -NR¹-; -C₁₋₆-Alkyl-O-C (=O) -NR¹-; -C₁₋₆-Alkyl-; -O-C₁₋₆-Alkyl-; -C₁₋₆-Alkyl-O-; NR¹-C₁₋₆-Alkyl-; -C₁₋₆-Alkyl-NR¹-; -NR¹-C₁₋₆-Alkyl-NR¹-; -NR¹-C₁₋₆-Alkyl-C₃₋₇-cycloalkyl-; -C₂₋₆-Alkenyl-; -C₂₋₆-Alkinyl-; -O-C₂₋₆-Alkenyl-; -C₂₋₆-Alkenyl-O-; -NR¹-C₂₋₆-Alkenyl.-; -C₂₋₆-Alkenyl-NR¹- -NR¹- C₂₋₆-Alkenyl-NR¹-*i* -NR¹-C₂₋₆-Alkenyl-C₃₋₇-cycloalkyl-; -O-C₂₋₆-Alkinyl-; -C₂₋₆-Alkinyl-O-; -NR¹-C₂₋₆-Alkinyl-; -C₂₋₆-Alkinyl-NR¹-; -NR¹-C₂₋₆-Alkinyl-NR¹-; -NR¹-C₂₋₆-Alkinyl-C₃₋₇-cycloalkyl-; -O-C₁₋₆-Alkyl-O-; -O-C₂₋₆-Alkenyl-O-; -O-C₂₋₆-Alkinyl-O-; -CHOH-;-S (=O)-;-S(=O)₂-; -S(=O)-NR¹-; -S (=O) ₂-NR¹-; -NR¹-S(=O)-, - NR¹- S (=O)₂-; -S-C₁₋₆-Alkyl-; -C₁₋₆-Alkyl-S- ; -S-C₂₋₆-Alkenyl-; -C₂₋₆-Alkenyl-S-; -S-C₂₋₆-Alkinyl-; -C₂-₆-Alkinyl-S- oder -O-C₁₋₆-Alkyl-S(=O)₂- steht;
R³ für Hydroxy; Halogen; mit Cyano, Hydroxy oder - C(=O)R⁷ substituiertes C₁₋₆-Alkyl; C₂₋₆-Alkenyl; mit einem oder mehreren Halogenatomen oder Cyano substituiertes C₂₋₆-Alkenyl; C₂₋₆-Alkinyl; mit einem oder mehreren Halogenatomen oder Cyano substituiertes C₂₋₆-Alkinyl; C₁₋₆-Alkyloxy; C₁₋₆-Alkylthio; C₁₋₆-Alkyloxycarbonyl; C₁₋₆-Alkylcarbonyloxy, Carboxyl; Cyano; Nitro; Amino; Mono- oder Di(C₁₋₆-alkyl)amino; Polyhalogen-C₁₋₆-alkyl; Polyhalogen-C₁₋₆-alkyloxy; Polyhalogen-C₁₋₆-alkylthio; R²¹; R²¹-C₁₋₆-Alkyl; R²¹-O-; R²¹-S-; R²¹ C(=O)-; R²¹-S(=O)ₚ-; R⁷-S (=O)ₚ-; R⁷-S(=O)ₚ-NH; R²¹-S(=O)ₚ-NH-; R⁷-C (=O) -; -NHC(=O)H; -C(=O)NHNH₂; R⁷-C(=O)-NH- ; R²¹-C(=O) -NH-; -C(=NH)R⁷ oder -C (=NH) R²¹ steht.

3. Verbindung nach Anspruch 1 oder 2, in der X nicht für NR¹ oder S steht.

4. Verbindung nach Anspruch 1, in der
R¹ für Wasserstoff steht;
X für -NR¹-; -O-; -O-C₁₋₆-Alkyl-; -NR¹- C₁₋₆-Alkyl- oder eine direkte Bindung steht;
R² für Wasserstoff, C₁₋₁₀-Alkyl oder R²⁰ steht, wobei jede dieser R² darstellenden Gruppen, sofern möglich, gegebenenfalls mit einem oder mehreren unabhängig voneinander unter R¹⁵; Cyano; R¹⁵-O- oder R⁵R⁶N-C(=O)- ausgewählten Substituenten substituiert sein kann;
R³ für Wasserstoff; Halogen; Cyano; Nitro; Amino oder R²¹-C₁₋₆-Alkyl steht;
R⁵ und R⁶ für Wasserstoff stehen;
R¹² R¹³ und R¹⁴ für R¹⁵ stehen;
R¹⁵ für C₁₋₆-Alkyl; einen monocyclischen, bicyclischen oder tricyclischen aromatischen Carbocyclus; einen monocyclischen, bicyclischen oder tricyclischen aromatischen Heterocyclus oder mit einen monocyclischen, bicyclischen oder tricyclischen aromatischen Carbocyclus substituiertes C₁₋₆-Alkyl steht;
R²⁰ für einen monocyclischen, bicyclischen oder tricyclischen aromatischen Carbocyclus steht;
R²¹ für einen monocyclischen, bicyclischen oder tricyclischen aromatischen Carbocyclus steht.

5. Verbindung nach einem der Ansprüche 1 bis 4, in der X-R² und R³ nicht für Wasserstoff stehen.

6. Verbindung nach einem der vorhergehenden Ansprüche, in der R²nicht für Wasserstoff oder C₁₋₆-Alkyl steht .

7. Verbindung nach Anspruch 1, bei der es sich um 3-[[5-Brom-4-(phenylmethoxy)-2-pyrimidinyl]amino]-benzamid;
3-[[5-Cyano-4-(phenylmethoxy)-2-pyrimidinyl]-amino]benzamid;
ein *N*-Oxid, ein pharmazeutisch unbedenkliches Additionssalz, ein quaternäres Amin und eine stereochemisch isomere Form davon handelt.

8. Verbindung nach Anspruch 1, bei der es sich um
3-(4-Benzyloxypyrimidin-2-ylamino)benzamid;
3-(4-Hydroxypyrimidin-2-ylamino)benzamid;
3-(5-Brom-4-hydroxypyrimidin-2-ylamino)benzamid;
ein N-Oxid, ein pharmazeutisch unbedenkliches Additionssalz, ein quaternäres Amin und eine stereochemisch isomere Form davon handelt.

9. Verbindung nach einem der Ansprüche 1 bis 8 zur Verwendung als Medizin.

10. Verwendung einer Verbindung nach einem der Ansprüche 1 bis 8 zur Herstellung eines Arzneimittels zur Prävention oder Behandlung von durch GSK3 vermittelten Erkrankungen.

11. Verwendung einer Verbindung nach einem der Ansprüche 1 bis 8 zur Herstellung eines Arzneimittels zur Prävention oder Behandlung von bipolarer Störung (insbesondere manischer Depression), Diabetes, Alzheimer-Krankhezt, Leukopenie, FTDP-17 (Frontotemporaler Demenz mit Parkinsonismus), kortikobasaler Degeneration, progressiver supranukleärer Lähmung, multipler Systematrophie, Pick-Krankheit, Niemann-Pick-Krankheit Typ C, Dementia pugilistica, Demenz mit Tangles, Demenz mit Tangles und Kalzifikation, Down-Syndrom, myotonischer Dystrophie, Guam-Parkinson-Demenz-Komplex, AIDS-bezogener Demenz, Parkinson-Syndrom, Prion-Erkrankungen mit Tangles, subakuter sklerosierender Panenzephalitis, Frontallappendegeneration (FLD), Silberkornkrankheit, subakuter sklerotisierender Panenzephalitis (SSPE) (Spätkomplikation von Virusinfektionen im Zentralnervensystem), Entzündungserkrankungen, Krebs, dermatologischen Störungen, neuronalen Schäden, Schizophrenie oder Schmerzen.

12. Verwendung nach Anspruch 11 zur Prävention oder Behandlung von Alzheimer-Krankheit, Diabetes, Krebs, Entzündungserkrankungen oder bipolarer Störung.

13. Pharmazeutische Zusammensetzung, enthaltend einen pharmazeutisch unbedenklichen Träger und als Wirkstoff eine therapeutisch wirksame Menge einer Verbindung nach einem der Ansprüche 1 bis 8.

14. Verfahren zur Herstellung einer pharmazeutischen Zusammensetzung nach Anspruch 13, **dadurch gekennzeichnet, daß** man eine therapeutisch wirksame Menge einer Verbindung nach einem der Ansprüche 1 bis 8 innig mit einem pharmazeutisch unbedenklichen Träger vermischt.

15. Verfahren zur Herstellung einer Verbindung nach Anspruch 1, **dadurch gekennzeichnet, daß** man
a) ein zwischenprodukt der Formel (II) in Gegenwart eines geeigneten Lösungsmittels und gegebenenfalls in Gegenwart einer geeigneten Säure oder einer geeigneten Base mit einem Zwischenprodukt der Formel (III) umsetzt 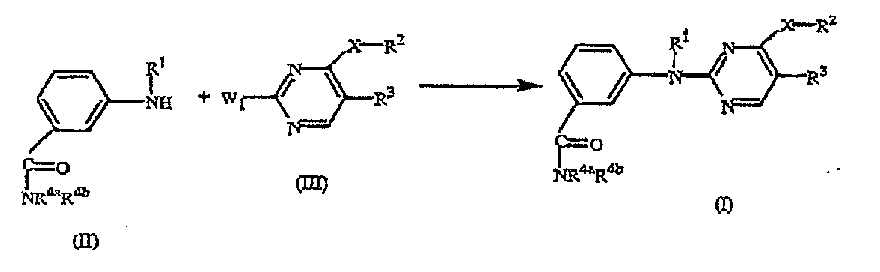 wobei W₁ für eine geeignete Abgangsgruppe steht und R¹ , R², R³, R^{4a}, R^{4b} und X die in Anspruch 1. angegebene Bedeutung besitzen;
b) ein Zwischenprodukt der Formel (IV) gegebenenfalls in Gegenwart eines geeigneten Lösungsmittels mit einem Zwischenprodukt der Formel (V) umsetzt 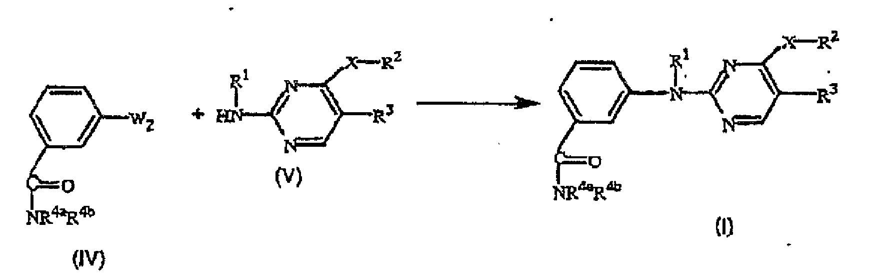 wobei W₂ für eine geeignete Abgangsgruppe steht und R¹ , R², R³ , R^{4a}, R^{4b} und X die in Anspruch 1 angegebene Bedeutung besitzen;
c) ein Zwischenprodukt der Formel (VI) in Gegenwart eines geeigneten Lösungsmittels mit einem Zwischenprodukt der Formel (VII) umsetzt 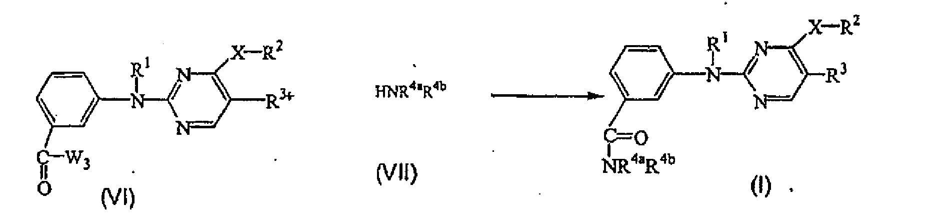 wobei W₃ für eine geeignete Abgangsgruppe steht und R¹ R² , R³ , R^{4a}, R^{4b} und X die in Anspruch 1 angegebene Bedeutung besitzen;
d) ein zwischenprodukt der Formel (VIII) in Gegenwart eines geeigneten Lösungsmittels und gegebenenfalls in Gegenwart einer geeigneten Base mit einem geeigneten Oxidationsmittel umsetzt 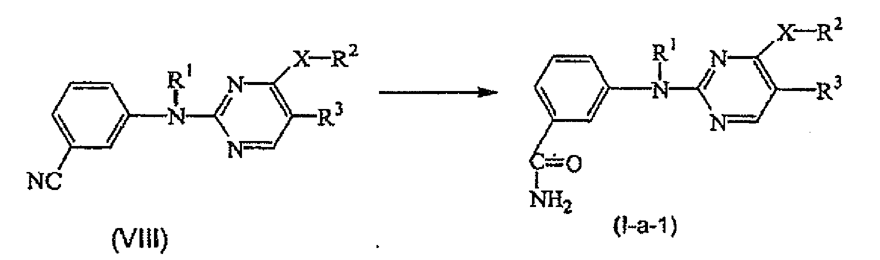
wobei R¹, R², R³ und X die in Anspruch 1 angegebene Bedeutung besitzen;
und gegebenenfalls Verbindungen der Formel (I) durch Behandlung mit einer Säure in ein therapeutisch wirksames nichttoxisches Säureadditionssalz oder durch Behandlung mit einer Base in ein therapeutisch wirksames nichttoxisches Basenadditionssalz oder umgekehrt die Säureadditionssalzform durch Behandlung mit Alkali in die freie Base oder die Basenadditionssalzform durch Behandlung mit Säure in die freie Säure umwandelt und gegebenenfalls stereochemisch isomere Formen, quaternäre Amine oder N-Oxidformen davon herstellt.

## Revendications

1. Composé de formule 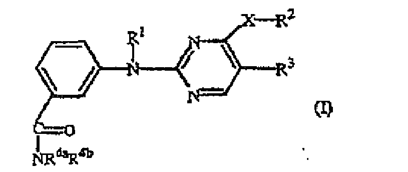 un N-oxyde, un sel d'addition d'acide pharmaceutiquement acceptable, une amine quaternaire et une forme stéréochimiquement isomère de celui-ci, où
R¹ représente hydrogène ; aryle ; formyle ; C₁₋₆alkylcarbonyle ; C₁₋₆alkyle ; C₁₋₆alkyloxycarbonyle; C₁₋₆alkyle substitué par formyle, C₁₋₆alkylcarbonyle, C₁₋₆alkyloxycarbonyle, C₁₋₆alkylcarbonyloxy; C₁₋₆alkyloxyC₁₋₆alkylcarbonyle éventuellement substitué par C₁₋₆alkyloxycarbonyle ;
X représente -NR¹- ; -NH-NH- ; -N=N- ; -O-; -C(=O); -C(=S)-; -O-C(=O)- -C(=O)-O-; -O-C(=O)-C₁₋₆alkyle ; -C (=O)-O-C₁₋₆alkyle ; -O-C₁₋₆alkyl-C(=O)- ; -C (=O) -C₁₋₆alkyl-O- ; -O-C(=O)-NR¹-; -NR¹-C (=O) -O- -O-C (=O)-C(=O)- ; -C (=O)-NR¹- , -NR¹-C(=O)- ; -C(=S)-NR¹, -NR¹=-C (=S) - ; -NR¹-C (=O) -NR¹- ; -NR¹-C (=S) -NR¹- ; -NR¹-S (=O) -NR¹- ; -NR¹-S (=O)₂-NR¹- ; -C₁₋₆alkyl-C (=O) -NR¹- ; O-C₁₋₆alkyl-C (=O)-NR¹- ; -C₁₋₆alkyl-O-C (=O) -NR¹- -C₁₋₆alkyl- ; -O-C₁₋₆alkyl-; -C₁₋₆alkyl-O- ; -NR¹-C₁₋₆alkyl- ; C₁₋₆alkyl-NR¹- ; -NR¹-C₁₋₆alkyl-NR¹- ; -NR¹-C₁₋₆alkyl-C₃₋₇cycloalkyl- ; -C₂₋₆alcényl- ; -C₂₋₆alcynyl- ; -O-C₁₋₆alcényl- ; -C₂₋₆alcényl-O- ; -NR¹-C₂₋₆alcényle ; -C₂₋₆alcényl-NR¹- ; -NR C₂₋₆alcényl-NR¹- ; -NR¹-C₂₋₆alcényl-C₃₋₇cycloalkyl- ; -O-C₂₋₆alcynyl- ; -C₂₋₆alcynyl-O- ; -NR¹-C₂₋₆alcynyle ; -C₂₋₆alcynyl-NR¹- ; -NR¹-C₂₋₆alcynyl-NR¹- ; -NR¹-C₂₋₆alcynyl-C₃₋₇cycloalkyl- ; -O-C₁₋₆alkyl-O- ; -O-C₂₋₆alcényl-O- ; -O-C₂₋₆alcynyl-O- ; -CHOH- ; -S- ; -S (=O) - ; -S (=O)₂-; -S(=O)-NR¹- ; -S(=O)₂-NR¹- ; -NR¹-S(=O) - ; -NR¹-S(=O)₂- ; S-C₁₋₆alkyl- ; -C₁₋₆alkyl-S- ;-S-C₂₋₆alcényl- ; -C₂₋₆alcényl-S- ; -S-C₂₋₆alcynyl- C₂₋₆alcynyl-S- ; -O-C₁₋₆alkyl-S(=O)₂- ou une liaison directe ;
R² représente hydrogène, C₁₋₁₀alkyle, C₂₋₁₀alcényle, C₂₋₁₀alcynyle, R²⁰, chacun desdits groupes représentant R² pouvant éventuellement, lorsque cela est possible, être substitué par un ou plusieurs substituants, choisis, chacun indépendamment, parmi =S; =O ; R¹⁵ ; hydroxy ; halogéno ; nitro ; cyano ; R¹⁵-O- _{;} SH ; R¹⁵- S- ; formyle ; carboxyle ; R¹⁵-C (=O) - ; R¹⁵-O-C ( =O )-; R¹⁵-C (=O)-O- ; R¹⁵-O-C(=O)-O- ; -SO₃H ; R¹⁵-S (=O )- ; R¹⁵-S (=O)₂- ; R⁵R⁶N ; R⁵R⁶N-C₁₋₆alkyl- ; R⁵R⁶ N-C₃₋₇cycloalkyl- ; R⁵R⁶N-C₁₋₆alkyloxy- ; R⁵R⁶N-C (=O)- ; R⁵R⁶N-C (=S)- ; R⁵R⁶N-C (=O)-NH- ; R⁵R⁶N-C (=S)-NH- ; R⁵R⁶N-S (=O)ₙ- ; R⁵R⁶N-S (=O)ₙ-NH- ; R¹⁵-C (=S ) - ; R¹⁵-C(=O)-NH- ; R¹⁵-O-C (=O)-NH- ; R¹⁵-S (=O)ₙ-NH- ; R¹⁵-O-S (=O)ₙ-NH- ; R¹⁵-C(=S)-NH- ; R¹⁵-O-C (=S) -NH- ; R¹⁷R¹⁸N-Y₁ₐ- R¹⁷R¹⁸N-Y₂-NR¹⁶-Y₁- ; R¹⁵-Y₂-NR¹⁹-Y₁- : H-Y₂-NR¹⁹-Y₁- ;
R³ représente hydrogène ; hydroxy ; halogène ; C₁₋₆alkyle ; C₁₋₆alkyle substitué par cyano, hydroxy ou -C (=O) R⁷ ; C₂₋₆alcényle ; C₂₋₆alcényle substitué par un ou plusieurs atomes d'halogène ou cyano ; C₂₋₆alcynyle ; C₂₋₆alcynyle substitué par un ou plusieurs atomes d'halogène ou cyano ; C₁₋₆alkyloxy ; C₁₋₆alkylthio ; C₁₋₆alkyloxycarbonyle ; C₁₋₆alkylcarbonyloxy ; carboxyle ; cyano ; nitro ; amino ; mono- ou di(C₁₋₆alkyl)amino ; polyhalogénoC₁₋₆alkyle ; polyhalogénoC₁₋₆alkyloxy ; polyhalogénoC₁₋₆alkylthio R²¹ ; R²¹-C₁₋₆alkyle ; R²¹ -O- ; R²¹ -S- ; R²¹ -C (=O)- ; R²¹ -S(=O)ₚ- ; R⁷ -S(=O)ₚ- ; R⁷ -S(=O)ₚ-NH- ; R²¹ -S (=O)ₚ-NH- ; R⁷-C(=O)- *;* -NHC(=O)H ; -C(=O)NHNH₂ ; R⁷-C (=O) -NH- ; R²¹ -C(=O)-NH- ; -C(=NH)R⁷; -C (=NH) R²¹ ;
R^{4a} ou R^{4b} représentent, chacun indépendamment, hydrogène ou R⁸ ;
R⁵ et R⁶ représentent, chacun indépendamment, hydrogène, R⁸, -Y₁-NR⁹-Y₂-NR₁₀R¹¹, -Y₁-NR⁹ -Y₁-R⁸, -Y₁-NR⁹R¹⁰, ou
R⁵ et R⁶ peuvent former, ensemble avec l'azote auquel ils sont attachés, un hétérocycle saturé ou partiellement saturé, monocyclique, de 3 à 8 chaînons ou un hétérocycle aromatique monocyclique de 4 à 8 chaînons, chacun desdits hétérocycles pouvant éventuellement être substitué par un ou plusieurs substituants choisis parmi R¹² R¹³ et R¹⁴, ou chacun desdits hétérocycles pouvant éventuellement être condensé avec un cycle benzène, chaque cycle benzène étant éventuellement substitué par un ou plusieurs substituants choisis parmi R¹², R¹³ et R¹⁴ ;
R⁷ représente C₁₋₆alkyle, C₁₋₆alkyloxy, amino, mono- ou di(C₁₋₆alkyl)amino ou polyhalogénoC₁₋₆alkyle ;
R⁸ représente C₁₋₆alkyle ;
R⁹ R¹⁰ et R¹¹ représentent, chacun indépendamment, hydrogène ou R⁸, ou
deux radicaux quelconques parmi R⁹ R¹⁰ et R¹¹ peuvent former ensemble C₁₋₆alcanediyle ou C₂₋₆alcènediyle, formant ainsi un hétérocycle saturé ou partiellement saturé, monocyclique, de 3 à 8 chaînons ou un hétérocycle aromatique monocyclique de 4 à 8 chaînons ensemble avec les atomes d'azote auxquels ils sont attachés, chacun desdits hétérocycles pouvant éventuellement être substitué par un ou plusieurs substituants choisis parmi R¹²_{,} R¹³ et R¹⁴;
R¹², R¹³ et R¹⁴ représentent, chacun indépendamment, hydrogène ; R¹⁵ ; hydroxy ; halogéno ; nitro ; cyano ; R¹⁵-O- ; SH ; R¹⁵-S- ; formyle ; carboxyle ; R¹⁵-C (=O) - R¹⁵-O-C (=O) - ; R¹⁵-C (=O) -O- ; R¹⁵-OC (=O) -O- ; -SO₃H ; R¹⁵-S (=O) R¹⁵-S (=O)₂- ; R¹⁵R¹⁶N-S (=O) - R¹⁵R¹⁶N-S (=O)₂- R¹⁷R¹⁸N-Y₁- ; R¹⁷R¹⁸N - Y₂ - NR¹⁶-Y₁- ;R¹⁵ -Y₂-NR¹⁹ - Y₁-; H - Y₂ - NR¹⁹ - Y₁ -; oxo, ou
deux radicaux quelconques parmi R¹² R¹³ et R¹⁴ peuvent former ensemble C₁₋₆alcanediyle ou C₂₋₆alcènediyle, formant ainsi un cycle carboné ou un hétérocycle saturé ou partiellement saturé, monocyclique, de 3 à 8 chaînons ou un cycle carboné ou un hétérocycle aromatique monocyclique de 4 à 8 chaînons ensemble avec les atomes auxquels ils sont attachés ;
deux radicaux quelconques parmi R¹² R¹³ et R¹⁴ peuvent former ensemble -O-(CH₂)ᵣ-O-, formant ainsi un cycle carboné ou un hétérocycle saturé, partiellement saturé ou aromatique, monocyclique, de 4 à 8 chaînons avec les atomes auxquels ils sont attachés ;
R¹⁵ représente C₁₋₆alkyle, C₂₋₆alcényle, C₂₋₆alcynyle, un cycle carboné monocyclique, bicyclique ou tricyclique saturé ; un cycle carboné monocyclique, bicyclique ou tricyclique partiellement saturé ; un cycle carboné monocyclique, bicyclique ou tricyclique aromatique ; un hétérocycle monocyclique, bicyclique ou tricyclique saturé ; un hétérocycle monocyclique, bicyclique ou tricyclique partiellement saturé ; un hétérocycle monocyclique, bicyclique ou tricyclique aromatique ; C₁₋₆alkyle substitué par un cycle carboné monocyclique, bicyclique ou tricyclique saturé ou par un cycle carboné monocyclique, bicyclique ou tricyclique partiellement saturé ou par un cycle carboné monocyclique, bicyclique ou tricyclique aromatique ou par un hétérocycle monocyclique, bicyclique ou tricyclique saturé ou par un hétérocycle monocyclique, bicyclique ou tricyclique partiellement saturé ou par un hétérocycle monocyclique, bicyclique ou tricyclique aromatique ; chacun desdits substituants représentant R¹⁵ peut éventuellement être substitué par un ou plusieurs substituants choisis parmi R¹², R¹³ et R¹⁴ ; ou chacun desdits carbocycliques ou hétérocycles peut éventuellement être condensé par un cycle benzène, ledit cycle benzène étant éventuellement substitué par un ou plusieurs substituants choisis parmi R¹², R¹³ et R¹⁴;
R¹⁶, R¹⁷, R¹⁸ et R¹⁹ représentent, chacun indépendamment, hydrogène ou R¹⁵, ou
R¹⁷ et R¹⁸ ou R¹⁵ et R¹⁹ peuvent former ensemble C₁₋₆alcanediyle ou C₂₋₆alcènediyle, formant ainsi un hétérocycle saturé ou partiellement saturé, monocyclique, de 3 à 8 chaînons ou un hétérocycle aromatique monocyclique de 4 à 8 chaînons, chacun desdits hétérocycles pouvant éventuellement être substitué par un ou plusieurs substituants choisis parmi R¹², R¹³ et R¹⁴ _{;} ou
R¹⁷ et R¹⁸ ensemble avec R¹⁶ peuvent former C₁₋₆alcanediyle ou C₂₋₆alcènediyle, formant ainsi un hétérocycle saturé ou partiellement saturé, monocyclique, de 3 à 8 chaînons ou un hétérocycle aromatique monocyclique de 4 à 8 chaînons ensemble avec les atomes d'azote auxquels ils sont attachés, chacun desdits hétérocycles pouvant éventuellement être substitué par un ou plusieurs substituants choisis parmi R¹², R¹³ et R¹⁴ *i*
R²⁰ représente un cycle carboné monocyclique, bicyclique ou tricyclique saturé ; un cycle carboné monocyclique, bicyclique ou tricyclique partiellement saturé ; un cycle carboné monocyclique, bicyclique ou tricyclique aromatique ; un hétérocycle monocyclique, bicyclique ou tricyclique saturé ; un hétérocycle monocyclique, bicyclique ou tricyclique partiellement saturé ; un hétérocycle monocyclique, bicyclique ou tricyclique aromatique ;
R²¹ représente un cycle carboné monocyclique, bicyclique ou tricyclique saturé ; un cycle carboné monocyclique, bicyclique ou tricyclique partiellement saturé ; un cycle carboné monocyclique, bicyclique ou tricyclique aromatique ; un hétérocycle monocyclique, bicyclique ou tricyclique saturé ; un hétérocycle monocyclique, bicyclique ou tricyclique partiellement saturé ; un hétérocycle monocyclique, bicyclique ou tricyclique aromatique, chacun desdits cycles carbonés ou hétérocycles représentant R²¹ pouvant éventuellement être substitué par un ou plusieurs substituants choisis parmi R¹², R¹³ et R¹⁴ ;
Y₁ₐ représente -Y₃-S (=O) -Y₄- ; -Y₃-S (=O)₂-Y₄-, Y₃-C(=O) -Y₄-, -Y₃-C (=S) -Y₄-, -Y₃-O-Y₄-, -Y₃-S-Y₄-, -Y₃-S-Y₄-, -Y₃-O-C (=O) -Y₄- ou -Y₃-C (=O) -O-Y₄- ;
Y₁ ou Y₂ représentent, chacun indépendamment, une liaison directe, -Y₃-S (=O)Y₄- ; -Y₃-S (=O) ₂-Y₄-, -Y₃-C (=O)-Y₄-, Y₃C(=S)-Y₄-, -Y₃-O-Y₄-, -Y₃-S-Y₄, -Y₃-O-C(=O) -Y₄- ou -Y₃-C(=O)-O-Y₄- ;
Y₃ ou Y₄ représentent, chacun indépendamment, une liaison directe, C₁₋₆alcanediyle, C₂₋₆alcènediyle ou C₂₋₆alcynediyle ;
n vaut 1 ou 2 ;
m vaut 1 ou 2 ;
p vaut 1 ou 2 ;
r vaut 1 à 5 ;
aryle représente phényle ou phényle substitué par un, deux, trois, quatre ou cinq substituants, choisis, chacun indépendamment, parmi halogéno, C₁₋₆alkyle, C₃₋₇cycloalkyle, . C₁₋₆alkyloxy, cyano, nitro, polyhalogénoC₁₋₆alkyle et polyhalogénoC₁₋₆alkyloxy ;
à condition que : -X-R² et/ou R³ soient différents d'hydrogène.

2. Composé selon la revendication 1, où
X représente -NR¹- ; -NH-NH- ; -N=N- ; -C(=O)- ;-C(=S)- ; -O-C(=O)- ; -C(=O)-O- ; -O-C (=O) -C₁₋₆alkyl- ; -C (=O) -O-C₁₋₆alkyl- ; -O-C₁₋₆alkyl-C (=O) - ; -C (=O) -C₁₋₆alkyl-O- ; -O-C (=O) -NR¹ ; -NR¹-C (=O) -O- ; -O-C(=O)-C(=O)- ; -C(=O)-NR¹-, -NR¹-C (=O) - ; -C (=S) -NR¹- ; -NR¹-C (=S)- ; -NR¹-C (=O) -NR¹- ; -NR¹-C (=S) -NR¹- ; -NR¹-S =O-NR¹ - ; -NR¹-S (=O) ₂-NR¹-; -C₁₋₆alkyl-C (=O) -NR¹ ; -O-C₁₋₆alkyl-C (=O) -NR¹- ; -C₁₋₆alkyl-O-C (=O) -NR¹- ; -C₁₋₆alkyl- ; -O-C₁₋₆alkyl- ; -C₁₋₆alkyl-O- ; -NR¹-C₁₋₆alkyl-; -C₁₋₆alkyl-NR¹- ; -NR¹-C₁₋₆alkyl-NR¹- ; -NR¹- C₁₋₆alkyl-C₃₋₇cycloalkyl- -C₂₋₆alcényl- ; -C₂₋₆alcynyl- ; -O-C₂₋₆alcényl- ; -C₂₋₆alcényl-O- ; -NR¹-C₂₋₆alcényl- ; -C₂₋₆alcényl-NR¹- ; -NR¹-C₂₋₆alcényl-NR¹- ; -NR¹- C₂₋₆alcényl-C₃₋₇cycloalkyl- -O-C₂₋₆alcynyl- ; -C₂₋₆alcynyl-O- ; NR¹- C₂₋₆alcynyl- ; -C₂₋₆alcynyl-NR¹- ; -NR -C₂₋₆alcynyl -NR¹- *i* -NR¹-C₂₋₆alcynyl-C₃₋₇cycloalkyl- ; -O-C₁₋₆alkyl-O- ; -O-C₂₋₆alcényl-O- ; -O-C₂₋₆alcynyl-O- ; -CHOH- ; -S(=O)- ; -S(=O)₂- ; -S (=O) -NR¹- ; -S (=O)₂-NR¹- ; -NR¹- S(=O)- -NR¹-S (=O)₂- ; -S-C₁₋₆alkyl- ; -C₁₋₆alkyl-S- ; -S-C₂₋₆alcényl- ; -C₂₋₆alcényl-S-; -S-C₂₋₆alcynyl- ; -C₂₋₆alcynyl-S- ; -O-C₁₋₆alkyl-S (=O) 2- ;
R³ représente hydroxy ; halogéno ; C₁₋₆alkyle substitué par cyano, hydroxy ou -C(=O)R⁷; C₂₋₆alcényle ; C₂₋₆alcényle substitué par un ou plusieurs atomes d'halogène ou cyano ; C₂₋₆alcynyle ; C₂₋₆alcynyle substitué par un ou plusieurs atomes d'halogène ou cyano ; C₁₋₆alkyloxy ; C₁₋₆alkylthio ; C₁₋₆alkyloxycarbonyle ;C₁₋₆alkylcarbonyloxy ; carboxyle ; cyano ; nitro ; amino ; mono- ou di (C₁₋₆alkyl) amine ; polyhalogénoC₁₋₆alkyle polyhalogénoC₁₋₆alkyloxy ; polyhalogénoC₁₋₆alkylthio ; R²¹ ; R²¹-C₁₋₆alkyle R²¹ -O- ;R²¹-S- ; R²¹-C (=O)- ;R²¹ -S (=O)ₚ- ; R⁷-S(=O)ₚ- ; R⁷-S(=O)ₚ-NH- ; R²¹-S(=O)ₚ-NH- ; R⁷-C(=O)- ; -NHC(=O)H ; -C(=O)NHNH₂ ; R⁷-C(=O)-NH- ; R²¹-C(=O)-NH- ; -C(=NH)R ⁷ ; -C(=NH)R²¹.

3. Composition selon la revendication 1 ou 2 où X est différent de NR¹ ou S.

4. Composé selon la revendication 1, où
R¹ représente hydrogène ;
X représente -NR¹- ; -O- ; -O-C₁₋₆alkyl- ; -NR¹-C₁₋₆alkyl- ou une liaison directe ;
R² représente hydrogène, C₁₋₁₀alkyle, R²⁰, chacun desdits groupes représentant R² pouvant éventuellement être substitué, lorsque cela est possible, par un ou plusieurs substituants choisis, chacun indépendamment, parmi R¹⁵; cyano ; R¹⁵-O- ; R⁵R⁶N-C (=O) - ;
R³ représente hydrogène ; halogène ; cyano ; nitro ; amino ; R²¹-C₁₋₆alkyle ;
R⁵ et R⁶ représentent hydrogène :
R¹², R¹³ et R¹⁴ représentent R¹⁵ ;
R¹⁵ représente C₁₋₆alkyle *i* un cycle carboné monocyclique, bicyclique ou tricyclique aromatique ; un hétérocycle monocyclique, bicyclique ou tricyclique aromatique ; C₁₋₆alkyle substitué par un cycle carboné monocyclique, bicyclique ou tricyclique aromatique ;
R²⁰ représente un cycle carboné monocyclique, bicyclique ou tricyclique aromatique ;
R²¹ représente un cycle carboné monocyclique, bicyclique ou tricyclique aromatique.

5. Composé selon l'une quelconque des revendications 1 à 4 où X-R² et R³ sont différents d'hydrogène.

6. Composé selon l'une quelconque des revendications précédentes, où R² est différent d'hydrogène ou de C₁₋₆alkyle.

7. Composé selon la revendication 1 où le composé est le
3-[[5-bromo-4-(phénylméthoxy)-2--pyrimidinyl]amino)-benz amide ; le
3-[[5-cyano-4-(phénylméthoxy)-2-pyrimidinyl]amino]-benz amide ; un N-oxyde, un sel d'addition d' acide pharmaceutiquement acceptable, une amine quaternaire et une forme stéréochimiquement isomère de ceux-ci.

8. Composé selon la revendication 1 où le composé est le 3-(4-benzyloxypyrimidin-2-ylamino)-benzamide ; le 3-(4-hydroxypyrimidin-2-ylamino)-benzamide ; le 3-(5-bromo-4-hydroxypyrimidin-2-ylamino)-benzamide, un N-oxyde, un sel d'addition d'acide pharmaceutiquement acceptable, une amine quaternaire et une forme stéréochimiquement isomère de ceux-ci.

9. Composé selon l'une quelconque des revendications 1 à 8 destiné à une utilisation comme médicament.

10. Utilisation d'un composé selon l'une quelconque des revendications 1 à 8 pour la préparation d'un médicament destiné à la prévention ou au traitement de maladies dans lesquelles intervient la GSK3.

11. Utilisation d'un composé selon l'une quelconque des revendications 1 à 8 pour la préparation d'un médicament destiné à la prévention ou au traitement de maladies telles qu'un trouble bipolaire (en particulier la psychose maniaco-dépressive), le diabète, la maladie d'Alzheimer, la leucopénie, la FTDP-17 (démence fronto-temporale associée à la maladie de Parkinson), la dégénérescence corticobasale, la paralysie supranucléaire progressive, l'atrophie multisystématisée, la maladie de Pick, la maladie de Niemann-Pick de type C, la Dementia Pugilistica, la démence avec enchevêtrements uniquement, la démence avec enchevêtrements et calcification, le syndrome de Down, la dystrophie myotonique, le complexe Parkinsonisme-démence de Guam, la démence liée au SIDA, le Parkinsonisme post-encéphalique, les maladies à prion avec enchevêtrements, la panencéphalite sclérosante subaiguë, la dégénérescence du lobe frontal (FLD), la maladie des grains agyrophiles, la panencéphalite sclérosante subaiguë (SSPE) (complication tardive des infections virales dans le système nerveux central), les maladies inflammatoires, le cancer, les troubles dermatologiques, les dommages neuronaux, la schizophrénie, la douleur.

12. Utilisation selon la revendication 11 pour la prévention ou le traitement de la maladie d'Alzheimer, le cancer, les maladies inflammatoires ou le trouble bipolaire.

13. Composition pharmaceutique comprenant un support pharmaceutiquement acceptable et, comme ingrédient actif, une quantité thérapeutiquement efficace d'un composé selon l'une quelconque des revendications 1 à 8.

14. Procédé de préparation d'une composition pharmaceutique selon la revendication 13, **caractérisé en ce qu'**une quantité thérapeutiquement efficace d'un composé selon l'une quelconque des revendications 1 à 8 est mélangée intimement avec un support pharmaceutiquement acceptable.

15. Procédé de préparation d'un composé selon la revendication 1, **caractérisé par**
a) la réaction d'un intermédiaire de formule (II) avec un intermédiaire de formule (III) en présence d'un solvant approprié et éventuellement en présence d'un acide approprié ou d'une base appropriée 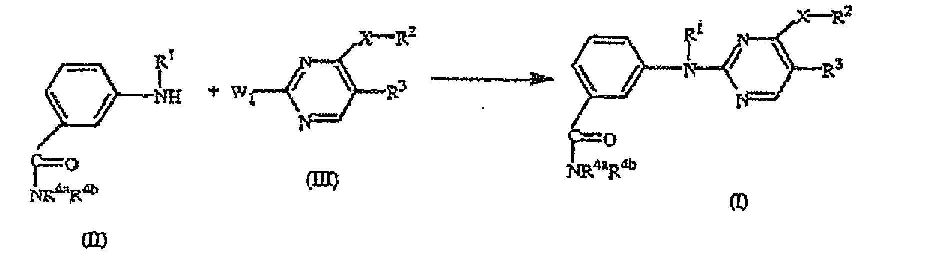 avec W₁ représentant un groupe partant approprié et R¹, R², R³, R^{4a}, R^{4b} et X étant tels que définis dans la revendication 1 ;
b) la réaction d'un intermédiaire de formule (IV) avec un intermédiaire de formule (V) éventuellement en présence d'un solvant approprié 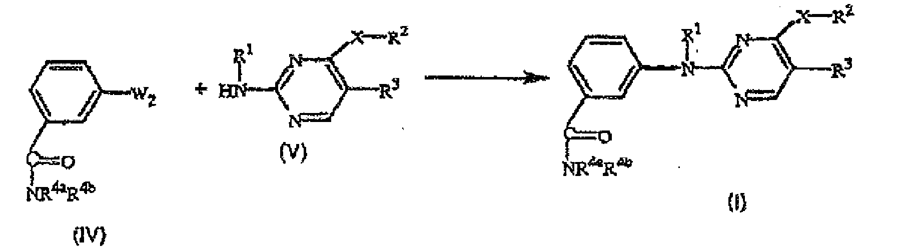 avec W₂ représentant un groupe partant approprié et R¹, R², R³, R^{4a} ,R^{4b} et X étant tels que définis dans la revendication 1 ;
c) la réaction d'un intermédiaire de formule (VI) avec un intermédiaire de formule (VII) en présence d'un solvant approprié 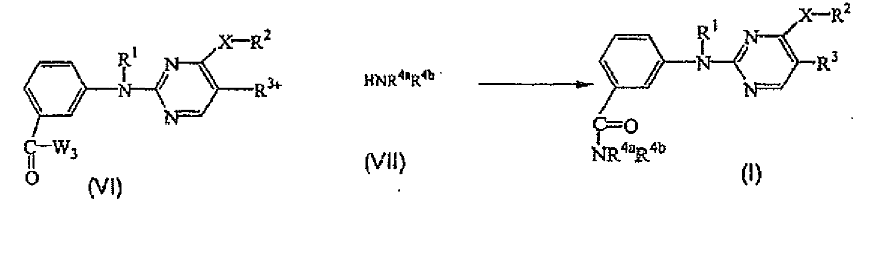 avec W₃ représentant un groupe partant approprié et R¹, R², R³, R^{4a}, R^{4b} et X étant tels que définis dans la revendication 1 ;
d) la réaction d'un intermédiaire de formule (VIII) avec un oxydant approprié en présence d'un solvant approprié et éventuellement en présence d'une base appropriée 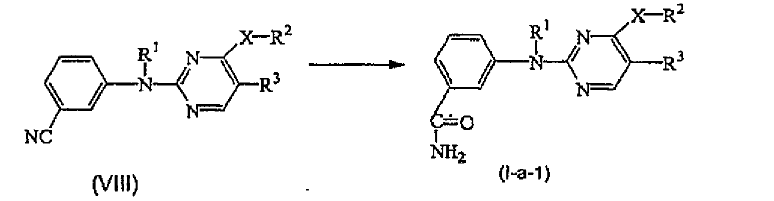 avec R¹, R², R³ et X tels que définis dans la revendication 1 ;
et, si souhaité, la conversion des composés de formule (I) en un sel d'addition d'acide thérapeutiquement actif, non toxique, par traitement avec un acide ou en un sel d'addition de base thérapeutiquement actif, non toxique par traitement avec une base ou, inversement, la conversion du sel d'addition d'acide en base libre par le traitement avec un métal alcalin ou la conversion du sel d'addition de base en acide libre par traitement avec un acide ; et, si souhaité, la préparation de formes stéréochimiquement isomères, d'amines quaternaires ou de formes N-oxyde.
